(19) Europäisches Patentamt
European Patent Office
Office européen des brevets

(11) EP 3 604 325 A1

(12) # EUROPEAN PATENT APPLICATION
published in accordance with Art. 153(4) EPC

(43) Date of publication:
**05.02.2020 Bulletin 2020/06**

(21) Application number: **18775236.5**

(22) Date of filing: **29.03.2018**

(51) Int Cl.:
***C07K 7/06*** *(2006.01)* ***A61K 39/00*** *(2006.01)*
***A61P 35/00*** *(2006.01)* ***A61P 35/02*** *(2006.01)*
***A61P 37/04*** *(2006.01)* ***C07K 7/08*** *(2006.01)*
***C07K 19/00*** *(2006.01)*

(86) International application number:
**PCT/JP2018/013092**

(87) International publication number:
**WO 2018/181648 (04.10.2018 Gazette 2018/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2017 JP 2017068541**

(71) Applicants:
- **Sumitomo Dainippon Pharma Co., Ltd.**
  **Osaka-shi**
  **Osaka 541-8524 (JP)**
- **International Institute of Cancer Immunology, Inc.**
  **Suita-shi, Osaka 564-0053 (JP)**

(72) Inventors:
- **BAN, Hitoshi**
  **Osaka-shi**
  **Osaka 554-0022 (JP)**
- **TAKANASHI, Yosuke**
  **Osaka-shi**
  **Osaka 554-0022 (JP)**

(74) Representative: **Vossius & Partner**
**Patentanwälte Rechtsanwälte mbB**
**Siebertstrasse 3**
**81675 München (DE)**

(54) **WT1 CANCER ANTIGEN PEPTIDE AND PEPTIDE CONJUGATE BODY CONTAINING SAME**

(57) The present disclosure relates to a WT1 peptide having a cysteine residue and including 10 to 12 residues, a peptide conjugate containing the same, and a combination of the WT1 peptide or the peptide conjugate and a WT1 helper peptide.

EP 3 604 325 A1

## Description

Technical Field

The present application claims the benefit of Japanese Patent Application No. 2017-068541, which is incorporated herein by reference in its entirety.

The present disclosure relates to cancer immunotherapy and includes cancer antigen peptides from WT1 protein and peptide conjugates containing the peptide.

Background

WT1 Gene has been isolated as a responsible gene of Wilms tumor, which is a kidney cancer in children. Leukemia and some solid cancers are known to be associated with high expression of WT1. WT1 Protein is one of cancer antigen proteins of strong interest for use in cancer vaccines. (Non-patent literature 1).

Cellular immunity, especially cytotoxic T cells (cytotoxic T lymphocytes) (also referred to as CTLs hereinafter) play an important role in cancer cell clearance by a living body. CTLs that attack cancer cells are derived from precursor T cells through their differentiation and proliferation, upon recognition by precursor T cells of an antigen peptide having 8 to 13 amino acid residues from a cancer antigen protein complexed with an MHC class I molecule.

As to WT1 protein, cancer antigen peptides such as $WT1_{235-243}$ peptide: CMTWNQMNL (Cys-Met-Thr-Trp-Asn-Gln-Met-Asn-Leu) (SEQ ID NO: 3) and $WT1_{235-243\ (2M\rightarrow Y)}$ peptide: CYTWNQMNL (Cys-Tyr-Thr-Trp-Asn-Gln-Met-Asn-Leu) (SEQ ID NO: 4), in which the amino acid at position 2 of $WT1_{235-243}$ peptide is substituted, are known to bind to an MHC class I molecule to be presented (see Patent Literatures 1 and 2).

Typical peptide vaccines using antigen peptides have been known to have weak immunogenicity, and their CTL induction has been desired to be efficiently enhanced. A complex of different cancer antigen peptides via a disulfide bond (referred to as a peptide conjugate hereafter) has been known as one of peptide cancer vaccines that can efficiently induce CTLs against WT1-expressing cancer cells (see Patent Literature 3). However, further improvements have been desired for improving characteristics such as pharmacological activity, physical properties, and stability, and for efficiently inducing CTLs *in vivo.*

A plurality of peptidases is involved in presenting a cancer antigen peptide to an MHC class I molecule. Among the peptidases, endoplasmic reticulum aminopeptidase 1 (hereinafter referred to as ERAP1) is one of trimming enzymes in endoplasmic reticula (referred to as ERs hereinafter). ERAP1 recognizes a specific antigen peptide sequence and peptide length, cuts out a peptide from its N-terminus, and modifies the peptide to an optimal length for binding to an MHC class I molecule (see Non Patent Literatures 2 to 4). However, trimming function of ERAP1 has high substrate specificity, and there have been no report of peptides decomposed by the trimming function of ERAP1 to produce the $WT1_{235-243}$ peptide or $WT1_{235-243(2M\rightarrow Y)}$ peptide.

Citation List

Patent Literature

Patent Literature 1: WO 00/06602
Patent Literature 2: WO 02/079253
Patent Literature 3: WO 2014/157692

Non Patent Literature

Non Patent Literature 1: Clinical Cancer Research, 2009; 15 (17); 5323-5337
Non Patent Literature 2: Proceedings of the national academy of sciences of United States of America, 2005; 102 (47); 17107-17112
Non Patent Literature 3: The Journal of Immunology, 2009; 183; 5526-5536
Non Patent Literature 4: The Journal of Immunology, 2010; 184; 4725-4732

Summary

Technical Problem

An object of the present disclosure is to provide a WT1 peptide having high immunogenicity and a peptide conjugate containing the same.

Solution to Problem

The inventors have previously found that ERAP1 acts *in vivo* on an amino acid adduct of a cancer antigen peptide in which a certain amino acid residue is attached to the N-terminus of the peptide such that the pharmacological activity, physical properties and/or stability is improved, and decomposes the amino acid adduct into the original cancer antigen peptide. While examining earnestly, the inventors have attempted to adopt this technology into the $WT1_{235-243}$ peptide or the $WT1_{235-243(2M\rightarrow Y)}$ peptide, and confirmed for the first time that amino acid adducts of these cancer antigen peptides are decomposed by ERAP1. These amino acid adducts have been strongly suggested to exhibit the same pharmacological effects as the original cancer antigen peptides in experiments such as a pharmacological test in an *in vivo* animal model. The inventors have further confirmed that the pharmacological activity in the *in vivo* animal model is improved when the amino acid adducts are adopted to peptide conjugates. Finally, the inventors have achieved a polyvalent antigen-presenting conjugate vaccine that efficiently induces CTLs in a living body, and completed the present invention.

Accordingly, the present invention includes embodiments described below.

(1) The First Embodiment

1. A compound of formula (1):

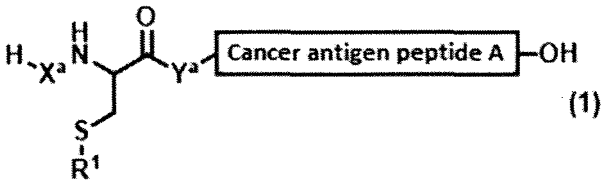

(1)

wherein $X^a$ and $Y^a$ independently represent a single bond or a divalent peptide group consisting of 1 to 4 amino acid residues, provided that the sum of the number of amino acid residues in $X^a$ and $Y^a$ is an integer of 0 to 4;
cancer antigen peptide A is an MHC class I-restricted WT1 peptide consisting of 7 to 30 amino acid residues, wherein an amino group of an N-terminal amino acid of the cancer antigen peptide A binds to $Y^a$ in the formula (1), and a carbonyl group of a C-terminal amino acid of the cancer antigen peptide A binds to the hydroxyl group in the formula (1); and
$R^1$ is hydrogen, a group represented by formula (2):

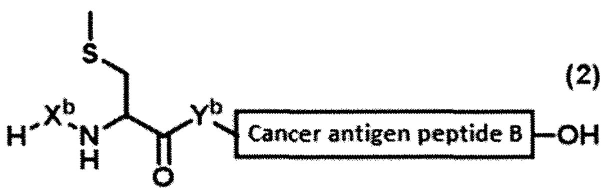

(2)

wherein $X^b$ and $Y^b$ independently represent a single bond or a divalent peptide group consisting of 1 to 4 amino acid residues, provided that the sum of the number of amino acid residues in $X^b$ and $Y^b$ is an integer of 0 to 4;
cancer antigen peptide B is an MHC class I-restricted WT1 peptide consisting of 7 to 30 amino acid residues, wherein an amino group of an N-terminal amino acid of the cancer antigen peptide B binds to $Y^b$ in the formula (2), and a carbonyl group of a C-terminal amino acid of the cancer antigen peptide B binds to the hydroxyl group in the formula (2), provided that the cancer antigen peptide B has a different amino acid sequence from the cancer antigen peptide A; and the formula (1) and the formula (2) binds via a disulfide

bond,

or cancer antigen peptide C,

wherein the cancer antigen peptide C is an MHC class I-restricted WT1 peptide consisting of 7 to 30 amino acid residues including one cysteine residue or an MHC class II-restricted WT1 peptide consisting of 7 to 30 amino acid residues including one cysteine residue, provided that the cancer antigen peptide C has a different amino acid sequence from the cancer antigen peptide A, and wherein the cysteine residue of the cancer antigen peptide C binds to the formula (1) via a disulfide bond, provided that, when $R^1$ is hydrogen, the sequence of the compound of formula (1) is not a partial sequence of WT1 protein;

or a pharmaceutically acceptable salt thereof.

2. The compound or a pharmaceutically acceptable salt thereof of item 1, wherein $X^a$ is a divalent peptide group consisting of 2 amino acid residues, and $Y^a$ is a single bond; $X^a$ and $Y^a$ are independently a divalent peptide group consisting of 1 amino acid residue; $X^a$ is a single bond, and $Y^a$ is a divalent peptide group consisting of 2 amino acid residues; $X^a$ is a divalent peptide group consisting of 1 amino acid residue, and $Y^a$ is a single bond; $X^a$ is a single bond, and $Y^a$ is a divalent peptide group consisting of 1 amino acid residue; or $X^a$ and $Y^a$ are single bonds.

3. The compound or a pharmaceutically acceptable salt thereof of item 1 or 2, wherein $X^a$ is a single bond, and $Y^a$ is a single bond, an alanine residue, a leucine residue or a methionine residue.

4. The compound or a pharmaceutically acceptable salt thereof of item 1 or 2, wherein $X^a$ is a single bond or a divalent peptide group consisting of 1 amino acid residue, and $Y^a$ is a single bond.

5. The compound or a pharmaceutically acceptable salt thereof of any one of items 1-4, wherein $X^a$ and $Y^a$ are single bonds.

6. The compound or a pharmaceutically acceptable salt thereof of any one of items 1-5, wherein the cancer antigen peptide A is an MHC class I-restricted WT1 peptide consisting of 7 to 15 amino acid residues.

7. The compound or a pharmaceutically acceptable salt thereof of any one of items 1-6, wherein the cancer antigen peptide A is a peptide comprising an amino acid sequence selected from the amino acid sequences:

RMFPNAPYL (SEQ ID NO: 2),
CMTWNQMNL (SEQ ID NO: 3),
ALLPAVPSL (SEQ ID NO: 5),
SLGEQQYSV (SEQ ID NO: 6), and
RVPGVAPTL (SEQ ID NO: 7)

or a peptide comprising an amino acid sequence that differs from the amino acid sequence selected from SEQ ID NOS: 2, 3, 5, 6 and 7 by alteration of an amino acid residue and having an ability to induce CTLs.

8. The compound or a pharmaceutically acceptable salt thereof of any one of items 1-7, wherein the cancer antigen peptide A is a peptide consisting of an amino acid sequence selected from the amino acid sequences:

RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6), and
RVPGVAPTL (SEQ ID NO: 7).

9. The compound or a pharmaceutically acceptable salt thereof of any one of items 1-8, wherein $R^1$ is hydrogen.

10. The compound or a pharmaceutically acceptable salt thereof of any one of items 1-9, wherein the compound of formula (1) is a peptide consisting of an amino acid sequence selected from the amino acid sequences:

CRMFPNAPYL (SEQ ID NO: 8),
CCMTWNQMNL (SEQ ID NO: 9),
CCYTWNQMNL (SEQ ID NO: 10),
CALLPAVPSL (SEQ ID NO: 11),
CSLGEQQYSV (SEQ ID NO: 12), and
CRVPGVAPTL (SEQ ID NO: 13).

11. The compound or a pharmaceutically acceptable salt thereof of any one of items 1-8, wherein $R^1$ is the group of formula (2).

12. The compound or a pharmaceutically acceptable salt thereof of any one of items 1-8 and 11, wherein $X^b$ is a divalent peptide group consisting of 2 amino acid residues, and $Y^b$ is a single bond; $X^b$ and $Y^b$ are independently divalent peptide groups consisting of 1 amino acid residue; $X^b$ is a single bond, and $Y^b$ is a divalent peptide group consisting of 2 amino acid residues; $X^b$ is a divalent peptide group consisting of 1 amino acid residue, and $Y^b$ is a single bond; $X^b$ is a single bond, and $Y^b$ is a divalent peptide group consisting of 1 amino acid residue; or $X^b$ and $Y^b$ are single bonds.
13. The compound or a pharmaceutically acceptable salt thereof of any one of items 1-8 and 11-12, wherein $X^b$ is a single bond, and $Y^b$ is a single bond, an alanine residue, a leucine residue or a methionine residue.
14. The compound or a pharmaceutically acceptable salt thereof of any one of items 1-8 and 11-12, wherein $X^b$ is a single bond or a divalent peptide group consisting of 1 amino acid residue, and $Y^b$ is a single bond.
15. The compound or a pharmaceutically acceptable salt thereof of any one of items 1-8 and 11-14, wherein $X^b$ and $Y^b$ are single bonds.
16. The compound or a pharmaceutically acceptable salt thereof of any one of items 1-8 and 11-12, wherein $X^b$ is a divalent peptide group consisting of 1 to 3 amino acid residues.
17. The compound or a pharmaceutically acceptable salt thereof of any one of items 1-8, 11-12 and 16, wherein $X^b$ is a divalent peptide group consisting of 1 amino acid residue, and $Y^b$ is a single bond.
18. The compound or a pharmaceutically acceptable salt thereof of item 16 or 17, wherein $X^b$ is a divalent peptide group consisting of an alanine residue, an arginine residue, an asparagine residue, an aspartic acid residue, a cysteine residue, a glutamine residue, a glutamic acid residue, a glycine residue, a histidine residue, an isoleucine residue, a leucine residue, a lysine residue, a methionine residue, a phenylalanine residue, a proline residue, a serine residue, a threonine residue, a tryptophan residue, a tyrosine residue or a valine residue.
19. The compound or a pharmaceutically acceptable salt thereof of item 18, wherein $X^b$ is a divalent peptide group consisting of an arginine residue, a glutamine residue, a glutamic acid residue, a histidine residue, a lysine residue, a phenylalanine residue or a tyrosine residue.
20. The compound or a pharmaceutically acceptable salt thereof of any one of items 1-8 and 11-19, wherein the cancer antigen peptide B is an MHC class I-restricted WT1 peptide consisting of 7 to 15 amino acid residues.
21. The compound or a pharmaceutically acceptable salt thereof of any one of items 1-8 and 11-20, wherein the cancer antigen peptide B is a peptide comprising an amino acid sequence selected from the amino acid sequences:

RMFPNAPYL (SEQ ID NO: 2),
CMTWNQMNL (SEQ ID NO: 3),
ALLPAVPSL (SEQ ID NO: 5),
SLGEQQYSV (SEQ ID NO: 6), and
RVPGVAPTL (SEQ ID NO: 7)

or a peptide comprising an amino acid sequence that differs from the amino acid sequence selected from SEQ ID NOS: 2, 3, 5, 6 and 7 by alteration of an amino acid residue and having an ability to induce CTLs.
22. The compound or a pharmaceutically acceptable salt thereof of any one of items 1-8 and 11-21, wherein the cancer antigen peptide B is a peptide consisting of an amino acid sequence selected from the amino acid sequences:

RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6), and
RVPGVAPTL (SEQ ID NO: 7).

23. The compound or a pharmaceutically acceptable salt thereof of any one of items 1-8 and 11-22, wherein the compound of formula (1) is a compound of formula (3):

CRMFPNAPYL
|
CSLGEQQYSV (3)

wherein C-C shown in the formula means that the C residues are linked together by a disulfide bond.
24. The compound or a pharmaceutically acceptable salt thereof of any one of items 1-8, wherein $R^1$ is the

cancer antigen peptide C.

25. The compound or a pharmaceutically acceptable salt thereof of any one of items 1-8 and 24, wherein the cancer antigen peptide C is an MHC class I-restricted WT1 peptide consisting of 7 to 15 amino acid residues.

26. The compound or a pharmaceutically acceptable salt thereof of any one of items 1-8 and 24-25, wherein the cancer antigen peptide C is a peptide comprising the amino acid sequence:
CMTWNQMNL (SEQ ID NO: 3)
or a peptide comprising an amino acid sequence that differs from the amino acid sequence of SEQ ID NO: 3 by alteration of an amino acid residue and having an ability to induce CTLs.

27. The compound or a pharmaceutically acceptable salt thereof of any one of items 1-8 and 24-26, wherein the cancer antigen peptide C is a peptide consisting of an amino acid sequence selected from the amino acid sequences:

CMTWNQMNL (SEQ ID NO: 3), and
CYTWNQMNL (SEQ ID NO: 4)

28. The compound or a pharmaceutically acceptable salt thereof of any one of items 1-8 and 24-27, wherein the compound of formula (1) is a compound of formula (4):

**CRMFPNAPYL**
**|**
**CMTWNQMNL** **(4)**

wherein C-C shown in the formula means that the C residues are linked together by a disulfide bond,
or a compound of formula (5):

**CRMFPNAPYL**
**|**
**CYTWNQMNL** **(5)**

wherein C-C shown in the formula means that the C residues are linked together by a disulfide bond.

29. The compound or a pharmaceutically acceptable salt thereof of any one of items 1-8 and 24, wherein the cancer antigen peptide C is an MHC class II-restricted WT1 peptide consisting of 14 to 30 amino acid residues.

30. The compound or a pharmaceutically acceptable salt thereof of any one of items 1-8, 24 and 29, wherein the cancer antigen peptide C is a peptide comprising an amino acid sequences selected from the amino acid sequences:

SGQARMFPNAPYLPSC (SEQ ID NO: 14),
SGQARMFPNAPYLPSCLES (SEQ ID NO: 15),
PGCNKRYFKLSHLQMHSRK (SEQ ID NO: 16),
PGCNKRYFKLSHLQMHSRKH (SEQ ID NO: 17),
PGCNKRYFKLSHLQMHSRKHTG (SEQ ID NO: 18),
CNKRYFKLSHLQMHSRK (SEQ ID NO: 19),
CNKRYFKLSHLQMHSRKH (SEQ ID NO: 20), and
CNKRYFKLSHLQMHSRKHTG (SEQ ID NO: 21)

or a peptide comprising an amino acid sequence that differs from the amino acid sequence selected from SEQ ID NOS: 14 to 21 by alteration of an amino acid residue and having an ability to induce helper T cells.

31. The compound or a pharmaceutically acceptable salt thereof of any one of items 1-8, 24 and 29-30, wherein the cancer antigen peptide C is a peptide consisting of an amino acid sequence selected from the amino acid sequences:

SGQARMFPNAPYLPSC (SEQ ID NO: 14),
SGQAYMFPNAPYLPSC (SEQ ID NO: 22),
SGQARMFPNAPYLPSCLES (SEQ ID NO: 15),
SGQAYMFPNAPYLPSCLES (SEQ ID NO: 23),
PGCNKRYFKLSHLQMHSRK (SEQ ID NO: 16),
PGCNKRYFKLSHLQMHSRKH (SEQ ID NO: 17),
PGCNKRYFKLSHLQMHSRKHTG (SEQ ID NO: 18),

CNKRYFKLSHLQMHSRK (SEQ ID NO: 19),
CNKRYFKLSHLQMHSRKH (SEQ ID NO: 20), and
CNKRYFKLSHLQMHSRKHTG (SEQ ID NO: 21).

32. The compound or a pharmaceutically acceptable salt thereof of any one of items 1-8, 24 and 29-31, wherein the compound of formula (1) is
a compound of formula (6):

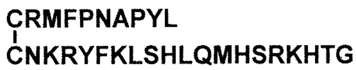

(6)

wherein C-C shown in the formula means that the C residues are linked together by a disulfide bond,
a compound of formula (7):

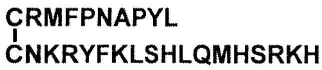

(7)

wherein C-C shown in the formula means that the C residues are linked together by a disulfide bond,
a compound of formula (8):

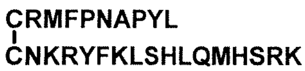

(8)

wherein C-C shown in the formula means that the C residues are linked together by a disulfide bond, or
a compound of formula (9):

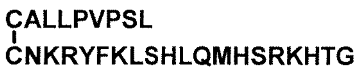

(9)

wherein C-C shown in the formula means that the C residues are linked together by a disulfide bond.
33. A pharmaceutical composition comprising the compound or a pharmaceutically acceptable salt thereof of any one of items 1-32, and a pharmaceutically acceptable carrier.
34. The pharmaceutical composition of item 33, wherein the pharmaceutical composition is for use as a cancer vaccine.
35. Use of the compound or a pharmaceutically acceptable salt thereof of any one of items 1-32 for the manufacture of a cancer vaccine.
36. A method of treating or preventing a cancer, comprising administering to a WT1-positive cancer patient in need thereof a therapeutically or prophylactically effective amount of the compound or a pharmaceutically acceptable salt thereof of any one of items 1-32.
37. A method for obtaining two different MHC class I-restricted epitopes, or an MHC class I-restricted epitope and an MHC class II-restricted epitope, comprising reacting the compound or a pharmaceutically acceptable salt thereof of any one of items 1-32 with ERAP1.

(2) The Second Embodiment

1. A compound represented by formula (1):

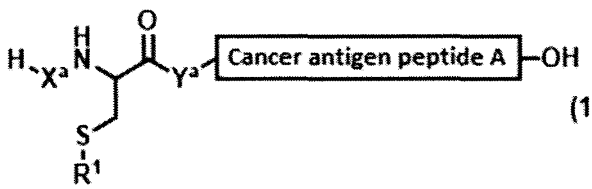

(1)

wherein each of $X^a$ and $Y^a$ represent a single bond;
cancer antigen peptide A is a peptide consisting of an amino acid sequence selected from

RMFPNAPYL (SEQ ID NO: 2),
ALLPAVPSL (SEQ ID NO: 5),
SLGEQQYSV (SEQ ID NO: 6), and
RVPGVAPTL (SEQ ID NO: 7)

wherein an amino group of an N-terminal amino acid of the cancer antigen peptide A binds to $Y^a$ in the formula (1), and a carbonyl group of a C-terminal amino acid of the cancer antigen peptide A binds to the hydroxyl group in the formula (1); and
$R^1$ represents cancer antigen peptide C,
wherein the cancer antigen peptide C is a peptide consisting of an amino acid sequence selected from

CMTWNQMNL (SEQ ID NO: 3), and
CYTWNQMNL (SEQ ID NO: 4),

provided that the cancer antigen peptide C has a different amino acid sequence from the cancer antigen peptide A, and the cysteine residue of the cancer antigen peptide C binds to the formula (1) via a disulfide bond,

or a pharmaceutically acceptable salt thereof.
2. The compound or a pharmaceutically acceptable salt thereof of item 1, wherein the compound of formula (1) is a compound of formula (4):

**CRMFPNAPYL**
**|**
**CMTWNQMNL** **(4)**

wherein C-C shown in the formula means that the C residues are linked together by a disulfide bond.
3. The compound or a pharmaceutically acceptable salt thereof of item 1, wherein the compound of formula (1) is a compound of formula (5):

**CRMFPNAPYL**
**|**
**CYTWNQMNL** **(5)**

wherein C-C shown in the formula means that the C residues are linked together by a disulfide bond.
4. A pharmaceutical composition comprising the compound or a pharmaceutically acceptable salt thereof of any one of items 1-3, and a pharmaceutically acceptable carrier.
5. The pharmaceutical composition of item 4, wherein the pharmaceutical composition comprises one or more peptides each consisting of an amino acid sequence selected from the group consisting of the amino acid sequences:

SGQARMFPNAPYLPSC (SEQ ID NO: 14),
SGQARMFPNAPYLPSCLES (SEQ ID NO: 15),
PGCNKRYFKLSHLQMHSRK (SEQ ID NO: 16),
PGCNKRYFKLSHLQMHSRKH (SEQ ID NO: 17),
PGCNKRYFKLSHLQMHSRKHTG (SEQ ID NO: 18),
CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21),
SGQAYMFPNAPYLPSC (SEQ ID NO: 22),
SGQAYMFPNAPYLPSCLES (SEQ ID NO: 23)
WAPVLDFAPPGASAYGSL (SEQ ID NO: 24),
CWAPVLDFAPPGASAYGSL (SEQ ID NO: 25),
WAPVLDFAPPGASAYGSLC (SEQ ID NO: 26), and
KRYFKLSHLQMHSRKH (SEQ ID NO: 68).

6. The pharmaceutical composition of item 5, wherein the pharmaceutical composition comprises one or more peptides each consisting of an amino acid sequence selected from the group consisting of the amino acid sequences:

CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21),
WAPVLDFAPPGASAYGSL (SEQ ID NO: 24),
CWAPVLDFAPPGASAYGSL (SEQ ID NO: 25), and
WAPVLDFAPPGASAYGSLC (SEQ ID NO: 26).

7. The pharmaceutical composition of item 5, wherein the pharmaceutical composition comprises one or more peptides each consisting of an amino acid sequence selected from the group consisting of the amino acid sequences:

PGCNKRYFKLSHLQMHSRK (SEQ ID NO: 16),
PGCNKRYFKLSHLQMHSRKH (SEQ ID NO: 17), and
PGCNKRYFKLSHLQMHSRKHTG (SEQ ID NO: 18).

8. A composition comprising a compound selected from the group consisting of a compound of formula (4):

CRMFPNAPYL
|
CMTWNQMNL (4)

wherein C-C shown in the formula means that the C residues are linked together by a disulfide bond,
and a compound of formula (5):

CRMFPNAPYL
|
CYTWNQMNL (5)

wherein C-C shown in the formula means that the C residues are linked together by a disulfide bond,
and one or more peptides each consisting of an amino acid sequence selected from the group consisting of the amino acid sequences:

SGQARMFPNAPYLPSC (SEQ ID NO: 14),
SGQARMFPNAPYLPSCLES (SEQ ID NO: 15),
PGCNKRYFKLSHLQMHSRK (SEQ ID NO: 16),
PGCNKRYFKLSHLQMHSRKH (SEQ ID NO: 17),
PGCNKRYFKLSHLQMHSRKHTG (SEQ ID NO: 18),
CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21),
SGQAYMFPNAPYLPSC (SEQ ID NO: 22),
SGQAYMFPNAPYLPSCLES (SEQ ID NO: 23)
WAPVLDFAPPGASAYGSL (SEQ ID NO: 24),
CWAPVLDFAPPGASAYGSL (SEQ ID NO: 25),
WAPVLDFAPPGASAYGSLC (SEQ ID NO: 26), and
KRYFKLSHLQMHSRKH (SEQ ID NO: 68).

9. The composition of item 8, wherein the composition comprises one or more peptides each consisting of an amino acid sequence selected from the group consisting of the amino acid sequences:

CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21),
WAPVLDFAPPGASAYGSL (SEQ ID NO: 24),

CWAPVLDFAPPGASAYGSL (SEQ ID NO: 25), and
WAPVLDFAPPGASAYGSLC (SEQ ID NO: 26).

10. The composition of item 8, wherein the composition comprises one or more peptides each consisting of an amino acid sequence selected from the group consisting of the amino acid sequences:

PGCNKRYFKLSHLQMHSRK (SEQ ID NO: 16),
PGCNKRYFKLSHLQMHSRKH (SEQ ID NO: 17), and
PGCNKRYFKLSHLQMHSRKHTG (SEQ ID NO: 18).

(3) The Third Embodiment

1. A compound consisting of 10 to 12 amino acids or a pharmaceutically acceptable salt thereof, wherein the compound comprises the amino acid sequence:

CMTWNQMNL (SEQ ID NO: 3), or
CYTWNQMNL (SEQ ID NO: 4),

and 1 to 3 amino acid residues attached to the amino group of the N-terminal cysteine residue of the amino acid sequence; or a pharmaceutically acceptable salt thereof.
2. The compound or a pharmaceutically acceptable salt thereof of item 1, wherein the compound consists of 10 to 12 amino acids and comprises the amino acid sequence of SEQ ID NO: 3.
3. The compound or a pharmaceutically acceptable salt thereof of item 1, wherein the compound consists of 10 to 12 amino acids and comprises the amino acid sequence of SEQ ID NO: 4.
4. The compound or a pharmaceutically acceptable salt thereof of any one of items 1-3, wherein the compound consists of 10 amino acids.
5. The compound or a pharmaceutically acceptable salt thereof of items 1-4, wherein the 1 to 3 amino acid residues attached to the amino group of the N-terminal cysteine residue are independently selected from an alanine residue, an arginine residue, an asparagine residue, a cysteine residue, a glutamine residue, a glutamic acid residue, a histidine residue, an isoleucine residue, a leucine residue, a lysine residue, a methionine residue, a phenylalanine residue, a proline residue, a serine residue, a threonine residue, a tryptophan residue, a tyrosine residue and a valine residue.
6. The compound or a pharmaceutically acceptable salt thereof of item 5, wherein the 1 to 3 amino acid residues attached to the amino group of the N-terminal cysteine residue are independently selected from an arginine residue, a glutamine residue, a glutamic acid residue, a histidine residue, a lysine residue, a phenylalanine residue and a tyrosine residue.
7. The compound or a pharmaceutically acceptable salt thereof of any one of items 1-6, wherein the compound or a pharmaceutically acceptable salt thereof is a peptide consisting of an amino acid sequence selected from the amino acid sequences:

RCMTWNQMNL (SEQ ID NO: 27),
RCYTWNQMNL (SEQ ID NO: 28),
QCMTWNQMNL (SEQ ID NO: 29),
QCYTWNQMNL (SEQ ID NO: 30),
ECMTWNQMNL (SEQ ID NO: 31),
ECYTWNQMNL (SEQ ID NO: 32),
HCMTWNQMNL (SEQ ID NO: 33),
HCYTWNQMNL (SEQ ID NO: 34),
KCMTWNQMNL (SEQ ID NO: 35),
KCYTWNQMNL (SEQ ID NO: 36),
FCMTWNQMNL (SEQ ID NO: 37),
FCYTWNQMNL (SEQ ID NO: 38),
YCMTWNQMNL (SEQ ID NO: 39), and
YCYTWNQMNL (SEQ ID NO: 40).

8. The compound or a pharmaceutically acceptable salt thereof of item 7, wherein the compound or a pharmaceutically acceptable salt thereof is a peptide consisting of an amino acid sequence selected from the amino acid sequences:

RCMTWNQMNL (SEQ ID NO: 27),
QCMTWNQMNL (SEQ ID NO: 29),
ECMTWNQMNL (SEQ ID NO: 31),
HCMTWNQMNL (SEQ ID NO: 33),
KCMTWNQMNL (SEQ ID NO: 35),
FCMTWNQMNL (SEQ ID NO: 37), and
YCMTWNQMNL (SEQ ID NO: 39).

9. The compound or a pharmaceutically acceptable salt thereof of item 7, wherein the compound or a pharmaceutically acceptable salt thereof is a peptide consisting of an amino acid sequence selected from the amino acid sequences:

RCYTWNQMNL (SEQ ID NO: 28),
QCYTWNQMNL (SEQ ID NO: 30),
ECYTWNQMNL (SEQ ID NO: 32),
HCYTWNQMNL (SEQ ID NO: 34),
KCYTWNQMNL (SEQ ID NO: 36),
FCYTWNQMNL (SEQ ID NO: 38), and
YCYTWNQMNL (SEQ ID NO: 40).

10. A compound of formula (1):

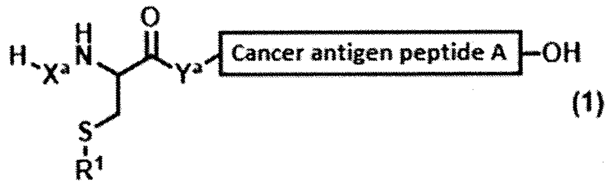

(1)

wherein $X^a$ and $Y^a$ independently represent a single bond or a divalent peptide group consisting of 1 to 4 amino acid residues, provided that the sum of the number of amino acid residues in $X^a$ and $Y^a$ is an integer of 0 to 4;
cancer antigen peptide A is an MHC class I-restricted WT1 peptide consisting of 7 to 30 amino acid residues, wherein an amino group of an N-terminal amino acid of the cancer antigen peptide A binds to $Y^a$ in the formula (1), and a carbonyl group of a C-terminal amino acid of the cancer antigen peptide A binds to the hydroxyl group in the formula (1); and
$R^1$ is cancer antigen peptide C,
wherein the cancer antigen peptide C is the compound of any one of items 1-9, and the cysteine residue in the compound binds to the formula (1) via a disulfide bond; or a pharmaceutically acceptable salt thereof.

11. The compound or a pharmaceutically acceptable salt thereof of item 10, wherein the cancer antigen peptide A is an MHC class I-restricted WT1 peptide consisting of 7 to 15 amino acid residues.
12. The compound or a pharmaceutically acceptable salt thereof of item 11, wherein the cancer antigen peptide A is a peptide comprising an amino acid sequences selected from the amino acid sequences:

RMFPNAPYL (SEQ ID NO: 2),
CMTWNQMNL (SEQ ID NO: 3),
ALLPAVPSL (SEQ ID NO: 5),
SLGEQQYSV (SEQ ID NO: 6), and
RVPGVAPTL (SEQ ID NO: 7)

or a peptide comprising an amino acid sequence that differs from the amino acid sequence selected from SEQ ID NOS: 2, 3, 5, 6 and 7 by alteration of 1 to 3 amino acid residues and having an ability to induce CTLs.
13. The compound or a pharmaceutically acceptable salt thereof of item 12, wherein the cancer antigen peptide A is a peptide comprising an amino acid sequence selected from the amino acid sequences:

RMFPNAPYL (SEQ ID NO: 2),
CMTWNQMNL (SEQ ID NO: 3),

ALLPAVPSL (SEQ ID NO: 5),
SLGEQQYSV (SEQ ID NO: 6), and
RVPGVAPTL (SEQ ID NO: 7)

or a peptide comprising an amino acid sequence that differs from the amino acid sequence selected from SEQ ID NOS: 2, 3, 5, 6 and 7 by alteration of 1 amino acid residue and having an ability to induce CTLs.
14. The compound or a pharmaceutically acceptable salt thereof of item 13, wherein the cancer antigen peptide A is a peptide consisting of an amino acid sequence selected from the amino acid sequences:

RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6), and
RVPGVAPTL (SEQ ID NO: 7).

15. The compound or a pharmaceutically acceptable salt thereof of any one of items 10-14, wherein $X^a$ and $Y^a$ are single bonds.
16. The compound or a pharmaceutically acceptable salt thereof of any one of items 10-15, wherein the compound of formula (1) is a compound of formula (10):

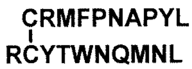 (10)

wherein C-C shown in the formula means that the C residues are linked together by a disulfide bond.
17. The compound or a pharmaceutically acceptable salt thereof of any one of items 10-15, wherein the compound of formula (1) is a compound of formula (11):

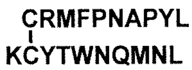 (11)

wherein C-C shown in the formula means that the C residues are linked together by a disulfide bond.
18. The compound or a pharmaceutically acceptable salt thereof of any one of items 10-15, wherein the compound of formula (1) is a compound of formula (12):

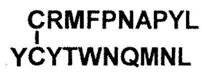 (12)

wherein C-C shown in the formula means that the C residues are linked together by a disulfide bond.
19. The compound or a pharmaceutically acceptable salt thereof of any one of items 10-15, wherein the compound of formula (1) is a compound of formula (13):

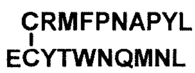 (13)

wherein C-C shown in the formula means that the C residues are linked together by a disulfide bond.
20. The compound or a pharmaceutically acceptable salt thereof of any one of items 10-15, wherein the compound of formula (1) is a compound of formula (14):

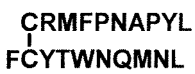 (14)

wherein C-C shown in the formula means that the C residues are linked together by a disulfide bond.

21. The compound or a pharmaceutically acceptable salt thereof of any one of items 10-15, wherein the compound of formula (1) is a compound of formula (15):

CRMFPNAPYL
HCYTWNQMNL (15)

wherein C-C shown in the formula means that the C residues are linked together by a disulfide bond.
22. A composition comprising the compound or a pharmaceutically acceptable salt thereof of any one of items 1-21, wherein the compound or a pharmaceutically acceptable salt thereof is used in combination with cancer antigen peptide D or a pharmaceutically acceptable salt thereof, and the cancer antigen peptide D is a peptide consisting of an amino acid sequence selected from the group consisting of the amino acid sequences:

SGQARMFPNAPYLPSC (SEQ ID NO: 14),
SGQARMFPNAPYLPSCLES (SEQ ID NO: 15),
PGCNKRYFKLSHLQMHSRK (SEQ ID NO: 16),
PGCNKRYFKLSHLQMHSRKH (SEQ ID NO: 17),
PGCNKRYFKLSHLQMHSRKHTG (SEQ ID NO: 18),
CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21),
SGQAYMFPNAPYLPSC (SEQ ID NO: 22),
SGQAYMFPNAPYLPSCLES (SEQ ID NO: 23)
WAPVLDFAPPGASAYGSL (SEQ ID NO: 24),
CWAPVLDFAPPGASAYGSL (SEQ ID NO: 25),
WAPVLDFAPPGASAYGSLC (SEQ ID NO: 26), and
KRYFKLSHLQMHSRKH (SEQ ID NO: 68).

23. The composition of item 22, wherein the cancer antigen peptide D is a peptide consisting of an amino acid sequence selected from the group consisting of the amino acid sequences:

CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21),
WAPVLDFAPPGASAYGSL (SEQ ID NO: 24),
CWAPVLDFAPPGASAYGSL (SEQ ID NO: 25), and
WAPVLDFAPPGASAYGSLC (SEQ ID NO: 26).

24. The composition of item 22, wherein the cancer antigen peptide D is a peptide consisting of an amino acid sequence selected from the group consisting of

WAPVLDFAPPGASAYGSL (SEQ ID NO: 24),
CWAPVLDFAPPGASAYGSL (SEQ ID NO: 25), and
WAPVLDFAPPGASAYGSLC (SEQ ID NO: 26).

25. The composition of item 22, wherein the cancer antigen peptide D is a peptide consisting of an amino acid sequence selected from the group consisting of

PGCNKRYFKLSHLQMHSRK (SEQ ID NO: 16),
PGCNKRYFKLSHLQMHSRKH (SEQ ID NO: 17), and
PGCNKRYFKLSHLQMHSRKHTG (SEQ ID NO: 18).

26. The composition of item 22, wherein the cancer antigen peptide D is WAPVLDFAPPGASAYGSL (SEQ ID NO: 24).
27. The composition of item 22, wherein the cancer antigen peptide D is CWAPVLDFAPPGASAYGSL (SEQ ID NO: 25).
28. The composition of item 22, wherein the cancer antigen peptide D is WAPVLDFAPPGASAYGSLC (SEQ ID NO: 26).

29. The composition of item 22, wherein the cancer antigen peptide D is PGCNKRYFKLSHLQMHSRK (SEQ ID NO: 16).
30. The composition of item 22, wherein the cancer antigen peptide D is PGCNKRYFKLSHLQMHSRKH (SEQ ID NO: 17).
31. The composition of item 22, wherein the cancer antigen peptide D is PGCNKRYFKLSHLQMHSRKHTG (SEQ ID NO: 18).
32. The composition of item 22, wherein the cancer antigen peptide D is KRYFKLSHLQMHSRKH (SEQ ID NO: 68).
33. The composition of item 22, wherein the compound of formula (1) is a compound of formula (10):

 CRMFPNAPYL
 |
RCYTWNQMNL (10)

wherein C-C shown in the formula means that the C residues are linked together by a disulfide bond, and the cancer antigen peptide D is WAPVLDFAPPGASAYGSL (SEQ ID NO: 24).
34. The composition of item 22, wherein the compound of formula (1) is a compound of formula (11):

 CRMFPNAPYL
 |
KCYTWNQMNL (11)

wherein C-C shown in the formula means that the C residues are linked together by a disulfide bond, and the cancer antigen peptide D is WAPVLDFAPPGASAYGSL (SEQ ID NO: 24).
35. The composition of item 22, wherein the compound of formula (1) is a compound of formula (12):

 CRMFPNAPYL
 |
YCYTWNQMNL (12)

wherein C-C shown in the formula means that the C residues are linked together by a disulfide bond, and the cancer antigen peptide D is WAPVLDFAPPGASAYGSL (SEQ ID NO: 24).
36. The composition of item 22, wherein the compound of formula (1) is a compound of formula (13):

 CRMFPNAPYL
 |
ECYTWNQMNL (13)

wherein C-C shown in the formula means that the C residues are linked together by a disulfide bond, and the cancer antigen peptide D is WAPVLDFAPPGASAYGSL (SEQ ID NO: 24).
37. The composition of item 22, wherein the compound of formula (1) is a compound of formula (14):

 CRMFPNAPYL
 |
FCYTWNQMNL (14)

wherein C-C shown in the formula means that the C residues are linked together by a disulfide bond, and the cancer antigen peptide D is WAPVLDFAPPGASAYGSL (SEQ ID NO: 24).
38. The composition of item 22, wherein the compound of formula (1) is a compound of formula (15):

 CRMFPNAPYL
 |
HCYTWNQMNL (15)

wherein C-C shown in the formula means that the C residues are linked together by a disulfide bond, and the cancer antigen peptide D is WAPVLDFAPPGASAYGSL (SEQ ID NO: 24).
39. The composition of item 22, wherein the compound of formula (1) is a compound of formula (10):

CRMFPNAPYL
|
RCYTWNQMNL (10)

wherein C-C shown in the formula means that the C residues are linked together by a disulfide bond, and the cancer antigen peptide D is PGCNKRYFKLSHLQMHSRKHTG (SEQ ID NO: 18).

40. The composition of item 22, wherein the compound of formula (1) is a compound of formula (11):

CRMFPNAPYL
|
KCYTWNQMNL (11)

wherein C-C shown in the formula means that the C residues are linked together by a disulfide bond, and the cancer antigen peptide D is PGCNKRYFKLSHLQMHSRKHTG (SEQ ID NO: 18).

41. The composition of item 22, wherein the compound of formula (1) is a compound of formula (12):

CRMFPNAPYL
|
YCYTWNQMNL (12)

wherein C-C shown in the formula means that the C residues are linked together by a disulfide bond, and the cancer antigen peptide D is PGCNKRYFKLSHLQMHSRKHTG (SEQ ID NO: 18).

42. The composition of item 22, wherein the compound of formula (1) is a compound of formula (13):

CRMFPNAPYL
|
ECYTWNQMNL (13)

wherein C-C shown in the formula means that the C residues are linked together by a disulfide bond, and the cancer antigen peptide D is PGCNKRYFKLSHLQMHSRKHTG (SEQ ID NO: 18).

43. The composition of item 22, wherein the compound of formula (1) is a compound of formula (14):

CRMFPNAPYL
|
FCYTWNQMNL (14)

wherein C-C shown in the formula means that the C residues are linked together by a disulfide bond, and the cancer antigen peptide D is PGCNKRYFKLSHLQMHSRKHTG (SEQ ID NO: 18).

44. The composition of item 22, wherein the compound of formula (1) is a compound of formula (15):

CRMFPNAPYL
|
HCYTWNQMNL (15)

wherein C-C shown in the formula means that the C residues are linked together by a disulfide bond, and the cancer antigen peptide D is PGCNKRYFKLSHLQMHSRKHTG (SEQ ID NO: 18).

45. The composition of any one of items 22-44, wherein the compound or a pharmaceutically acceptable salt thereof of any one of items 1-21 and the cancer antigen peptide D or a pharmaceutically acceptable salt thereof are formulated in separate compositions.

46. The composition of any one of items 22-44, wherein the compound or a pharmaceutically acceptable salt thereof of any one of items 1-21 and the cancer antigen peptide D or a pharmaceutically acceptable salt thereof are incorporated in a single composition.

47. A pharmaceutical composition comprising the compound or a pharmaceutically acceptable salt thereof of any one of items 1-21, or the composition of any one of items 22-46, and a pharmaceutically acceptable carrier.

48. The pharmaceutical composition of item 47, wherein the pharmaceutical composition comprises a compound of formula (10):

CRMFPNAPYL
RCYTWNQMNL (10)

wherein C-C shown in the formula means that the C residues are linked together by a disulfide bond, or a pharmaceutically acceptable salt thereof.

49. The pharmaceutical composition of item 47, wherein the pharmaceutical composition comprises a compound of formula (11):

CRMFPNAPYL
KCYTWNQMNL (11)

wherein C-C shown in the formula means that the C residues are linked together by a disulfide bond, or a pharmaceutically acceptable salt thereof.

50. The pharmaceutical composition of item 47, wherein the pharmaceutical composition comprises a compound of formula (12):

CRMFPNAPYL
YCYTWNQMNL (12)

wherein C-C shown in the formula means that the C residues are linked together by a disulfide bond, or a pharmaceutically acceptable salt thereof.

51. The pharmaceutical composition of item 47, wherein the pharmaceutical composition comprises a compound of formula (13):

CRMFPNAPYL
ECYTWNQMNL (13)

wherein C-C shown in the formula means that the C residues are linked together by a disulfide bond, or a pharmaceutically acceptable salt thereof.

52. The pharmaceutical composition of item 47, wherein the pharmaceutical composition comprises a compound of formula (14):

CRMFPNAPYL
FCYTWNQMNL (14)

wherein C-C shown in the formula means that the C residues are linked together by a disulfide bond, or a pharmaceutically acceptable salt thereof.

53. The pharmaceutical composition of item 47, wherein the pharmaceutical composition comprises a compound of formula (15):

CRMFPNAPYL
HCYTWNQMNL (15)

wherein C-C shown in the formula means that the C residues are linked together by a disulfide bond, or a pharmaceutically acceptable salt thereof.

54. The pharmaceutical composition of any one of items 47-53, wherein the cancer antigen peptide D is a peptide consisting of an amino acid sequence selected from the group consisting of:

WAPVLDFAPPGASAYGSL (SEQ ID NO: 24),
CWAPVLDFAPPGASAYGSL (SEQ ID NO: 25), and
WAPVLDFAPPGASAYGSLC (SEQ ID NO: 26).

55. The pharmaceutical composition of item 54, wherein the cancer antigen peptide D is WAPVLDFAPPGASAYGSL (SEQ ID NO: 24).
56. The pharmaceutical composition of item 54, wherein the cancer antigen peptide D is CWAPVLDFAPPGASAYGSL (SEQ ID NO: 25).
57. The pharmaceutical composition of item 54, wherein the cancer antigen peptide D is WAPVLDFAPPGASAYGSLC (SEQ ID NO: 26).
58. The pharmaceutical composition of any one of items 47-53, wherein the cancer antigen peptide D is a peptide consisting of an amino acid sequence selected from the group consisting of:

PGCNKRYFKLSHLQMHSRK (SEQ ID NO: 16),
PGCNKRYFKLSHLQMHSRKH (SEQ ID NO: 17), and
PGCNKRYFKLSHLQMHSRKHTG (SEQ ID NO: 18).

59. The pharmaceutical composition of item 58, wherein the cancer antigen peptide D is PGCNKRYFKLSHLQMHSRK (SEQ ID NO: 16).
60. The pharmaceutical composition of item 58, wherein the cancer antigen peptide D is PGCNKRYFKLSHLQMHSRKH (SEQ ID NO: 17).
61. The pharmaceutical composition of item 58, wherein the cancer antigen peptide D is PGCNKRYFKLSHLQMHSRKHTG (SEQ ID NO: 18).
62. The pharmaceutical composition of any one of items 47-53, wherein the cancer antigen peptide D is KRYFKLSHLQMHSRKH (SEQ ID NO: 68).
63. The pharmaceutical composition of item 47, wherein the pharmaceutical composition comprises a compound of formula (10):

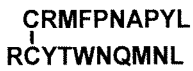
(10)

wherein C-C shown in the formula means that the C residues are linked together by a disulfide bond,
or a pharmaceutically acceptable salt thereof, and a peptide consisting of the amino acid sequence of WAPVLDFAPPGASAYGSL (SEQ ID NO: 24) or a pharmaceutically acceptable salt thereof.
64. The pharmaceutical composition of item 47, wherein the pharmaceutical composition comprises a compound of formula (11):

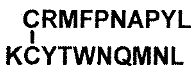
(11)

wherein C-C shown in the formula means that the C residues are linked together by a disulfide bond,
or a pharmaceutically acceptable salt thereof, and a peptide consisting of the amino acid sequence of WAPVLDFAPPGASAYGSL (SEQ ID NO: 24) or a pharmaceutically acceptable salt thereof.
65. The pharmaceutical composition of item 47, wherein the pharmaceutical composition comprises a compound of formula (12):

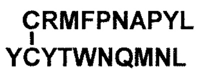
(12)

wherein C-C shown in the formula means that the C residues are linked together by a disulfide bond,
or a pharmaceutically acceptable salt thereof, and a peptide consisting of the amino acid sequence of WAPVLDFAPPGASAYGSL (SEQ ID NO: 24) or a pharmaceutically acceptable salt thereof.
66. The pharmaceutical composition of item 47 , wherein the pharmaceutical composition comprises a compound of formula (13):

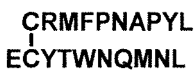
(13)

wherein C-C shown in the formula means that the C residues are linked together by a disulfide bond,
or a pharmaceutically acceptable salt thereof, and
a peptide consisting of the amino acid sequence of WAPVLDFAPPGASAYGSL (SEQ ID NO: 24) or a pharmaceutically acceptable salt thereof.

67. The pharmaceutical composition of item 47, wherein the pharmaceutical composition comprises a compound of formula (14):

CRMFPNAPYL
FCYTWNQMNL (14)

wherein C-C shown in the formula means that the C residues are linked together by a disulfide bond,
or a pharmaceutically acceptable salt thereof, and a peptide consisting of the amino acid sequence of WAPVLDFAPPGASAYGSL (SEQ ID NO: 24) or a pharmaceutically acceptable salt thereof.

68. The pharmaceutical composition of item 47, wherein the pharmaceutical composition comprises a compound of formula (15):

CRMFPNAPYL
HCYTWNQMNL (15)

wherein C-C shown in the formula means that the C residues are linked together by a disulfide bond,
or a pharmaceutically acceptable salt thereof, and a peptide consisting of the amino acid sequence of WAPVLDFAPPGASAYGSL (SEQ ID NO: 24) or a pharmaceutically acceptable salt thereof.

69. The pharmaceutical composition of item 47, wherein the pharmaceutical composition comprises a compound of formula (10):

CRMFPNAPYL
RCYTWNQMNL (10)

wherein C-C shown in the formula means that the C residues are linked together by a disulfide bond,
or a pharmaceutically acceptable salt thereof, and a peptide consisting of the amino acid sequence of PGCNKRYFKLSHLQMHSRKHTG (SEQ ID NO: 18) or a pharmaceutically acceptable salt thereof.

70. The pharmaceutical composition of item 47, wherein the pharmaceutical composition comprises a compound of formula (11) :

CRMFPNAPYL
KCYTWNQMNL (11)

wherein C-C shown in the formula means that the C residues are linked together by a disulfide bond,
or a pharmaceutically acceptable salt thereof, and a peptide consisting of the amino acid sequence of PGCNKRYFKLSHLQMHSRKHTG (SEQ ID NO: 18) or a pharmaceutically acceptable salt thereof.

71. The pharmaceutical composition of item 47, wherein the pharmaceutical composition comprises a compound of formula (12):

CRMFPNAPYL
YCYTWNQMNL (12)

wherein C-C shown in the formula means that the C residues are linked together by a disulfide bond,
or a pharmaceutically acceptable salt thereof, and a peptide consisting of the amino acid sequence of PGCNKRYFKLSHLQMHSRKHTG (SEQ ID NO: 18) or a pharmaceutically acceptable salt thereof.

72. The pharmaceutical composition of item 47, wherein the pharmaceutical composition comprises a compound of formula (13):

CRMFPNAPYL
|
ECYTWNQMNL (13)

wherein C-C shown in the formula means that the C residues are linked together by a disulfide bond,
or a pharmaceutically acceptable salt thereof, and a peptide consisting of the amino acid sequence of PGCNKRYFKLSHLQMHSRKHTG (SEQ ID NO: 18) or a pharmaceutically acceptable salt thereof.
73. The pharmaceutical composition of item 47, wherein the pharmaceutical composition comprises a compound of formula (14):

CRMFPNAPYL
|
FCYTWNQMNL (14)

wherein C-C shown in the formula means that the C residues are linked together by a disulfide bond,
or a pharmaceutically acceptable salt thereof, and
a peptide consisting of the amino acid sequence of PGCNKRYFKLSHLQMHSRKHTG (SEQ ID NO: 18) or a pharmaceutically acceptable salt thereof.
74. The pharmaceutical composition of item 47, wherein the pharmaceutical composition comprises a compound of formula (15):

CRMFPNAPYL
|
HCYTWNQMNL (15)

wherein C-C shown in the formula means that the C residues are linked together by a disulfide bond,
or a pharmaceutically acceptable salt thereof, and a peptide consisting of the amino acid sequence of PGCNKRYFKLSHLQMHSRKHTG (SEQ ID NO: 18) or a pharmaceutically acceptable salt thereof.
75. The pharmaceutical composition of any one of items 47-74, wherein the pharmaceutical composition is for use as a composition for treating a cancer associated with WT1 gene expression or an elevated level of WT1 gene expression.
76. The pharmaceutical composition of any one of items 47-74, wherein the pharmaceutical composition is for use as a composition for inducing CTLs in cellular immunotherapy for a cancer.
77. The pharmaceutical composition of any one of items 47-74, wherein the pharmaceutical composition is for use as a cancer vaccine.
78. The compound or a pharmaceutically acceptable salt thereof of any one of items 1-21, or the composition of any one of items 22-46, wherein the compound or a pharmaceutically acceptable salt thereof or the composition is for use in treatment or prevention of a cancer.
79. A kit for preventing or treating a cancer, wherein the kit comprises the compound or a pharmaceutically acceptable salt thereof of any one of items 1-21, or the composition of any one of items 22-46, and a pharmaceutically acceptable carrier.
80. Use of the compound or a pharmaceutically acceptable salt thereof of any one of items 1-21, or the composition of any one of items 22-46, for the manufacture of a cancer vaccine.
81. A method of treating or preventing a cancer, comprising administering to a patient in need thereof a therapeutically or prophylactically effective amount of the compound or a pharmaceutically acceptable salt thereof of any one of items 1-21, or the composition of any one of items 22-46.
82. The method of item 81, wherein the patient is WT1-positive.
83. The compound or a pharmaceutically acceptable salt thereof, the composition, the pharmaceutical composition, the kit, the use or the method of any one of items 75-82, wherein the cancer is selected from the group consisting of leukemia, myelodysplastic syndrome, multiple myeloma, malignant lymphoma, gastric cancer, colorectal cancer, lung cancer, breast cancer, germ cell cancer, liver cancer, skin cancer, urinary bladder cancer, prostate cancer, uterine cancer, cervical cancer, ovarian cancer, brain tumor, bone cancer, pancreatic cancer, cancer of the head or neck, cutaneous or intraocular malignant melanoma, rectal cancer, cancer of the anal region, testicular cancer, carcinoma of the fallopian tubes, carcinoma of the endometrium, carcinoma of the cervix, carcinoma of the vagina, carcinoma of the vulva, Hodgkin’s Disease, non-Hodgkin’s lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia such as acute myeloid leukemia, chronic myeloid leukemia,

acute lymphoblastic leukemia, or chronic lymphocytic leukemia, childhood solid tumor, lymphocytic lymphoma, cancer of the kidney or ureter, carcinoma of the renal pelvis, central nervous system (CNS) tumor, primary CNS lymphoma, tumor angiogenesis, spinal tumor, brainstem glioma, pituitary adenoma, Kaposi's sarcoma, epidermoid cancer, squamous cell cancer, T-cell lymphoma, glioblastoma multiforme, malignant melanoma, non-small cell lung cancer, renal cell carcinoma, and asbestos-induced cancer.

Effects of Invention

The present disclosure provides new WT1 peptides, peptide conjugates containing the same, and combinations of the WT1 peptides or the peptide conjugates and WT1 helper peptides, for example. Thus, the present disclosure provides a cancer vaccine or a composition for cancer immunotherapy that induces CTLs efficiently.

Brief Description of Drawings

Figure 1 shows *in vivo* CTL induction in an HLA-A*24:02 transgenic mouse by the compound of formula (5) synthesized in Reference Example 1 and the compound of formula (15) synthesized in Example 21 in an IFNγ ELISPOT assay of Experimental Example 1.
Figure 2 shows *in vivo* CTL induction in an HLA-A*24:02 transgenic mouse by the compound of formula (5) synthesized in Reference Example 1 and the compound of formula (10) synthesized in Example 22 in an IFNγ ELISPOT assay of Experimental Example 2.
Figure 3 shows *in vivo* CTL induction in an HLA-A*24:02 transgenic mouse by the compound of formula (5) synthesized in Reference Example 1 and the compound of formula (11) synthesized in Example 23 in an IFNγ ELISPOT assay of Experimental Example 3.
Figure 4 shows *in vivo* CTL induction in an HLA-A*24:02 transgenic mouse by the compound of formula (5) synthesized in Reference Example 1 and the compound of formula (13) synthesized in Example 24 in an IFNγ ELISPOT assay of Experimental Example 4.
Figure 5 shows *in vivo* CTL induction in an HLA-A*24:02 transgenic mouse by the compound of formula (5) synthesized in Reference Example 1 and the compound of formula (12) synthesized in Example 25 in an IFNγ ELISPOT assay of Experimental Example 5.
Figure 6 shows *in vivo* CTL induction in an HLA-A*24:02 transgenic mouse by the compound of formula (5) synthesized in Reference Example 1 and the compound of formula (14) synthesized in Example 26 in an IFNγ ELISPOT assay of Experimental Example 6.
Figure 7 shows *in vivo* CTL induction in an HLA-A*24:02 transgenic mouse by the peptide of SEQ ID NO: 34 synthesized in Example 1 in an IFNγ ELISPOT assay of Experimental Example 25.
Figure 8 shows *in vivo* CTL induction in an HLA-A*24:02 transgenic mouse by the peptide of SEQ ID NOS: 32, 38, 36, 30, 28 and 40 synthesized in Examples 5, 6, 9, 14, 15 and 20 in an IFNγ ELISPOT assay of Experimental Examples 26-31.
Figure 9 shows *in vivo* CTL induction in an HLA-A*24:02 transgenic mouse by the peptide of SEQ ID NO: 24 synthesized in Reference Example 6 or a cocktail vaccine of the peptide of SEQ ID NO: 24 and the compound of formula (11) synthesized in Example 23 in an IFNγ ELISPOT assay of Experimental Example 32.
Figure 10 shows *in vivo* CTL induction in an HLA-A*24:02 transgenic mouse by the peptide of SEQ ID NO: 24 synthesized in Reference Example 6 or a cocktail vaccine of the peptide of SEQ ID NO: 24 and the compound of formula (15) synthesized in Example 21 in an IFNγ ELISPOT assay of Experimental Example 33.
Figure 11 shows *in vivo* CTL induction in an HLA-A*24:02 transgenic mouse by the peptide of SEQ ID NO: 18 synthesized in Reference Example 7 or a cocktail vaccine of the peptide of SEQ ID NO: 18 and the compound of formula (11) synthesized in Example 23 in an IFNγ ELISPOT assay of Experimental Example 34.

Description of Embodiments

Embodiments of the present application are described in detail below.
An "amino acid residue" as used herein refers to a single amino acid unit among amino acids constituting a peptide or protein molecule. An "amino acid residue" may be a natural or non-natural α-amino acid residue, β-amino acid residue, γ-amino acid residue or δ-amino acid residue, more specifically, a natural α-amino acid residue, ornithine residue, homoserine residue, homocysteine residue, β-alanine, γ-aminobutanoic acid or δ-aminopentanoic acid. When an "amino acid residue" is optically active, it includes L-form and D-form, and is preferably L-form.
For describing an "amino acid residue", an abbreviation for it may be used. The following is a list of the abbreviations:

Ala or A: alanine residue
Arg or R: arginine residue
Asn or N: asparagine residue
Asp or D: aspartic acid residue
Cys or C: cysteine residue
Gln or Q: glutamine residue
Glu or E: glutamic acid residue
Gly or G: glycine residue
His or H: histidine residue
Ile or I: isoleucine residue
Leu or L: leucine residue
Lys or K: lysine residue
Met or M: methionine residue
Phe or F: phenylalanine residue
Pro or P: proline residue
Ser or S: serine residue
Thr or T: threonine residue
Trp or W: tryptophan residue
Tyr or Y: tyrosine residue
Val or V: valine residue
Abu: 2-aminobutyric acid residue (also referred to as $\alpha$-aminobutyric acid residue)
Orn: ornithine residue
Cit: citrulline residue

The term "N-terminus" as used herein in relation to a peptide refers to the terminal of a peptide chain where the peptide chain has a free amino group ($-NH_2$). The free amino group may optionally be modified. The term "C-terminus" as used herein in relation to a peptide refers to the terminal of a peptide chain where the peptide chain has a free carboxyl group (-COOH). The free carboxyl group may optionally be modified.

An amino acid sequence of a "peptide" is described herein so that an N-terminal amino acid residue is positioned on the left side, and a C-terminal amino acid residue is positioned on the right side in accordance with a usual description method. Unless otherwise indicated, a "peptide" has a free amino group in its N-terminal amino acid residue, and a hydroxyl group attaching to a carbonyl group in its C-terminal amino acid residue. A divalent peptide group means a peptide group that is able to bind to other chemical moieties via the N-terminal amino group and via the C-terminal carbonyl group.

Unless otherwise indicated, a peptide corresponding to partial structure of a compound of formula (1), for example, the compound of formula (3) or formula (4), has a free amino group in its N-terminal amino acid residue, and a hydroxyl group attaching to a carbonyl group in its C-terminal amino acid residue.

The "amino acid sequence" of WT1 protein as used herein may be an amino acid sequence that differs from the natural amino acid sequence of WT1 protein by alteration of 1 to 5 amino acid residues. The amino acid sequence of WT1 protein may preferably be an amino acid sequence that differs by alteration of 1 to 3 amino acid residues, further preferably an amino acid sequence that differs by alteration of 1 to 2 amino acid residues, and most preferably an amino acid sequence that differs by alteration of 1 amino acid residue.

In formula (1), "$X^a$" and "$Y^a$" independently represents a single bond or a divalent peptide group consisting of 1 to 4 amino acid residues, provided that the sum of the number of amino acid residues in $X^a$ and $Y^a$ is an integer of 0 to 4. For example, when the sum of the number of amino acid residues in $X^a$ and $Y^a$ is an integer of 0, both $X^a$ and $Y^a$ must be single bonds; and when the sum of the number of amino acid residues in $X^a$ and $Y^a$ is an integer of 4, each of $X^a$ and $Y^a$ may be a divalent peptide group consisting of two amino acid residues, or $X^a$ may be a divalent peptide group consisting of three amino acid residues, and $Y^a$ may be a divalent peptide group consisting of one amino acid residue, or $X^a$ may be a divalent peptide group consisting of four amino acid residues, and $Y^a$ may be a single bond.

The sum of the number of amino acid residues in $X^a$ and $Y^a$ is preferably an integer of 0 to 2, more preferably an integer of 0 to 1, or most preferably zero. That is, most preferably, $X^a$ and $Y^a$ are both single bonds.

When the sum of the number of amino acid residues in $X^a$ and $Y^a$ is an integer of 2, $X^a$ may be a divalent peptide group consisting of 2 amino acid residues, and $Y^a$ may be a single bond; $X^a$ and $Y^a$ may independently be a divalent peptide group consisting of 1 amino acid residue; or $X^a$ may be a single bond, and $Y^a$ is a divalent peptide group consisting of 2 amino acid residues.

When the sum of the number of amino acid residues in $X^a$ and $Y^a$ is an integer of 1, $X^a$ may be a divalent

peptide group consisting of one amino acid residue, and $Y^a$ may be a single bond; or $X^a$ may be a single bond, and $Y^a$ may be a divalent peptide group consisting of one amino acid residue. In a preferred embodiment, $X^a$ is a single bond, and $Y^a$ is a residue of alanine, leucine or methionine; or $X^a$ is a residue of alanine, leucine or methionine, and $Y^a$ is a single bond.

"Cancer antigen peptide A" is an MHC class I-restricted WT1 peptide consisting of 7 to 30 amino acid residues. In formula (1), an amino group of an N-terminal amino acid of the cancer antigen peptide A binds to $Y^a$ in the formula (1), and a carbonyl group of a C-terminal amino acid of the cancer antigen peptide A binds to the hydroxyl group in the formula (1).

In formula (1), "$R^1$" is hydrogen, a group of formula (2), or cancer antigen peptide C. Preferably, $R^1$ is a group of formula (2), or cancer antigen peptide C.

When $R^1$ is hydrogen, the compound of formula (1) is a compound (peptide) of formula (1-1):

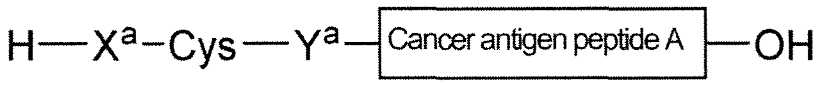


(1-1)

wherein $X^a$, $Y^a$ and cancer antigen peptide A have the same meanings as defined above in relation to formula (1), and Cys represents a cysteine residue.

When $R^1$ is hydrogen, that is, when the compound of formula (1) is a peptide of formula (1-1), the peptide is not a partial peptide of WT1 protein. When a peptide of formula (1) is described to be "not a partial peptide of WT1 protein", it is meant that the peptide of formula (1-1) is not a peptide consisting of contiguous amino acid residues in the amino acid sequence of human WT1 protein of SEQ ID NO: 1.

When $R^1$ is a group of formula (2), the compound of formula (1) is a compound of formula (1-2):

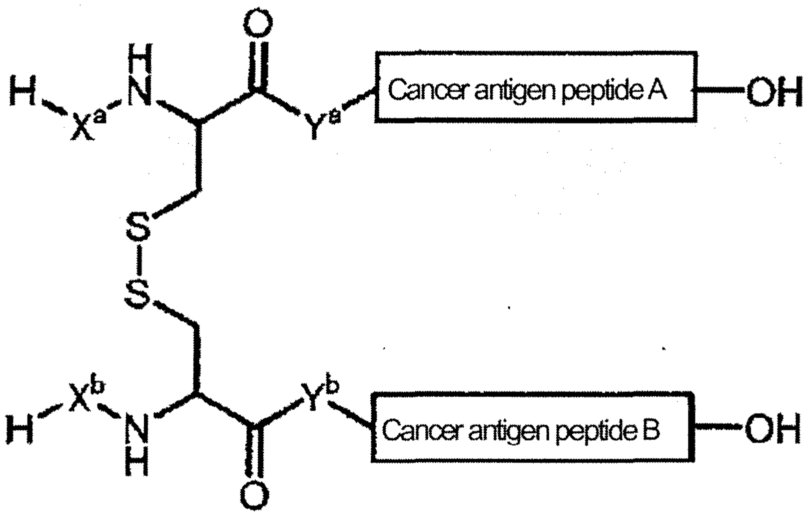


(1-2)

wherein $X^a$, $Y^a$ and cancer antigen peptide A have the same meanings as defined above in relation to formula (1), and $X^b$, $Y^b$ and cancer antigen peptide B have the same meanings as defined above in relation to formula (2).

"Cancer antigen peptide B" is an MHC class I-restricted WT1 peptide consisting of 7 to 30 amino acid residues. In formula (2) (or formula (1-2)), an amino group of an N-terminal amino acid of the cancer antigen peptide B binds to $Y^b$ in the formula (2), and a carbonyl group of a C-terminal amino acid of the cancer antigen peptide B binds to the hydroxyl group in the formula (2).

"$X^b$" And "$Y^b$" independently represent a single bond or a divalent peptide group consisting of 1 to 4 amino acid residues, provided that the sum of the number of amino acid residues in $X^b$ and $Y^b$ is an integer of 0 to 4. For example, when the sum of the number of amino acid residues in $X^b$ and $Y^b$ is an integer of 0, both $X^b$ and $Y^b$ must be single bonds; and when the sum of the number of amino acid residues in $X^b$ and $Y^b$ is an integer of 4, each of $X^b$ and $Y^b$ may be a divalent peptide group consisting of two amino acid residues, or $X^b$ may be a divalent peptide group consisting of three amino acid residues, and $Y^b$ may be a divalent peptide group consisting of one amino acid residue, or $X^b$ may be a divalent peptide group consisting of four amino acid residues, and $Y^b$ may be a single bond.

The sum of the number of amino acid residues in $X^b$ and $Y^b$ is preferably an integer of 0 to 2, more preferably an integer of 0 to 1, or most preferably zero. That is, most preferably, $X^b$ and $Y^b$ are both single bonds.

For example, when the sum of the number of amino acid residues in $X^b$ and $Y^b$ is an integer of 2, $X^b$ may be

a divalent peptide group consisting of two amino acid residues, and $Y^b$ may be a single bond, each of $X^b$ and $Y^b$ may independently be a divalent peptide group consisting of one amino acid residue, or $X^b$ may be a single bond, and $Y^b$ may be a divalent peptide group consisting of two amino acid residues.

When the sum of the number of amino acid residues in $X^b$ and $Y^b$ is an integer of 1, $X^b$ may be a divalent peptide group consisting of one amino acid residue, and $Y^b$ may be a single bond, or $X^b$ may be a single bond, and $Y^b$ may be a divalent peptide group consisting of one amino acid residue. In a preferred embodiment, $X^b$ is a single bond, and $Y^b$ is a residue of alanine, leucine or methionine, or $X^b$ is a residue of alanine, leucine or methionine, and $Y^b$ is a single bond.

"Cancer antigen peptide C" is an MHC class I-restricted WT1 peptide consisting of 7 to 30 amino acid residues including one cysteine residue, or an MHC class II-restricted WT1 peptide consisting of 7 to 30 amino acid residues including one cysteine residue. When $R^1$ is the cancer antigen peptide C, the cysteine residue of the cancer antigen peptide C binds to formula (1) via a disulfide bond. In the compound of formula (1), cancer antigen peptide C has a different amino acid sequence from cancer antigen peptide A.

Cancer antigen peptide C has least one cysteine residue in its amino acid sequence, wherein the number of cysteine residue is preferably 1 to 3, more preferably 1 to 2, or most preferably one.

In an embodiment, when $R^1$ is cancer antigen peptide B or cancer antigen peptide C, the compound of the formula (1) is not a homodimer but a heterodimer. In contrast to a homodimer, which means a dimerized product of the same peptide monomers, a heterodimer means a dimerized product of different peptide monomers.

"Cancer antigen peptide D" is an MHC class II-restricted WT1 peptide consisting of 7 to 30 amino acid residues.

As used herein, the term "WT1 peptide", which is synonymous with "partial peptide of WT1 protein", refers to a peptide consisting of contiguous amino acid residues of the amino acid sequence of human WT1 protein of SEQ ID NO: 1, or an altered peptide thereof.

The term "MHC class I-restricted" means an ability of a peptide to bind to a Major Histocompatibility Complex (MHC) class I molecule and induce CTLs.

MHC of human is called human leukocyte-type antigen (HLA). HLA Molecules corresponding to MHC class I-molecules include HLA-A, B, Cw, F and G subtypes. Restriction to HLA-A, HLA-B, or HLA-Cw is preferred as the restriction to MHC class I of an "MHC class I-restricted" peptide.

Allelic polymorphism is known for each HLA subtype. For HLA-A, 27 or more types of polymorphism including HLA-A1, HLA-A0201, and HLA-A24 are known. For HLA-B, 59 or more types of polymorphism including HLA-B7, HLA-B40, and HLA-B4403 are known. For HLA-Cw, 10 or more types of polymorphism including HLA-Cw0301, HLA-Cw0401, and HLA-Cw0602 are known. Among such polymorphism, HLA-A0201 and HLA-A24 are preferred.

The term "MHC class I-restricted WT1 peptide" as used herein refers to a WT1 peptide being capable of binding to an MHC class I molecule *in vitro* and/or *in vivo* to form a complex that is recognizable by precursor T cells. Upon recognizing the complex, precursor T cells differentiate into CTLs. (Thus, an MHC class I-restricted WT1 peptide has an ability to induce CTLs.) The "MHC class I-restricted WT1 peptide" is often herein referred to as "WT1 killer peptide". The "MHC class I-restricted WT1 peptide" may consists of a sequence of any number of amino acids of any type, so long as it functions as the "MHC class I-restricted WT1 peptide" as defined above. However, the longer a peptide chain is, the more susceptible it may be to degradation by a proteolytic enzyme. Also, too small peptide may not successfully be caught in a peptide-binding groove of an MHC class I molecule. The "MHC class I-restricted WT1 peptide" typically consists of 7 to 30 amino acid residues, preferably 7 to 15 amino acid residues, more preferably 8 to 12 amino acid residues, still more preferably 8 to 11 amino acid residues, or most preferably 8 or 9 amino acid residues.

The "MHC class I-restricted WT1 peptide" includes such a peptide that may be degraded *in vivo* or *in vitro* by proteasome and/or protease, and/or be cut or trimmed with ERAP1 to an appropriate peptide length, to provide an "MHC class I-restricted epitope". The term "MHC class I-restricted WT1 epitope" refers to a peptide corresponding to an actual peptide complexed with an MHC class I molecule and presented. That is, the term "MHC class I-restricted WT1 peptide" includes a peptide that produces a peptide being capable of binding to an MHC class I molecule through a process such as degradation or trimming *in vitro* or *in vivo* (that is, a peptide that binds to an MHC class I molecule after a process such as degradation or trimming *in vitro* or *in vivo*).

An "MHC class I-restricted epitope" may be derived from an "MHC class I-restricted WT1 peptide" by degradation with proteasome and/or protease, followed by trimming (cutting) with ERAP1, wherein a C-terminal amino acid residue and an N-terminal amino acid residue of the "MHC class I-restricted epitope" may be determined by the action of proteasome/protease and ERAP1, respectively. However, an "MHC class I-restricted epitope" may be derived by any other way. ERAP1, which is also referred to as ERAAP (ER aminopeptidase associated with antigen presentation), was formerly called A-LAP, PILS-AP or ARTS-1.

Accordingly, when an amino acid(s) is attached to an "MHC class I-restricted epitope", it is preferable to be attached to the C-terminus of the epitope. The "MHC class I-restricted epitope" typically consists of 7 to 12 residues, and preferably 9 amino acid residues. In an embodiment, the "MHC class I-restricted WT1 peptide" is a peptide consisting of 8 to 30 amino acid residues wherein 1 to 23 amino acid residues are attached to a "MHC class I-restricted epitope"

consisting of 7 to 12 residues, preferably 9 amino acid residues, via its C-terminal carbonyl group.

The "MHC class I-restricted WT1 peptide" may preferably be a peptide consisting of an amino acid sequence selected from:

RMFPNAPYL (SEQ ID NO: 2),
CMTWNQMNL (SEQ ID NO: 3),
ALLPAVPSL (SEQ ID NO: 5),
SLGEQQYSV (SEQ ID NO: 6),
RVPGVAPTL (SEQ ID NO: 7), and
VLDFAPPGA (SEQ ID NO: 41),

or a peptide consisting of an amino acid sequence that differs from the amino acid sequence selected from SEQ ID NOS: 2, 3, 5, 6, 7 and 41 by alteration of one or several amino acid residues. A peptide consisting of an amino acid sequence selected from SEQ ID NOS: 2, 3, 5, 6, 7 and 41 is also preferable.

As used herein, the term "peptide comprising an amino acid sequence" refers to a peptide having a particular amino acid sequence, which may, as usually understood, optionally have extra sequence(s) of amino acid residue(s) attached to the N-terminal and/or C-terminal amino acid of the particular sequence. An "MHC class I-restricted WT1 peptide" as "cancer antigen peptide A" or "cancer antigen peptide B" may have an extra sequence of amino acid residue(s) attached preferably to its C-terminus.

As used herein, the term "altered peptide" refers to a peptide consisting of an amino acid sequence that differs from the amino acid sequence of the original peptide by alteration of one or several amino acid residues. In an altered peptide, one or several amino acid residues, for example, 1 to 9 amino acid residues, preferably 1 to 5, 1 to 4 or 1 to 3 amino acid residues, more preferably 1 to 2 amino acid residues, or most preferably one amino acid residue is deleted from, substituted in, and/or added (or inserted) to an amino acid sequence that differs from an amino acid sequence of an original peptide. The number of amino acid(s) deleted from an original peptide may preferably be 1 to 5, 1 to 4, or 1 to 3, more preferably 1 to 2, or most preferably one. The number of amino acid(s) added (or inserted) to an original peptide may preferably be 1 to 5, 1 to 4, or 1 to 3, more preferably 1 to 2, or most preferably one. Amino acid substitution for altering a peptide may be made at any position of amino acid residue in an original sequence with any type of amino acid. Conservative amino acid substitution is preferred. For example, substitution of Asp for Glu; Tyr for Phe; Ile for Leu; Ser for Ala; or Arg for His may be made. Amino acid addition or deletion may preferably be made at N- or C-terminus of a peptide. However, amino acid addition or deletion may be made internally. Amino acid addition or substitution may be added (or inserted) to, or replaced with any of the twenty genetically encoded amino acids or even any non-natural amino acid.

As used herein, a peptide having an ability to induce CTLs is referred to as a killer peptide, and a peptide having an ability to induce helper T cells is referred to as a helper peptide. As used herein, a killer peptide consisting of an altered amino acid sequence is also referred to especially as an "altered killer peptide", and a helper peptide consisting of an altered amino acid sequence is also referred to especially as an "altered helper peptide".

In an altered killer peptide, amino acid substitution may be made, specifically at amino acid position 1 (N-terminus), 2, 3 or 9 in a peptide consisting of nine amino acid residues. When an altered killer peptide has added (or inserted) amino acid residue(s), the number of added amino acid(s) is preferably 1 or 2, or more preferably one. Amino acid addition to N-terminus or C-terminus is preferred. When a killer peptide is altered by amino acid deletion, the number of deleted amino acid(s) is preferably one.

In an aspect, an altered killer peptide is an altered peptide wherein 1 to 4 amino acid residues are attached to the N-terminus of a killer peptide. In an embodiment, the amino acid(s) to be added is independently selected from an alanine residue, an arginine residue, an asparagine residue, a cysteine residue, a glutamine residue, a glutamic acid residue, a histidine residue, an isoleucine residue, a leucine residue, a lysine residue, a methionine residue, a phenylalanine residue, a proline residue, a serine residue, a threonine residue, a tryptophan residue, a tyrosine residue, and a valine residue, preferable an arginine residue, a glutamine residue, a glutamic acid residue, a histidine residue, a lysine residue, a phenylalanine residue, and a tyrosine residue. Preferably 1 to 3 amino acid residues, further preferably 1 to 2 amino acid residues, and most preferably 1 amino acid residue are added.

In a preferred aspect, a WT1 killer peptide consisting of an altered amino acid sequence is a peptide consisting of 10 to 12 amino acids that comprises the amino acid sequence of SEQ ID NO: 3 or SEQ ID NO: 4 and 1 to 3 amino acid residues attached to the amino group of the N-terminal cysteine residue of the sequence.

The WT1 killer peptide wherein an amino acid is attached to the N-terminus may be, but not limited to, a peptide consisting of an amino acid sequence selected from the following amino acid sequences:

RCMTWNQMNL (SEQ ID NO: 27),
RCYTWNQMNL (SEQ ID NO: 28)

QCMTWNQMNL (SEQ ID NO: 29),
QCYTWNQMNL (SEQ ID NO: 30),
ECMTWNQMNL (SEQ ID NO: 31),
ECYTWNQMNL (SEQ ID NO: 32),
HCMTWNQMNL (SEQ ID NO: 33),
HCYTWNQMNL (SEQ ID NO: 34),
KCMTWNQMNL (SEQ ID NO: 35),
KCYTWNQMNL (SEQ ID NO: 36),
FCMTWNQMNL (SEQ ID NO: 37),
FCYTWNQMNL (SEQ ID NO: 38),
YCMTWNQMNL (SEQ ID NO: 39), and
YCYTWNQMNL (SEQ ID NO: 40).

Each HLA subtype carries polymorphism. Peptides that can complex with a polymorphic sequence of an HLA antigen have a specific pattern of amino acid sequence (that is, binding motif) for binding to the polymorphic sequence of the HLA antigen. For example, an HLA-A24-binding peptide that consists of 8 to 11 amino acid residues is known to have Tyr, Phe, Met or Trp at position 2, and Phe, Leu, Ile, Trp or Met at the C-terminus (J. Immunol., 152, p3913, 1994; J. Immunol., 155, p4307, 1994; Immunogenetics, 41, p178, 1995). Therefore, for example, a peptide consisting of nine amino acid residues may be altered by amino acid substitution to have Tyr, Phe, Met or Trp at position 2, and/or Phe, Leu, Ile, Trp or Met at position 9 to give an altered peptide useful as an altered killer peptide. Also, an HLA-A0201-binding peptide that consists of 8 to 11 amino acid residues is known to have Leu or Met at position 2, and Val or Leu at the C-terminus. Therefore, for example, a peptide consisting of nine amino acid residues may be altered by amino acid substitution to have Leu or Met at position 2, and/or Val or Leu at position 9 to give an altered peptide useful as an altered killer peptide.

Examples of the WT1 killer peptide consisting of an altered amino acid sequence include the following peptides.

RYFPNAPYL (SEQ ID NO: 2) as an altered killer peptide of
RMFPNAPYL (SEQ ID NO: 42) (WO 03/106682);
FMFPNAPYL (SEQ ID NO: 43),
RLFPNAPYL (SEQ ID NO: 44),
RMMPNAPYL (SEQ ID NO: 45),
RMFPNAPYV (SEQ ID NO: 46), or
YMFPNAPYL (SEQ ID NO: 47) (WO 2009/072610);
CYTWNQMNL (SEQ ID NO: 3) as an altered killer peptide of
CMTWNQMNL (SEQ ID NO: 4) (WO 02/79253);
Xaa-Met-Thr-Trp-Asn-Gln-Met-Asn-Leu (SEQ ID NO: 48), (wherein Xaa is Ser or Ala), or
Xaa-Tyr-Thr-Trp-Asn-Gln-Met-Asn-Leu (SEQ ID NO: 49) (wherein Xaa is Ser, Ala, Abu, Arg, Lys, Orn, Cit, Leu, Phe or Asn) (WO 2004/026897);
AYLPAVPSL (SEQ ID NO: 50) as an altered killer peptide of
ALLPAVPSL (SEQ ID NO: 5) (WO 2003/106682);
FLGEQQYSV (SEQ ID NO: 51),
SMGEQQYSV (SEQ ID NO: 52), or
SLMEQQYSV (SEQ ID NO: 53) as an altered killer peptide of
SLGEQQYSV (SEQ ID NO: 6) (WO 2009/072610); or
RYPGVAPTL (SEQ ID NO: 54) as an altered killer peptide of
RVPGVAPTL (SEQ ID NO: 7) (WO 2003/106682).

The term "MHC class II-restricted" means the ability of a peptide to bind to an MHC class II molecule and induce helper T cells.

HLA Corresponding to MHC class II-molecules has subtypes including HLA-DR, DQ and DP subtypes. Restriction to HLA-DR, HLA-DQ, or HLA-DP is preferred as the restriction to MHC class II of an "MHC class II-restricted" peptide. Restriction to a subtype selected from the following is more preferred: DRB1*0101, DRB1*0405, DRB1*0802, DRB1*0803, DRB1*0901, DRB1*1201, DRB1*1403, DRB1*1501, DRB1*1502, DPB1*0201, DPB1*0202, DPB1*0402, DPB1*0501, DPB1*0901, DQB1*0301, DQB1*0302, DQB1*0401, DQB1*0501, DQB1*0601, DQB1*0602 and DRB5*0102. Restriction to a subtype selected from DRB1*0101, DRB1*0405, DRB1*1502, DPB1*0201, DPB1*0202 and DQB1*0601 is most preferred.

The term "MHC class II-restricted WT1 peptide" as used herein refers to a WT1 peptide being capable of binding to an MHC class II molecule *in vitro* and/or *in vivo* and inducing helper T cells. (Thus, an MHC class II-restricted WT1

peptide has an ability to induce helper T cells.) The "MHC class II-restricted WT1 peptide" is often herein referred to as "WT1 helper peptide". The "MHC class II-restricted WT1 peptide" may consists of a sequence of any number of amino acids of any type, so long as it functions as the "MHC class II-restricted WT1 peptide" as defined above. However, the longer a peptide chain is, the more susceptible it may be to degradation by a proteolytic enzyme. Also, too small peptide may not successfully be caught in a peptide-binding groove of an MHC class II molecule. The "MHC class II-restricted WT1 peptide" typically consists of 7 to 30 amino acid residues, preferably 9 to 30 amino acid residues, more preferably 10 to 25 amino acid residues, still more preferably 12 to 24 amino acid residues, or most preferably 15 or 22 amino acid residues.

The "MHC class II-restricted WT1 peptide" includes such a peptide that may be degraded by proteasome and/or protease, and/or be cut or trimmed with Endoplasmic reticulum aminopeptidase 1 (ERAP1) to an appropriate peptide length, to provide an "MHC class I-restricted WT1 epitope". The term "MHC class II-restricted WT1 epitope" refers to a peptide corresponding to an actual peptide complexed with an MHC class II molecule and presented. That is, the term "MHC class II-restricted WT1 peptide" includes a peptide that produces a peptide being capable of binding to an MHC class II molecule through a process such as degradation or trimming *in vitro* or *in vivo* (that is, a peptide that binds to an MHC class II molecule after a process such as degradation or trimming *in vitro* or *in vivo*).

An MHC class II-restricted WT1 peptide may complex with an MHC class II molecule in any of HLA-DR, HLA-DQ or HLA-DP subclass. In a preferred embodiment, an MHC class II-restricted WT1 peptide induces helper T cells by binding to an MHC class II molecule selected from DRB1*0101, DRB1*0405, DRB1*0802, DRB1*0803, DRB1*0901, DRB1*1201, DRB1*1403, DRB1*1501, DRB1*1502, DPB1*0201, DPB1*0202, DPB1*0402, DPB1*0501, DPB1*0901, DQB1*0301, DQB1*0302, DQB1*0401, DQB1*0501, DQB1*0601, DQB1*0602 and DRB5*0102. More preferably, an MHC class II-restricted WT1 peptide induces helper T cells by binding to an MHC class II molecule selected from DRB1*0101, DRB1*0405, DRB1*1403, DRB1*1502, DPB1*0201, DPB1*0202, DPB1*0901, DQB1*0301, DQB1*0601 and DRB5*0102. Most preferably, an MHC class II-restricted WT1 peptide induces helper T cells by binding to an MHC class II molecule selected from DRB1*0101, DRB1*0405, DRB1*1502, DPB1*0201, DPB1*0202 and DQB1*0601.

When a helper peptide consisting of an amino acid sequence of nine amino acid residues including a binding motif to HLA-DRB1*0405 is altered by amino acid substitution, the substitution may preferably be made at positions 1, 4, 6 and/or 9. In a preferred embodiment, a helper peptide consisting of a sequence of nine amino acid residues including a binding motif to HLA-DRB1*0405 may be altered by amino acid substitution to have an amino acid residue (s) selected from:

phenylalanine, tryptophan, valine, isoleucine, leucine or methionine at position 1;
valine, isoleucine, methionine, aspartic acid and glutamic acid at position 4;
asparagine, serine, threonine, glutamine, lysine and aspartic acid at position 6; and/or
aspartic acid, glutamic acid and glutamine at position 9.

Examples of the "MHC class II-restricted WT1 peptide" include preferably a peptide comprising an amino acid sequences selected from the following amino acid sequences:

SGQARMFPNAPYLPSC (SEQ ID NO: 14),
SGQARMFPNAPYLPSCLES (SEQ ID NO: 15),
PGCNKRYFKLSHLQMHSRK (SEQ ID NO: 16),
PGCNKRYFKLSHLQMHSRKH (SEQ ID NO: 17),
PGCNKRYFKLSHLQMHSRKHTG (SEQ ID NO: 18),
CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21)
WAPVLDFAPPGASAYGSL (SEQ ID NO: 24),
CWAPVLDFAPPGASAYGSL (SEQ ID NO: 25), and
WAPVLDFAPPGASAYGSLC (SEQ ID NO: 26)

or a peptide comprising an amino acid sequence that differs from the amino acid sequence by alteration of an amino acid residue and having an ability to induce helper T cells; and further preferably a peptide consisting of an amino acid sequence selected from the following amino acid sequences:

SGQARMFPNAPYLPSC (SEQ ID NO: 14),
SGQAYMFPNAPYLPSC (SEQ ID NO: 22),
SGQARMFPNAPYLPSCLES (SEQ ID NO: 15),
SGQAYMFPNAPYLPSCLES (SEQ ID NO: 23),

PGCNKRYFKLSHLQMHSRK (SEQ ID NO: 16),
PGCNKRYFKLSHLQMHSRKH (SEQ ID NO: 17),
PGCNKRYFKLSHLQMHSRKHTG (SEQ ID NO: 18),
CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21)
WAPVLDFAPPGASAYGSL (SEQ ID NO: 24),
CWAPVLDFAPPGASAYGSL (SEQ ID NO: 25), and
WAPVLDFAPPGASAYGSLC (SEQ ID NO: 26).

In an embodiment, $R^1$ represents the cancer antigen peptide C, and the cancer antigen peptide C is a peptide consisting of an amino acid sequences selected from the following amino acid sequences:

RCMTWNQMNL (SEQ ID NO: 27),
RCYTWNQMNL (SEQ ID NO: 28)
QCMTWNQMNL (SEQ ID NO: 29),
QCYTWNQMNL (SEQ ID NO: 30),
ECMTWNQMNL (SEQ ID NO: 31),
ECYTWNQMNL (SEQ ID NO: 32),
HCMTWNQMNL (SEQ ID NO: 33),
HCYTWNQMNL (SEQ ID NO: 34),
KCMTWNQMNL (SEQ ID NO: 35),
KCYTWNQMNL (SEQ ID NO: 36),
FCMTWNQMNL (SEQ ID NO: 37),
FCYTWNQMNL (SEQ ID NO: 38),
YCMTWNQMNL (SEQ ID NO: 39), and
YCYTWNQMNL (SEQ ID NO: 40).

Examples of the compound of formula (1) include a compound of formula (4):

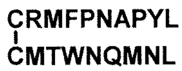
(4)

wherein C-C shown in the formula means that the C residues are linked together by a disulfide bond; a compound of formula (5):

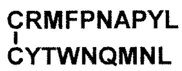
(5)

wherein C-C shown in the formula means that the C residues are linked together by a disulfide bond; a compound of formula (6):

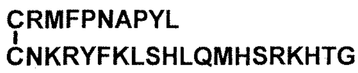
(6)

wherein C-C shown in the formula means that the C residues are linked together by a disulfide bond; a compound of formula (7):

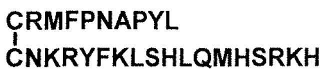
(7)

wherein C-C shown in the formula means that the C residues are linked together by a disulfide bond; a compound of formula (8):

CRMFPNAPYL
|
CNKRYFKLSHLQMHSRK (8)

wherein C-C shown in the formula means that the C residues are linked together by a disulfide bond;
a compound of formula (9):

CALLPVPSL
|
CNKRYFKLSHLQMHSRKHTG (9)

wherein C-C shown in the formula means that the C residues are linked together by a disulfide bond;
a compound of formula (10):

CRMFPNAPYL
|
RCYTWNQMNL (10)

wherein C-C shown in the formula means that the C residues are linked together by a disulfide bond;
a compound of formula (11):

CRMFPNAPYL
|
KCYTWNQMNL (11)

wherein C-C shown in the formula means that the C residues are linked together by a disulfide bond;
a compound of formula (12):

CRMFPNAPYL
|
YCYTWNQMNL (12)

wherein C-C shown in the formula means that the C residues are linked together by a disulfide bond;
a compound of formula (13):

CRMFPNAPYL
|
ECYTWNQMNL (13)

wherein C-C shown in the formula means that the C residues are linked together by a disulfide bond;
a compound of formula (14):

CRMFPNAPYL
|
FCYTWNQMNL (14)

wherein C-C shown in the formula means that the C residues are linked together by a disulfide bond;
a compound of formula (15):

CRMFPNAPYL
|
HCYTWNQMNL (15)

wherein C-C shown in the formula means that the C residues are linked together by a disulfide bond.

A peptide may have modification in amino acid residue(s) in its sequence. Modification may be made by a conventional way, for example by esterification, alkylation, halogenation, phosphorylation, sulfonation, or amidation on a functional group in an amino acid residue. Amino acid modification in a peptide can also be addition of an amino acid, a peptide, or an analog thereof, or other moiety to N-terminus and/or C-terminus of a peptide. A peptide modified by

such addition of a moiety may undergo, for example, biological enzymatic decomposition or intracellular processing, and be converted to a peptide capable of complexing with an MHC class I molecule or an MHC class II molecule. A peptide may be modified by addition of such a moiety that would modulate solubility of the peptide, stabilize the peptide against, for example, proteolytic degradation, direct the peptide to a specific tissue or organ, or improve capturing of the peptide by antigen presenting cells. A peptide may be altered by addition of a different peptide that may be a different killer or helper peptide.

In a peptide, an amino group of its N-terminal amino acid or a carboxyl group of its C-terminal amino acid may be modified. The amino group may be modified, for example by addition of one to three groups selected from $C_{1-6}$-alkyl, phenyl, cycloalkyl, or acyl such as $C_{1-6}$-alkanoyl, phanyl-$C_{1-6}$-alkanoyl, $C_{5-7}$-cycloalkyl-carbonyl, $C_{1-6}$-alkylsulfonyl, phenylsulfonyl, $C_{2-6}$-alkoxy-carbonyl, phenyl-alkoxycarbonyl, $C_{5-7}$-cycloalkoxy-carbonyl, or phenoxycarbonyl. The carboxyl group of the C-terminal amino acid may be modified, for example by conversion to an ester, such as a $C_{1-6}$-alkyl ester, a phenyl-$C_{0-6}$-alkyl ester, or a $C_{5-7}$-cycloalkyl ester, or to an amide such as an unsubstituted amide, a mono- or di-$C_{1-6}$-alkylamide, a mono- or di-phenyl-$C_{0-6}$-alkylamide, or a disubstituted amide wherein the two substituents forms together with the nitrogen atom they attach to a 5- to 7-membered azacycloalkane.

In a peptide, a bond between amino acid residues may be peptide bond or other type of bond such as carbon-carbon bond, carbon-nitrogen bond, or carbon-sulfur bond. A peptide as described herein may comprise one or more D-amino acid residues.

The above description about modification in a peptide is illustrative only, and variations thereof would be conceivable to a person skilled in the art. Such a modified peptide would be prepared, tested or used by an ordinarily skilled person in the art.

The ability of a peptide to induce CTLs or helper T cells can be confirmed by a conventional method. Induction of CTLs can be confirmed, for example by counting CTLs by HLA tetramer method (Int. J. Cancer: 100, 565-570 (2002)) or limiting dilution method (Nat. Med.: 4, 321-327 (1998)). Induction of CTLs by an HLA-A24-restricted peptide may also be confirmed by using an HLA-A24 mouse model as described in WO 02/47474 or Int. J. Cancer: 100, 565-570 (2002). Induction of helper T cells can be confirmed, for example by a method as described in Cancer Immunol. Immunother. 51: 271 (2002) or in the Example section herein.

In another aspect, the present disclosure provides a polynucleotide encoding a WT1 peptide or an altered WT1 peptide (hereinafter also referred to as WT1 polynucleotide). The polynucleotide of the present disclosure can be DNA or RNA. The polynucleotide has a nucleotide sequence corresponding to an amino acid sequence of a WT1 peptide or an altered WT1 peptide the polynucleotide encodes. The polynucleotide may be synthesized by a method for DNA or RNA synthesis, or PCR.

The present disclosure includes a polynucleotide that is able to hybridize to a complementary sequence of a polynucleotide encoding a WT1 peptide or an altered WT1 peptide under a stringent condition, and encodes a peptide having a similar ability of inducing CTLs or helper T cells to a WT1 peptide or an altered WT1 peptide as described herein. The hybridization under a stringent condition may be conventional hybridization as described in Sambrook J., Frisch E. F., Maniatis T., Molecular Cloning 2nd edition, Cold Spring Harbor Laboratory press. A "stringent condition" may be created, for example in a solution of 6 x SSC (in this regard, 10 x SSC contains 1.5 M NaCl, and 0.15 M trisodium citrate) with 50% formamide at 45°C for forming a hybrid, and in a solution of 2 x SSC for washing a hybrid (Molecular Biology, John Wiley & Sons, N.Y. (1989), 6.3.1-6.3.6).

In another aspect, the present disclosure provides an expression vector comprising a polynucleotide of the present disclosure (hereinafter also referred to as a WT1 expression vector). The expression vector may be of any appropriate type, and have any appropriate sequence outside a sequence of the polynucleotide of the present disclosure, depending on type of a host to be transfected with the vector or any other specific factors. The vector may be a plasmid, a phage vector, or a viral vector. For transfection to E. coli, a plasmid vector such as pUC118, pUC119, pBR322 or pCR3, or a phage vector such as λZAPII or λgt11 may be used. For transfection to yeast, pYES2 or pYEUra3 may be used. For transfection to insect cells, pAcSGHisNT-A may be used. For transfection to animal cells, a plasmid vector such as pKCR, pCDM8, pGL2, pcDNA3.1, pRc/RSV or pRc/CMV, or a viral vector such as a retroviral vector, an adenoviral vector, or an adeno-associated viral vector may be used. The vector of the present disclosure may further comprise a promotor for expression induction, a gene coding a signal sequence, a marker gene for selection, or a terminator. The vector of the present disclosure may also comprise a sequence encoding a tag such as thioredoxin, a His tag or GST (glutathione S-transferase) so that a protein is expressed with a tag fused thereon for facilitation of isolation or purification of the protein. Such a vector may comprise an appropriate promotor depending on a host to be transfected with the vector, and can be a GST-fused protein expression vector (for example pGEX4T), a vector comprising a sequence of a tag such as Myc or His (for example, pcDNA3.1/Myc-His), or an expression vector for a protein fused to thioredoxin or a His tag (for example, pET32a).

The expression vector of the present disclosure expresses *in vitro* or *in vivo* a WT1 peptide or an altered WT1 peptide capable of inducing WT1-specific CTLs or helper T-cells and, therefore, is useful for treatment or prevention of a cancer.

In a further aspect, the present disclosure provides an antibody against a peptide or polynucleotide as described herein (hereinafter also referred to as WT1 antibody). The antibody of the present disclosure may be polyclonal or monoclonal, and can be prepared in accordance with a conventional method for preparation of a polyclonal or monoclonal antibody (Current protocols in Molecular Biology edit. Ausubel et al. (1987) Publish. John Wiley and Sons. Section 11.12-11.13, Antibodies; A Laboratory Manual, Lane, H, D. et al (Ed.), Cold Spring Harber Laboratory Press, New York 1989). The antibody of the present disclosure can be obtained as an antibody that recognizes the peptide as described herein, or an antibody that recognizes and neutralizes the peptide, from an animal immunized by a conventional method with the peptide. The antibody can be used in affinity chromatography, or in immunological diagnosis based, for example, on immunoblotting, radioimmunoassay (RIA), enzyme-linked immunosorbent assay (ELISA), or fluorescent or luminescent immunoassay, for a cancer, especially a cancer associated with WT1 gene expression or an elevated level of WT1 gene expression.

A peptide or compound as described herein, or an intermediate peptide for the synthesis thereof may be synthesized in accordance with a method described in the Example section herein or by using a conventional technique for peptide synthesis as described, for example, in Peptide Synthesis, Interscience, New York, 1966; The Proteins, Vol 2, Academic Press Inc., New York, 1976; peptide synthesis, Maruzen Co., LTD., 1975; Basics and Experiment of Peptide Synthesis, Maruzen Co., LTD., 1985; or Development of Pharmaceutical Product subsequent vol. 14, Peptide Synthesis, Hirokawa Shoten, 1991. Examples of such a technique include solid phase synthesis by Fmoc method or Boc method, or liquid phase synthesis by sequential condensation of Boc-amino acid or Z-amino acid in a liquid phase (wherein Fmoc means 9-fluorenylmethoxycarbonyl, Boc means t-butoxycarbonyl, and Z means benzyloxycarbonyl). The peptide may be obtained by a genetic technique by using a nucleotide sequence encoding the peptide in accordance with a conventionally known method as described, for example, in Molecular Cloning, T. Maniatis et al., CSH Laboratory (1983), DNA Cloning, DM. Glover, IRL PRESS (1985).

The compound of formula (1) can be prepared from two different MHC class I-restricted WT1 peptides, or from an MHC class I-restricted WT1 peptide and an MHC class II-restricted WT1 peptide, by linking the peptides by a disulfide bond (WO 2014/157692), for example.

In the course of the synthesis of a compound of formula (1), a functional group on an intermediate compound, such as amino, carboxyl or mercapto may be protected with an appropriate protecting group, or deprotected as needed by a conventional technique. For information about such a protecting group, or a protection or deprotection method, "Protective Groups in Organic Synthesis 2nd Edition (John Wiley & Sons, Inc.; 1990)" may be referred to. As a protecting group for mercapto, an acetamidomethyl group or a trityl group may be useful.

When a compound of formula (1) includes a disulfide bond, the linkage is formed between two different, cysteine-comprising peptide components in the compound, or between a cysteine-comprising peptide component and a cysteine residue in the compound. Such a disulfide bond can be formed by a method as described, for example, in Peptide Synthesis, Interscience, New York, 1966; The Proteins, Vol. 2, Academic Press Inc., New York, 1976; peptide synthesis, Maruzen Co., LTD., 1975; Basics and Experiment of peptide synthesis, Maruzen Co., LTD., 1985; or Development of Pharmaceutical Product sequential vol. 14, Peptide Synthesis, Hirokawa Shoten, 1991.

Specifically, for preparing a compound having a disulfide bond (a disulfide compound) from a peptide having one cysteine residue, the peptide may be subjected to a deprotection reaction for removal of any protecting groups on functional groups including mercapto on the cysteine residue, and then treated in an inert solvent under an oxidative condition for forming a disulfide bond. A disulfide compound may also be prepared from two different, mercapto-having intermediates by treating them in an appropriate solvent under an oxidative condition. Oxidative conditions for disulfide bond formation are known in the field of peptide synthesis. For example, a known method of iodine oxidation, air oxidation under an alkaline condition, or oxidation by an oxidizing agent under an alkaline or acidic condition may be used for forming a disulfide bond. As an oxidizing agent, iodine, dimethyl sulfoxide (DMSO), or potassium ferricyanide may be used. As a solvent for the reaction, water, acetic acid, methanol, chloroform, DMF, or DMSO, or a mixture thereof may be used. Such an oxidative condition often gives a product in the form of a mixture of symmetric and asymmetric disulfide compounds. A desired asymmetric disulfide compound may be recovered or purified by an appropriate chromatographic method or recrystallization. A mercapto group may be activated for disulfide bond formation by conversion of the group to an Npys group (3-nitro-2-pyridinesulphenyl group). For forming a disulfide bond on a certain mercapto group on an intermediate, the group may be activated by 2,2'-dithiobis(5-nitropyridine) in advance of coupling with another intermediate compound (Tetrahedron Letters. Vol. 37. No. 9, pp. 1347-1350).

The methods as described above may be useful for preparing a disulfide compound from a peptide having more than one cysteine residue. In that case, however, a mixture of different disulfide compounds having disulfide bonds between different cysteine residues may be formed. For selectively preparing a product dimerized by a disulfide bond between specific positions of monomers, different protecting groups can be used in combination for protection of functional groups on the cysteine residues. Examples of such combination of protecting groups include a combination of MeBzl (methylbenzyl) and Acm (acetamidomethyl); Trt (trityl) and Acm; Npys (3-nitro-2-pyridylthio) and Acm group; and S-Bu-t (S-tert-butyl) and Acm. For example, when a peptide protected with a combination of MeBzl and Acm is used for

selective disulfide bond formation, all the MeBzl protecting groups, and the Acm protecting groups on functional groups on amino acid residues other than certain cysteine residues may be removed in the first step. Then, by treating the peptide monomer in a solution under air oxidation condition, a disulfide bond can be formed between selectively deprotected cysteine residues of the monomers. Then, through removal of remaining Acm protecting groups and treatment under oxidative condition with iodine, a further disulfide bond can be formed on the newly deprotected cysteine residues.

The peptide, compound or intermediate synthesized may be purified by any purification method know in the art or in the field of peptide chemistry. Examples of such a purification technique include various types of chromatography (e.g., silica gel column chromatography, ion exchange column chromatography, gel filtration or reversed-phase chromatography), and recrystallization from a solvent, for example an alcohol, such as methanol, ethanol or 2-propanol; an ether, such as diethyl ether; an ester, such as ethyl acetate; an aromatic hydrocarbon, such as benzene or toluene; a ketone, such as acetone; a hydrocarbon, such as hexane; an aprotic solvent, such as dimethylformamide or acetonitrile; water; or a mixture thereof. For further useful purification methods, reference can be made, for example, to Jikken Kagaku Kouza (The Chemical Society of Japan ed., Maruzen) vol. 1. Purification methods for disulfide compounds are also described in Peptide Synthesis, Interscience, New York, 1966; peptide synthesis, Maruzen Co., LTD., 1975; Basics and Experiment of peptide synthesis, Maruzen Co., LTD., 1985; or Development of Pharmaceutical Product sequential vol. 14, Peptide Synthesis, Hirokawa Shoten, 1991. Purification by HPLC is preferred.

A compound of formula (1) may have one or more asymmetric centers. Such a compound can be prepared from a starting material (an amino acid) having corresponding asymmetric centers. Also, a compound of formula (1) can be obtained in a high optical purity by inclusion of an optical resolution step in a process for its synthesis. For example, in accordance with a diastereomer method for optical resolution, a compound of formula (1) or an intermediate product can be treated with an optically active acid (e.g., a monocarboxylic acid such as mandelic acid, N-benzyloxyalanine, or lactic acid, a dicarboxylic acid such as tartaric acid, o-diisopropylidenetartaric acid, or malic acid, or a sulfonic acid such as camphorsulfonic acid or bromocamphorsulfonic acid) in an inert solvent (e.g., an alcohol such as methanol, ethanol, or 2-propanol, an ether such as diethyl ether, an ester such as ethyl acetate, a hydrocarbon such as toluene, an aprotic solvent such as acetonitrile, or a mixture thereof) to form salts. For optically resolving a compound of formula (1) or an intermediate having an acidic functional group such as carboxyl, its salts can be formed with an optically active amine (e.g., an organic amine such as $\alpha$-phenethylamine, kinin, quinidine, cinchonidine, cinchonine, or strychnine).

The salts may be formed at a temperature in the range from room temperature up to the boiling point of a solvent used. For obtaining a product in a highly optically pure form, it may be desirable to raise the temperature to around the boiling point of the solvent for a period of time. Salts formed are crystallized, and then filtered, optionally with cooling for an improved yield. An optically active acid or amine used for the salt formation may be used in an amount of about 0.5 to about 2.0 equivalents, preferably about 1 equivalent, relative to the amount of a compound to optically resolve. A crystalline product may optionally be further purified by recrystallization from an inert solvent (e.g., an alcohol such as methanol, ethanol, or 2-propanol, an ether such as diethyl ether, an ester such as ethyl acetate, a hydrocarbon solvent such as toluene, an aprotic solvent such as acetonitrile, or a mixture thereof). A product recovered in the form of a salt may optionally be converted to a free base or acid by treatment with an acid or base.

The term "pharmaceutically acceptable salt" as used herein includes an acid addition salt and a base addition salt. The acid addition salt may be an inorganic acid salt, such as hydrochloride, hydrobromide, sulfate, hydroiodide, nitrate, or phosphate, or an organic acid salt such as citrate, oxalate, acetate, formate, propionate, benzoate, trifluoroacetate, maleate, tartrate, methanesulfonate, benzenesulfonate, or p-toluenesulfonate. The base addition salt may be a salt with an inorganic base, such as sodium salt, potassium salt, calcium salt, magnesium salt, or ammonium salt, a salt with an organic base, such as triethylammonium salt, triethanolammonium salt, pyridinium salt, or diisopropylammonium salt. The "pharmaceutically acceptable salt" also includes a salt with a basic or acidic amino acid, such as arginine, aspartic acid, or glutamic acid. The term "peptide" or "compound" used herein includes a peptide or compound in the form of a pharmaceutically acceptable salt, unless the context requires otherwise.

The present invention further includes a hydrate or a solvate such as an ethanol solvate of the peptide or the compound of formula (1) or a pharmaceutically acceptable salt thereof as described herein. The present invention also includes any stereoisomer such as a diastereomer or an enantiomer, and any crystalline form of the peptide or the compound as described herein.

When two of more of the peptide and the compound of formula (1) and a pharmaceutically acceptable salt thereof as described herein, and optionally a further active agent are used in combination, they may be formulated in separate compositions or incorporated in a single composition. In an embodiment, a WT1 killer peptide or a compound of formula (1) and a WT1 helper peptide are incorporated in a single composition. In another embodiment, a WT1 killer peptide or a compound of formula (1) and a WT1 helper peptide are formulated in separate compositions. A composition comprising a WT1 killer peptide, a compound of formula (1) or a WT1 helper peptide may be provided together with instructions of dosage and administration for use of the composition in combination with the other active agent. A composition comprising a WT1 killer peptide or a compound of formula (1), and a composition comprising a WT1 helper peptide may be incorporated in a single kit. Such a kit may further comprise instructions of dosage and administration

for use of the compositions in combination, or may be packaged. In administration of more than one active agent in combination, the agents may be administered on the same administration schedule or different administration schedules.

The peptide or the compound of formula (1), or a pharmaceutically acceptable salt thereof, or a combination thereof as described herein is useful in treatment or prevention (including prevention of recurrence) of a cancer, especially a cancer associated with WT1 gene expression or an elevated level of WT1 gene expression. For example, the peptide or the compound of formula (1), or a pharmaceutically acceptable salt thereof, or a combination thereof as described herein can be an active ingredient of a pharmaceutical composition (for example, a cancer vaccine), or a composition for inducing CTLs in cellular immunotherapy for a cancer.

The peptide, the compound of formula (1), a pharmaceutically acceptable salt thereof, or a combination thereof as described herein may be administered in combination with other drug(s) (hereinafter referred to as coadministration drug(s)).

In an embodiment, the peptide or the compound of formula (1), or a pharmaceutically acceptable salt thereof, or a combination thereof as described herein may be administered in combination with an "immunomodulator". As used herein, the term "immunomodulator" means any agent that controls transmission of costimulatory signals generated during T cell activation by antigen-presenting cells by interacting with molecules that are involved in the transmission of the costimulatory signals and are present on the antigen-presenting cells and/or T cells, as well as any agent that directly or indirectly controls function of molecules involved in establishment of immune tolerance (immunosuppression) in the immune system. Since a cancer antigen peptide is effective for increasing tumor-reactive CTLs in a tumor, it is potentially useful as an agent for coadministration with an immunomodulator, for lowering a necessary dose of an immunomodulator or reducing adverse event caused by an immunomodulator. Thus, the present disclosure provides, through the use of a WT1 antigen peptide in combination with an immunomodulator, patients with a therapy having improved efficacy and safety.

The "immunomodulator" can be an agent in the form of an antibody, a nucleic acid, a protein, a peptide, or a small compound, but is not limited thereto. The "antibody" as the "immunomodulator" includes an antibody fragment. Examples of the antibody fragment include heavy and light chain variable regions of an antibody (VH and VL), F(ab')2, Fab', Fab, Fv, Fd, sdFv, and scFV. The "protein" as the "immunomodulator" means any protein other than antibodies. Examples of the "immunomodulator" include immune checkpoint inhibitors, costimulatory molecule agonists, immune activating agents, and low-molecular inhibitors.

The "immune checkpoint inhibitor" inhibits immunosuppressive effect induced by cancer cells or antigen presenting cells. Examples of the immune checkpoint inhibitor include, but are not limited to, agents against a molecule selected from the group consisting of: (1) CTLA-4 (e.g., ipilimumab and tremelimumab); (2) PD-1 (e.g., nivolumab, pembrolizumab, AMP-224, AMP-514 (MEDI0680), and pidilizumab (CT-011)); (3) LAG-3 (e.g., IMP-321 and BMS-986016); (4) BTLA; (5) KIR (e.g., IPH2101); (6) TIM-3; (7) PD-L1 (e.g., durvalumab (MEDI4736), MPDL3280A, BMS-936559, and avelumab (MSB0010718C)); (8) PD-L2; (9) B7-H3 (e.g., MGA-271); (10) B7-H4; (11) HVEM; (12) GAL9; (13) CD160; (14) VISTA; (15) BTNL2; (16) TIGIT; (17) PVR; (18) BTN1A1; (19) BTN2A2; (20) BTN3A2 (Nat Rev Drug Discov. 2013; 12: 130-146; Nikkei Medical Cancer Review 2014; 9; Nat Rev Immunol. 2014; 14: 559-69); and (21) CSF1-R.

The "costimulatory molecule agonist" enhances T cell activation by transmission of an auxiliary signal via a costimulatory molecule on the T cells and/or antigen presenting cells, and attenuates the immunosuppressive effect of cancer cells or antigen presenting cells. Examples of the costimulatory molecule agonist include, but are not limited to, agents against a molecule selected from the group consisting of: (1) 4-1BB; (2) 4-1BB-L; (3) OX40 (4) OX40-L; (5) GITR; (6) CD28; (7) CD40; (8) CD40-L; (9) ICOS; (10) ICOS-L; (11) LIGHT; and (12) CD27.

The "immune activating agent" efficiently stimulates killer T cells in the lymph nodes by directly or indirectly activating immune cells such as T cells and dendritic cells. Examples of the immune activating agent include, but are not limited to, Toll-like receptor (TLR) agonists, stimulator of interferon genes (STING) agonists, cytokines, and agents against heat shock protein (HSP).

Examples of the "Toll-like receptor (TLR) agonist" include, but are not limited to, TLR1/2 agonists, TLR2 agonists, TLR3 agonists (e.g., PolyI:C), TLR4 agonists (e.g., S-type lipopolysaccharide, paclitaxel, lipid A, and monophosphoryl lipid A), TLR5 agonists (e.g., flagellin), TLR6/2 agonists (e.g., MALP-2), TLR7 agonist, TLR7/8 agonists (e.g., gardiquimod, imiquimod, loxoribine, and resiquimod (R848)), TLR7/9 agonists (e.g., hydroxychloroquine sulfate), TLR8 agonists (e.g., motolimod (VTX-2337)), TLR9 agonists (e.g., CpG-ODN), and TLR11 agonists (e.g., profilin).

Examples of the "cytokine" include, but are not limited to, IL-1α, IL-1β, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (INF)-α, INF-β, INF-γ, SCF, GM-CSF, G-CSF, M-CSF, erythropoietin, thrombopoietin, macrophage inflammatory protein (MIP), and monocyte chemoattractant protein (MCP).

Examples of the "heat shock protein (HSP)" include, but are not limited to, HSP70, HSP90, HSP90α, HSP90β, HSP105, HSP72, and HSP40. Agents against a heat shock protein include HSP inhibitors. Examples of inhibitors to HSP90, for example, include, but are not limited to, tanespimycin (17-AAG), luminespib (AUY-922, NVP-AUY922), alvespimycin (17-DMAG) hydrochloride, ganetespib (STA-9090), BIIB021, onalespib (AT13387), geldanamycin, NVP-BEP800, SNX-2112 (PF-04928473), PF-4929113 (SNX-5422), KW-2478, XL888, VER155008, VER-50589, CH5138303, VER-49009, NMS-E973, PU-H71, HSP990 (NVP-HSP990) and KNK437.

Examples of the "low-molecular inhibitor" include, but are not limited to, histone deacetylase inhibitors, histone demethylase inhibitors, histone acetyltransferase inhibitors, histone methyltransferase inhibitors, DNA methyltransferase inhibitors, anthracycline antibiotics, platinum formulations, MAPK inhibitors, β-catenin inhibitors, STAT3 inhibitors, NF-kB inhibitors, JAK inhibitors, mTOR inhibitors, IDO inhibitors, COX-2 inhibitors, CXCR4 inhibitors, and arginase inhibitors.

Examples of the "histone deacetylase inhibitor" include, but are not limited to, vorinostat (SAHA, MK0683), entinostat (MS-275), panobinostat (LBH589), trichostatin A (TSA), mocetinostat (MGCD0103), BG45, BRD73954, belinostat (PXD101), romidepsin (FK228, depsipeptide), 4SC-202, HPOB, LMK-235, CAY10603, tasquinimod, TMP269, nexturastat A, rocilinostat (ACY-1215), RGFP966, RG2833 (RGFP109), scriptaid, tubastatin A, pracinostat (SB939), CUDC-101, M344, PCI-34051, dacinostat (LAQ824), tubastatin A hydrochloride, abexinostat (PCI-24781), CUDC-907, AR-42, sodium phenylbutyrate, resminostat, tubacin, quisinostat (JNJ-26481585) dihydrochloride, MC1568, givinostat (ITF2357), droxinostat, chidamide (C S055, HBI-8000), CHR-2485, CHR-3996, DAC-060, FRM-0334 (EVP-0334), MGCD-290, CXD-101 (AZD-9468), CG200745, arginine butyrate, sulforaphane, SHP-141, CUDC-907, YM753 (OBP-801), sodium valproate, apicidin, and CI994 (tacedinaline).

Examples of the "histone demethylase inhibitor" include, but are not limited to, GSK J4 HCl, OG-L002, JIB-04, IOX1, SP2509, ORY-1001 (RG-6016), GSK J1, ML324, and GSK-LSD1 2HCl.

Examples of the "histone acetyltransferase inhibitor" include, but are not limited to, C646, MG149, remodelin, and anacardic acid.

Examples of the "histone methyltransferase inhibitor" include, but are not limited to, pinometostat (EPZ5676), EPZ005678, GSK343, BIX01294, tazemetostat (EPZ6438), 3-deazaneplanocin A (DZNeP) HCl, UNC1999, MM-102, SGC0946, entacapone, EPZ015666, UNC0379, EI1, MI-2 (menin-MLL inhibitor), MI-3 (menin-MLL inhibitor), PFI-2, GSK126, EPZ04777, BRD4770, GSK-2816126, and UNC0631.

Examples of the "DNA methyltransferase inhibitor" include, but are not limited to, decitabine, azatidine, RG108, thioguanine, zebularine, SGI-110, CC-486, SGI-1027, lomeguatrib, and procainamide hydrochloride.

The "anthracycline antibiotic" is intercalated between DNA strands to inhibit DNA relaxation. Examples of the anthracycline antibiotic include, but are not limited to, doxorubicin, liposomal doxorubicin, daunorubicin, pirarubicin, epirubicin, idarubicin, aclarubicin, amrubicin, aloin, and mitoxantrone.

Examples of the "platinum formulation" include, but are not limited to, cisplatin, carboplatin, miboplatin, nedaplatin, satraplatin (JM-126), oxaliplatin (ELOXATIN), triplatin tetranitrate, and DDS formulations thereof.

Examples of the "MAPK inhibitor" include, but are not limited to, SB203580, doramapimod (BIRB796), SB202190 (FHPI), LY2228820, VX-702, SB239063, pexmetinib (ARRY-614), PH-797804, VX-745, and TAK-715.

Examples of the "β-catenin inhibitor" include, but are not limited to, XAV-939, ICG-001, IWR-1-endo, Wnt-C59 (C59), LGK-974, KY02111, IWP-2, IWP-L6, WIKI4, and FH535.

Examples of the "STAT3 inhibitor" include, but are not limited to, S3I-201, Stattic, niclosamide, nifuroxazide, napabucasin (BBI608), cryptotanshinone, HO-3867, WHI-P154, FLLL32, STA-21, WP1066, and SH-4-54.

Examples of the "NF-kB inhibitor" include, but are not limited to, QNZ (EVP4593), sodium 4-aminosalicylate, JSH-23, phenethyl caffeate, sodium salicylate, andrographolide, and SC75741.

Examples of the "JAK inhibitor" include, but are not limited to, ruxolitinib (INCB018424), tofacitinib (CP-690550) citrate, AZD1480, fedratinib (SAR302503, TG101348), AT9283, tyrphostin B42 (AG-490), momelotinib (CYT387), tofacitinib (CP-690550, tasocitinib), WP1066, TG101209, gandotinib (LY2784544), NVP-BSK805 2HCl, baricitinib (LY3009104, INCB02850), AZ960, CEP-33779, pacritinib (SB1518), WHI-P154, XL019, S-ruxolitinib (INCB018424), ZM39923 HCl, decernotinib (VX-509), cerdulatinib (PRT062070, PRT2070), filgotinib (GLPG0634), FLLL32, peficitinib (ASP015K, JNJ-54781532), GLPG0634 analogue, Go6976, and Curcumol.

Examples of the "mTOR inhibitor" include, but are not limited to, sirolimus (rapamycin), deforolimus (AP23573, MK-8669), everolimus (RAD-001), temsirolimus (CCI-779, NSC683864), zotarolimus (ABT-578), biolimus A9 (umirolimus), AZD8055, KU-0063794, voxtalisib (XL765, SAR245409), MHY1485, dactolisib (BEZ235, NVP-BEZ235), PI-103, and torkinib (PP242).

Examples of the "IDO inhibitor" include, but are not limited to, NLG919, INCB024360 analog, indoximod (NLG-8189), and epacadostat (INCB024360).

Examples of the "COX-2 inhibitor" include, but are not limited to, valdecoxib, rofecoxib, carprofen, celecoxib, lumiracoxib, tolfenamic acid, nimesulide, niflumic acid, asaraldehyde, lornoxicam, sodium meclofenamate, amfenac sodium hydrate, diclofenac sodium, ketoprofen, ketorolac, naproxen sodium, indomethacin, ibuprofen, aspirin, mefenamic acid, bromfenac sodium, oxaprozin, zaltoprofen, and nepafenac.

Examples of the "CXCR4 inhibitor" include, but are not limited to, WZ811, plerixafor (AMD3100), and plerixafor 8HCl (AMD3100 8HCl).

The peptide or the compound of formula (1), or a pharmaceutically acceptable salt thereof, or a combination thereof as described herein may also be used in combination with one or more drugs selected from the group consisting

of "hormone therapy agent", "immunotherapeutic agent", "biopharmaceutical", "cell growth factor", "cell growth factor inhibitor", "cell growth factor receptor inhibitor", "radiotherapeutic agent", "auxiliary agent", and "chemotherapeutic agent". For example, one to five drugs, one to three drugs, or one drug selected from the above group of drugs may be used in combination with the peptide or the compound of formula (1), or a pharmaceutically acceptable salt thereof, or a combination thereof as described herein.

Examples of the "hormone therapy agent" include adrenal cortical hormone agents (e.g., steroidal anti-inflammatory agents, estrogen preparations, progesterone preparations, and androgen preparations), anti-estrogen agents, estrogen-controlling agents, estrogen synthesis inhibitors, anti-androgen agents, androgen-controlling agents, androgen synthesis inhibitors, LH-RH agonist preparations, LH-RH antagonist preparations, aromatase inhibitors, steroid-lactonase inhibitors, contraceptive pills, retinoids, and agents that delay metabolism of a retinoid.

Examples of the "hormone therapy agent" include fosfestrol, diethylstilbestrol, fluoxymesterol, chlorotrianisene, methyl testosterone, medroxyprogesterone acetate, megestrol acetate, chlormadinone acetate, cyproterone acetate, danazol, allylestrenol, gestrinone, mepartricin, raloxifene, ormeloxifene, levormeloxifene, tamoxifen citrate, toremifene citrate, iodoxyfene, pill formulations, mepitiostane, testololactone, aminoglutethimide, goserelin acetate, buserelin, leuprorelin, leuprolide, droloxifene, epitiostanol, ethinylestradiol sulfonate, estramustine, fadrozole hydrochloride, anastrozole, tetrazole, ketoconazole, letrozole, exemestane, vorozole, formestane, flutamide, bicalutamide, nilutamide, enzalutamide, mifepristone, finasteride, dexamethasone, prednisolone, betamethasone, triamcinolone, abiraterone, liarozole, bexarotene, and DN101.

Examples of the "immunotherapeutic agent" include picibanil, krestin, sizofiran, lentinan, ubenimex, interferon (IFN)-$\alpha$, interferon (IFN)-$\beta$, interferon (IFN)-$\gamma$, interleukin, macrophage colony stimulating factor, granulocyte-colony stimulating factor, erythropoietin, lymphotoxin, BCG vaccine, *Corynebacterium parvum,* levamisole, polysaccharide K, procodazole, anti-CTLA4 antibody, anti-PD-1 antibody, and TLR agonists (e.g., TLR7 agonists, TLR8 agonists, TLR9 agonists).

Examples of the "biopharmaceutical" include, but are not limited to, interleukin-2 (aldesleukin), interferon-a, interferon-p, interferon-y, erythropoietin (EPO), granulocyte-colony stimulating factor (filgrastim), granulocyte-macrophage-colony stimulating factor (sargramostim), IL13-PE38QQR, Bacille Calmette-Guerin, levamisole, octreotide, CPG7909, Provenge, GVAX, Myvax, Favld, lenalidomide, trastuzumab, rituximab, gemtuzumab ozogamicin, alemtuzumab, endostatin, ibritumomab tiuxetan, tositumomab, cetuximab, zanolimumab, ofatumumab, HGS-ETR1, pertuzumab, M200, SGN-30, matuzumab, adecatumumab, denosumab, zalutumumab, MDX-060, nimotuzumab, MORAb-003, Vitaxin, MDX-101, MDX-010, DPC4 antibodies, NF-1 antibodies, NF-2 antibodies, Rb antibodies, p53 antibodies, WT1 antibodies, BRCA1 antibodies, BRCA2 antibodies, ganglioside (GM2), prostate specific antigens (PSA), $\alpha$-fetoprotein (AFP), carcinoembryonic antigens (CEA), melanoma-associated antigens (MART-1, gap100, MAGE 1,3 tyrosine), papilloma virus E6 and E7 fragments, and DDS formulations thereof.

Regarding the "cell growth factor", "cell growth factor inhibitor" and "cell growth factor receptor inhibitor", cell growth factor may be any agent that promotes cell proliferation. For example, a cell growth factor may be a peptide that has a molecular weight of not more than 20,000 and can bind to a receptor and function at a low concentration.

Examples of the "cell growth factor" include, but are not limited to, epidermal growth factor (EGF), insulin-like growth factor (IGF (e.g., insulin, IGF-1, and IGF-2)), transforming growth factor (TGF (e.g., TGF-$\alpha$ and TGF-$\beta$)), nerve growth factor (NGF), brain-derived neurotrophic factor (BDNF), vascular endothelial growth factor (VEGF), colony stimulating factor (CSF (e.g., granulocyte-colony stimulating factor (G-CSF)), granulocyte-macrophage-colony stimulating factor (GM-CSF)), platelet-derived growth factor (PDGF), erythropoietin (EPO), fibroblast growth factor (FGF (e.g., acidic FGF, basic FGF, keratinocyte growth factor (KGK), and FGF-10)), hepatocyte growth factor (HGF), heregulin, and angiopoietin. The term "cell growth factor" is synonymous with the term "growth factor".

Examples of the "cell growth factor inhibitor" include, but are not limited to, epidermal growth factor inhibitors (EGF inhibitors), insulin-like growth factor inhibitors (IGF inhibitors), nerve growth factor inhibitors (NGF inhibitors), brain-derived neurotrophic factor inhibitors (BDNF inhibitors), vascular endothelial cell growth factor inhibitors (VEGF inhibitors), colony stimulating factor inhibitors (CSF inhibitors), platelet-derived growth factor inhibitors (PDGF inhibitors), erythropoietin inhibitors (EPO inhibitors), fibroblast growth factor inhibitors (FGF inhibitors), hepatocyte growth factor inhibitors (HGF inhibitors), heregulin inhibitors, and angiopoietin inhibitors. The term "cell growth factor inhibitor" is synonymous with the term "growth factor inhibitor".

Examples of the "cell growth factor receptor inhibitor" include, but are not limited to, epidermal growth factor receptor inhibitors (EGFR inhibitors), insulin-like growth factor receptor inhibitors (IGFR inhibitors), nerve growth factor receptor inhibitors (NGFR inhibitors), brain-derived neurotrophic factor receptor inhibitors (BDNFR inhibitors), vascular endothelial cell growth factor receptor inhibitors (VEGFR inhibitors), colony stimulating factor receptor inhibitors (CSFR inhibitors), platelet-derived growth factor receptor inhibitors (PDGFR inhibitors), erythropoietin receptor inhibitors (EPOR inhibitors), fibroblast growth factor receptor inhibitors (FGFR inhibitors), hepatocyte growth factor receptor inhibitors (HGFR inhibitors), heregulin receptor inhibitors, and angiopoietin receptor inhibitors. The term "cell growth factor receptor inhibitor" is synonymous with the term "growth factor receptor inhibitor".

Examples of the "radiotherapeutic agent" include, but are not limited to, radioactive materials and radiosensitizers.

The "auxiliary agent" is an agent used together with an anticancer agent for suppressing a side effect or vomiting caused by the anticancer agent. Examples of the "auxiliary agent" include, but are not limited to, aprepitant, ondansetron, lorazepam, dexamethasone, diphenhydramine, ranitidine, cimetidine, ranitidine, famotidine, cimetidine, Procrit, epoetin alfa, filgrastim, oprelvekin, leucovorin, and granulocyte-macrophage-colony stimulating factor (GM-CSF) .

Examples of the "chemotherapeutic agent" include, but are not limited to, alkylating agents, platinum formulations, antimetabolites, topoisomerase inhibitors, DNA intercalators, antimitotic agents, antitumor antibiotics, plant-derived anticancer agents, epigenome drugs, immunomodulators, molecular targeted drugs, angiogenesis inhibitors, and other chemotherapeutic agents. Some typical examples of chemotherapeutic agent are listed below.

Examples of the "alkylating agent" include, but are not limited to, nitrogen mustard, nitrogen mustard N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, procarbazine, ranimustine, estramustine sodium phosphate, triethylenemelamine, carmustine, lomustine, streptozocin, pipobroman, etoglucid, altretamine, ambamustine, dibrospidium hydrochloride, fotemustine, prednimustine, bendamustine, uramustine, semustine, pumitepa, ribomustin, temozolomide, treosulfan, trofosfamide, zinostatin stimalamer, adozelesin, cystemustine, bizelesin, mechlorethamine, uracil mustard, trabectedin, chlormethine, mannosulfan, triaziquone, procarbazine, canfosfamide, nitrosoureas, and DDS formulations thereof.

Examples of the "platinum formulation" include, but are not limited to, cisplatin, carboplatin, miboplatin, nedaplatin, satraplatin, oxaliplatin, triplatin tetranitrate, and DDS formulations thereof.

Examples of the "antimetabolite" include, but are not limited to, antifolates, pyrimidine metabolism inhibitors, purine metabolism inhibitors, ribonucleotide reductase inhibitors, and nucleotide analogs.

Examples of the "antimetabolite" include, but are not limited to, mercaptopurine, 6-mercaptopurine riboside, thioinosine, methotrexate, pemetrexed, eoshitabin, enocitabine, cytarabine, cytarabine ocfosfate, ancitabine hydrochloride, 5-FU agents (e.g., fluorouracil, Carzonal, Bennan, Lunachol, Lunapon, tegafur, tegafur-uracil, tegafur-gimeracil-oteracil potassium (TS-1), UFT, doxifluridine, carmofur, gallocitabine, emitefur, and capecitabine), aminopterin, nelarabine, leucovorin calcium, Tabloid, butocine, folinate calcium, levofolinate calcium, cladribine, emitefur, fludarabine, gemcitabine, hydroxycarbamide, pentostatin, piritrexim, idoxuridine, mitoguazone, tiazofurine, ambamustine, bendamustine, floxuridine, leucovorin, hydroxyurea, thioguanine, asparaginase, bortezomib, raltitrexed, clofarabine, enocitabine, sapacitabine, azacytidine, sulfadiazine, sulfamethoxazole, trimethoprim, Liproxstatin-1, D4476, Xanthohumol, Epacadostat (INCB024360), Vidofludimus, P7C3, GMX1778 (CHS828), NCT-501, SW033291, Ro61-8048, and DDS formulations thereof.

Examples of the "topoisomerase inhibitor" include, but are not limited to, doxorubicin, daunorubicin, epirubicin, idarubicin, anthracenedione, mitoxantrone, mitomycin C, bleomycin, dactinomycin, plicatomycin, irinotecan, camptothecin, rubitecan, belotecan, etoposide, teniposide, topotecan, amsacrine, and DDS formulations thereof.

Examples of the "DNA intercalator" include, but are not limited to, proflavine, doxorubicin (adriamycin), daunorubicin, dactinomycin, thalidomide, and DDS formulations thereof.

Examples of the "antimitotic agent" include, but are not limited to, paclitaxel, paclitaxel derivatives (e.g., DHA paclitaxel, paclitaxel polyglutamate, nab-paclitaxel, micellar paclitaxel, 7$\alpha$-glucosyloxyacetylpaclitaxel, and BMS-275183), docetaxel, vinorelbine, vincristine, vinblastine, vindesine, vinzolidine, etoposide, teniposide, ixabepilone, larotaxel, ortataxel, tesetaxel, ispinesib, colchicine, vinflunine, and DDS formulations thereof.

Examples of the "antitumor antibiotic" include, but are not limited to, actinomycin D, actinomycin C, mitomycin C, chromomycin A3, mithramycin A, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, amrubicin hydrochloride, neocarzinostatin, zinostatin stimalamer, mithramycin, sarkomycin, carzinophilin, mitotane, zorubicin hydrochloride, mitoxantrone hydrochloride, idarubicin hydrochloride, liposomal doxorubicin, and DDS formulations thereof.

Examples of the "plant-derived anticancer agent" include, but are not limited to, irinotecan, nogitecan, etoposide, etoposide phosphate, eribulin, sobuzoxane, vinblastine sulfate, vincristine sulfate, vindesine sulfate, teniposide, paclitaxel, paclitaxel injection, docetaxel, DJ-927, vinorelbine, topotecan, and DDS formulations thereof.

Examples of the "epigenome drug" include, but are not limited to, DNA methylation inhibitors, histone deacetylase (HDAC) inhibitors, DNA methyl transferase (DNMT) inhibitors, histone deacetylase activators, histone demethylase inhibitors, and methylated nucleotides.

Specific examples of the "epigenome drug" include, but are not limited to, vorinostat, belinostat, mocetinostat (MGCD0103), entinostat (SNDX-275), romidepsin, azacytidine, decitabine, GSK2879552 2HI, SGC707, ORY-1001 (RG-6016), PFI-4, SirReal2, GSK2801, CPI-360, GSK503, AMI-1, CPI-169, and DDS formulations thereof.

Examples of the "immunomodulator" include, but are not limited to, thalidomide, lenalidomide, pomalidomide, and DDS formulations thereof.

The "molecular targeted drug" can be a small compound or an antibody. Examples of the "molecular targeted drug" include, but are not limited to, kinase inhibitors, proteasome inhibitors, monoclonal antibodies, mTOR inhibitors, TNF inhibitors, and T-cell inhibitors.

Examples of the "kinase inhibitor" include, but are not limited to, tyrosine kinase inhibitors, serine/threonine kinase inhibitors, Raf kinase inhibitors, cyclin-dependent kinase (CDK) inhibitors, and mitogen-activated protein kinase (MEK) inhibitors.

Specific examples of the "kinase inhibitor" include, but are not limited to, imatinib, gefitinib, erlotinib, afatinib, dasatinib, bosutinib, vandetanib, sunitinib, axitinib, pazopanib, lenvatinib, lapatinib, nintedanib, nilotinib, crizotinib, ceritinib, alectinib, ruxolitinib, tofacitinib, ibrutinib, sorafenib, vemurafenib, dabrafenib, palbociclib, trametinib, regorafenib, cedivanib, lestaurtinib, bandetinib, vatalanib, seliciclib, tivantinib, canertinib, pelitinib, tesevatinib, cediranib, motesanib, midostaurin, foretinib, cabozantinib, selumetinib, neratinib, volasertib, saracatinib, enzastaurin, tandutinib, semaxanib, alvocidib, ICR-62, AEE788, PD0325901, PD153035, TK787, amcasertib (BBI503), E6201, E7050, and DDS formulations thereof.

Examples of the "proteasome inhibitor" include, but are not limited to, bortezomib, carfilzomib, and DDS formulations thereof.

Examples of the "monoclonal antibody" include, but are not limited to, anti-CD22 antibodies, anti-CD20 antibodies, anti-CD25 antibodies, anti-CD30 antibodies, anti-CD33 antibodies, anti-CD5 antibodies, anti-CD52 antibodies, anti-epidermal growth factor receptor antibodies (EGFR antibodies), anti-vascular endothelial cell growth factor antibodies (VEGF antibodies), anti-TNF-a antibodies, anti-IL-1 receptor antibodies, anti-IL-2 receptor antibodies, anti-IL-5 receptor antibodies, anti-IL-6 receptor antibodies, anti-HER2 antibodies, anti-IgE antibodies, anti-IgG antibodies, anti-RS virus antibodies, anti-CCR4 antibodies, anti-cytotoxic T lymphocyte-associated antigen 4 (CTLA-4, CD152) antibodies, anti-PD-1 antibodies, anti-receptor activator of nuclear factor κB ligand (RANKL) antibodies, anti-c-Met antibodies, and anti-CXCR4 antibodies.

Specific examples of the "monoclonal antibody" include, but are not limited to, ibritumomab tiuxetan, rituximab, cetuximab, infliximab, basiliximab, brentuximab vedotin, tocilizumab, trastuzumab, bevacizumab, omalizumab, mepolizumab, gemtuzumab, ozogamicin, palivizumab, ranibizumab, certolizumab, ocrelizumab, mogamulizumab, eculizumab, pertuzumab, alemtuzumab, inotuzumab, panitumumab, ofatumumab, golimumab, adalimumab, ramucirumab, nivolumab, anakinra, denosumab, ipilimumab, pembrolizumab, matuzumab, farletuzumab, MORAb-004, MORA-b009, and DDS formulations thereof.

Examples of the "mTOR inhibitor" include, but are not limited to, everolimus (RAD001), rapamycin (sirolimus), AZD8055, temsirolimus (CCI-779, NSC683864), KU-0063794, voxtalisib (XL-765, SAR245409), MHY1485, dactolisib (BEZ235), PI-103, torkinib (PP242), ridaforolimus (deforolimus, MK-8669), INK-128 (MLN0128), Torin1, omipalisib (GSK2126458, GSK458), OSI-027, PF-04691502, apitolisib (GDC-0980, RG7422), GSK1059615, gedatolisib (PF-05212384, PKI-587), WYE-132, PP121, WYE-354, AZD2014, Torin2, WYE-687, CH5132799, WAY-600, ETP-46464, GDC-0349, XL388, zotarolimus (ABT-578), tacrolimus (FK506), BGT226 (NVP-BGT226), Palomid 529 (P529), chrysophanic acid, and DDS formulations thereof.

Examples of the "TNF inhibitor" include, but are not limited to, etanercept, lenalidomide (CC-5013), pomalidomide, thalidomide, necrostatin-1, and QNZ (EVP4593).

Examples of the "T-cell inhibitor" include, but are not limited to, abatacept.

Examples of the "angiogenesis inhibitor" include, but are not limited to, CM101, IFN-α, IL-12, platelet factor-4, suramin, semaxanib, thrombospondin, VEGFR antagonists, combinations of an angiostatic steroid and heparin, cartilage-derived angiogenesis inhibitors, matrix metalloproteinase inhibitors, batimastat, marimastat, angiostatin, endostatin, 2-methoxyestradiol, tecogalan, thrombospondin, αVβ3 inhibitors, linomide, ADH-1, E7820, and DDS formulations thereof.

Examples of the "other chemotherapeutic agent" include, but are not limited to, finasteride, sobuzoxane, obatoclax, efaproxiral, tipifarnib, and lonafarnib.

The peptide or the compound of formula (1), or a pharmaceutically acceptable salt thereof, or a combination thereof as described herein may be administered in combination with a non-drug therapy, or even more than one non-drug therapy selected, for example, from surgery, radiotherapy, gene therapy, hyperthermia, cryotherapy, or laser burning therapy. For example, the peptide or the compound of formula (1), or a pharmaceutically acceptable salt thereof, or a combination thereof as described herein may be administered before or after a non-drug therapy such as surgery, or before or after a combination of two or three non-drug therapies.

The pharmaceutical composition of the present disclosure may comprise the peptide or the compound of formula (1), or a pharmaceutically acceptable salt thereof, or a combination thereof as described herein as an active ingredient together with a pharmaceutically acceptable carrier. Also, the pharmaceutical composition of the present disclosure may further comprise, or be administered in combination with, an appropriate adjuvant for enhancing the induction of WT1-specific CTLs and/or helper T cells by the composition.

The "pharmaceutically acceptable carrier" refers to a carrier that is non-toxic to a cell or a mammal exposed to the carrier at an amount or concentration it is used. In some embodiments, a pH buffered aqueous solution is used

as a pharmaceutically acceptable carrier. Examples of the "pharmaceutically acceptable carrier" include buffering agents (such as phosphate, citrate, lactate, tartrate, trifluoroacetate and other organic acids); antioxidants (such as ascorbic acid); low molecular weight polypeptides (less than about 10 residues); proteins (such as serum albumin, gelatin or immunoglobulin); hydrophilic polymers (such as polyvinylpyrrolidone); amino acids (such as glycine, glutamine, asparagine, arginine, methionine or lysine); monosaccharides, disaccharides and other carbohydrates (such as glucose, mannose or dextrin); chelating agents (such as EDTA); sugar alcohols (such as mannitol, trehalose or sorbitol); stabilizers (such as diethylenetriaminepentaacetic acid); salt forming counterions (such as sodium); solubilizing agents (such as polysorbate 80®), and/or nonionic surfactants (such as TWEEN®, polyethylene glycol (PEG) and PLURONICS®). A macromolecular material that is metabolized slowly, such as a protein, a polypeptide, a liposome, a polysaccharide, polylactide, polyglycolic acid, polymeric amino acids, amino acid copolymers, and inactive virus particles may also be useful as a pharmaceutically acceptable carrier. For administration of a WT1 antigen peptide as described herein, the peptide may be formulated in a liposome preparation, attached to beads having a diameter of a micrometer order, or associated with a lipid carrier.

The adjuvant may be any of adjuvants as described in Clin. Microbiol. Rev., 7: 277-289, 1994. Specifically, the adjuvant may be a microorganism-derived agent, GM-CSF, a cytokine such as interleukin-2, interleukin-7, or interleukin-12, a plant-derived agent, a marine organism-derived agent, a mineral gel such as aluminum hydroxide, lysolecithin, a surfactant such as pluronic polyol, a polyanion, a peptide, or an oil emulsion (an emulsion preparation). Examples of the microorganism-derived agent include lipid A, monophosphoryl lipid A, which is a derivative of lipid A, killed bacteria (e.g., Mycobacterium bacteria such as BCG bacteria), bacterium-derived proteins, polynucleotides, Freund's incomplete adjuvant, Freund's complete adjuvant, cell wall skeleton components (e.g., BCG-CWS), trehalose dimycolate (TDM).

The adjuvant may also be a sedimentary adjuvant or an oil adjuvant. A sedimentary adjuvant can be a suspension of an inorganic substance to which a peptide can be adsorbed. Examples of the sedimentary adjuvant include sodium hydroxide, aluminum hydroxide (Alum), calcium phosphate, aluminum phosphate, alum, Pepesu, and carboxyvinyl polymer. An oil adjuvant can be an oil emulsifier that is able to emulsify a peptide by forming micelles comprising an aqueous peptide solution phase encapsulated in a mineral oil membrane. Examples of the oil adjuvant include, but are not limited to, liquid paraffin, lanolin, Freund's adjuvant (Freund's complete adjuvant, and Freund's incomplete adjuvant), Montanide, and a W/O emulsion (see WO2006/078059).

The pharmaceutical composition of the present disclosure may comprise a sugar alcohol such as mannitol, trehalose, or lactose; a pH adjusting agent conventionally used for pharmaceutical preparations, for example selected from the group consisting of hydrochloric acid, sulfuric acid, nitric acid, acetic acid, citric acid, tartaric acid, lactic acid, maleic acid, phosphoric acid, sodium hydroxide, potassium hydroxide, aqueous ammonia, sodium acetate hydrate, anhydrous sodium acetate, sodium citrate hydrate, sodium dihydrogen citrate, sodium tartrate, disodium phosphate, dipotassium phosphate, sodium dihydrogen phosphate, potassium dihydrogen phosphate, and trisodium phosphate; a filler; a buffer; a suspending agent; a wetting agent; a solubilizer; a dispersant; a preservative; and/or a coloring agent; or any other excipient.

The pharmaceutical composition of the present disclosure may be provided as a solid or liquid dosage form for oral administration, or a dosage form for parenteral administration, for example an injectable preparation, a suppository, an inhalable preparation, or a nasal preparation. Examples of the solid dosage form for oral administration include a tablet, a pill, a capsule (including a hard capsule and a soft capsule), a powder, and a granule. The pharmaceutical composition may also be formulated into such a tablet form as a sublingual tablet, a buccal tablet, or a rapidly disintegrating oral tablet. In a preferred embodiment, the pharmaceutical composition of the present disclosure is provided as an injectable preparation.

The pharmaceutical composition in a solid oral dosage form may be prepared in accordance with a conventionally known preparation method, and may comprise one or more active agents either alone or in admixture with a filler (such as lactose, mannitol, glucose, microcrystalline cellulose, or starch), a binder (such as hydroxypropyl cellulose, polyvinylpyrrolidone, or magnesium aluminometasilicate), a disintegrating agent (such as calcium cellulose glycolate), a lubricant (such as magnesium stearate), a stabilizer, a solubilizing aid (such as glutamic acid, or aspartic acid), or other appropriate excipient. A solid oral dosage form may optionally be coated with sucrose, gelatin, hydroxypropyl cellulose, hydroxypropylmethyl cellulose phthalate, or other appropriate coating agent. Two or more layers of coating may be applied on the dosage form. A solid oral dosage form may be prepared in a capsule that may be formed of a bioabsorbable material such as gelatin. A solid oral dosage form may additionally comprise a preservative, an antioxidant, a coloring agent, a sweetener, or other appropriate additive.

The pharmaceutical composition in the form of a sublingual tablet may be prepared in accordance with a conventionally known preparation method, an may comprise one or more active agents in admixture with a filler (such as lactose, mannitol, glucose, microcrystalline cellulose, colloidal silica, or starch), a binder (such as hydroxypropyl cellulose, polyvinylpyrrolidone, or magnesium aluminometasilicate), a disintegrating agent (such as starch, L-hydroxypropyl cellulose, carboxymethyl cellulose, croscarmellose sodium, or calcium cellulose glycolate), a lubricant (such as magnesium stearate), a swelling agent (such as hydroxypropyl cellulose, hydroxypropylmethyl cellulose, carbopol, carboxymethyl cellulose, polyvinyl alcohol, xanthan gum, or guar gum), a swelling aid (such as glucose, fructose, mannitol, xylitol, erythritol, maltose, trehalose, phosphate, citrate, silicate, glycine, glutamic acid, or arginine), a stabilizer, a solubilizing aid (such as polyethylene glycol, propylene glycol, glutamic acid, or aspartic acid), a flavoring agent (such as an orange, strawberry, mint, lemon, or vanilla flavor), or other appropriate excipient. A sublingual tablet may optionally be coated with sucrose, gelatin, hydroxypropyl cellulose, hydroxypropylmethyl cellulose phthalate, or other appropriate coating agent. Two or more layers of coating may be applied on a tablet. A sublingual tablet may additionally comprise a preservative, an antioxidant, a coloring agent, a sweetener, or other appropriate additive.

The pharmaceutical composition in the form of a buccal tablet may be prepared in accordance with a conventionally known preparation method, and may comprise one or more active agents in admixture with a filler (such as lactose, mannitol, glucose, microcrystalline cellulose, colloidal silica, or starch), a binder (such as hydroxypropyl cellulose, polyvinylpyrrolidone, or magnesium aluminometasilicate), a disintegrating agent (such as starch, L-hydroxypropyl cellulose, carboxymethyl cellulose, croscarmellose sodium, or calcium cellulose glycolate), a lubricant (such as magnesium stearate), an adhesive agent (such as hydroxypropyl cellulose, hydroxypropylmethyl cellulose, carbopol, carboxymethyl cellulose, polyvinyl alcohol, xanthan gum, or guar gum), an adhesive aid (such as glucose, fructose, mannitol, xylitol, erythritol, maltose, trehalose, phosphate, citrate, silicate, glycine, glutamic acid, or arginine), a stabilizer, a solubilizing aid (such as polyethylene glycol, propylene glycol, glutamic acid, or aspartic acid), a flavoring agent (such as an orange, strawberry, mint, lemon, or vanilla flavor), or other appropriate excipient. A buccal tablet may optionally be coated with sucrose, gelatin, hydroxypropyl cellulose, hydroxypropylmethyl cellulose phthalate, or other appropriate coating agent. Two or more layers of coating may be applied on a tablet. A buccal tablet may additionally comprise a preservative, an antioxidant, a coloring agent, a sweetener, or other appropriate additive.

The pharmaceutical composition in the form of a rapidly disintegrating oral tablet may be prepared in accordance with a conventionally known preparation method, which may comprise steps of providing one or more active agents in a powder or granule form, and optionally coating them with a coating agent (such as ethyl cellulose, hydroxypropyl cellulose, hydroxypropylmethyl cellulose, or an acrylic acid/methacrylic acid copolymer) or a plasticizer (such as polyethylene glycol, or triethyl citrate), and admixing them with a filler (such as lactose, mannitol, glucose, microcrystalline cellulose, colloidal silica, or starch), a binder (such as hydroxypropyl cellulose, polyvinylpyrrolidone, or magnesium aluminometasilicate), a disintegrating agent (such as starch, L-hydroxypropyl cellulose, carboxymethyl cellulose, croscarmellose sodium, or calcium cellulose glycolate), a lubricant (such as magnesium stearate), a disintegrating aid (such as glucose, fructose, mannitol, xylitol, erythritol, maltose, trehalose, phosphate, citrate, silicate, glycine, glutamic acid, or arginine), a stabilizer, a solubilizing aid (such as polyethylene glycol, propylene glycol, glutamic acid, or aspartic acid), a flavoring agent (such as an orange, strawberry, mint, lemon, or vanilla flavor), or other appropriate excipient. A rapidly disintegrating oral tablet may optionally be coated with sucrose, gelatin, hydroxypropyl cellulose, hydroxypropylmethyl cellulose phthalate, or other appropriate coating agent. Two or more layers of coating may be applied on a tablet. A rapidly disintegrating oral tablet may additionally comprise a preservative, an antioxidant, a coloring agent, a sweetener, or other appropriate additive.

The pharmaceutical composition in a liquid dosage form for oral administration may be in the form of a solution, a suspension, an emulsion, a syrup, or an elixir, in which one or more active agents are dissolved, dispersed or emulsified in a conventionally used pharmaceutically acceptable vehicle (such as purified water, ethanol, or a mixture thereof). A liquid oral dosage form may optionally comprise a wetting agent, a dispersant, an emulsifier, a sweetener, a flavoring agent, a preservative, a buffer, or other appropriate additive.

The pharmaceutical composition in a dosage form for topical application may be in the form of an ointment, a gel, a cream, a plaster, a patch, a liniment, a spray, an inhalant, an aerosol, an eye drop, or a nasal drop, which may be prepared in accordance with a conventionally known preparation method, and may comprise one or more active agents.

An ointment may be prepared in accordance with a conventionally known preparation method, for example by incorporating one or more active agents in an ointment base by grinding or melting. For preparing an ointment, any conventionally used ointment base may be used, which may comprise a higher fatty acid or fatty acid ester (such as adipic acid, myristic acid, palmitic acid, stearic acid, or oleic acid, or an ester thereof), a wax (such as beeswax, spermaceti, or ceresin), a surfactant (such as polyoxyethylene alkyl ether phosphate), a higher alcohol (such as cetanol, stearyl alcohol, or cetostearyl alcohol), a silicone oil (such as dimethylpolysiloxane), a hydrocarbon (such as a hydrophilic petrolatum, white petrolatum, purified lanolin, or liquid paraffin), a glycol (such as ethylene glycol, diethylene glycol, propylene glycol, polyethylene glycol, or macrogol), a vegetable oil (such as castor oil, olive oil, sesame oil, or turpentine oil), an animal oil (such as mink oil, egg-yolk oil, squalane, or squalene), water, an absorption enhancer, a skin protective agent, or a combination thereof. An ointment may additionally comprise a humectant, a preservative, a stabilizer, an antioxidant, a fragrance, or other appropriate additive.

The pharmaceutical composition in a gel form may be prepared in accordance with a conventionally known preparation method, for example by incorporating one or more active agents in a gel base by melting. For preparing a gel, any conventionally used pharmaceutical gel base may be used, which may comprise a lower alcohol (such as ethanol, or isopropyl alcohol), a gelling agent (such as carboxymethyl cellulose, hydroxyethyl cellulose, hydroxypropyl

cellulose, or ethyl cellulose), a neutralizing agent (such as triethanolamine, or diisopropanolamine), a surfactant (such as polyoxyethylene glycol monostearate), a gum, water, an absorption enhancer, a skin protective agent, or a combination thereof. A gel may additionally comprise a preservative, an antioxidant, a fragrance, or any other appropriate additive(s). The pharmaceutical composition in a cream form may be prepared in accordance with a conventionally known preparation method, for example by incorporating one or more active agents in a pharmaceutical cream base by melting or emulsification. For preparing a cream, any conventionally used pharmaceutical cream base may be used, which may comprise a higher fatty acid ester, a lower alcohol, a hydrocarbon, a polyhydric alcohol (such as propylene glycol, or 1,3-butylene glycol), a higher alcohol (such as 2-hexyldecanol, or cetanol), an emulsifier (such as a polyoxyethylene alkyl ether, or a fatty acid ester), water, an absorption enhancer, a skin protective agent, or a combination thereof. A cream may additionally comprise a preservative, an antioxidant, a fragrance, or other appropriate additive.

The pharmaceutical composition in the form of a plaster may be prepared in accordance with a conventionally known preparation method, for example by incorporating one or more active agents in a plaster base by melting, and applying the mixture onto a support. For preparing a plaster, any conventionally used pharmaceutical plaster base may be used, which may comprise a thickening agent (such as polyacrylic acid, polyvinylpyrrolidone, gum arabic, starch, gelatin, or methyl cellulose), a humectant (such as urea, glycerol, or propylene glycol), a filler (such as kaolin, zinc oxide, talc, calcium, or magnesium), water, a solubilizing aid, a tackifier, a skin protective agent, or a combination thereof. A plaster may additionally comprise a preservative, an antioxidant, a fragrance, or other appropriate additive.

The pharmaceutical composition in the form of a patch may be prepared in accordance with a conventionally known preparation method, for example by incorporating one or more active agents in a patch base by melting, and applying the mixture onto a support. For preparing a patch, any conventionally used pharmaceutical patch base may be used, which may comprise a polymer, an oil or fat, a higher fatty acid, a tackifier, a skin protective agent, or a combination thereof. A patch may additionally comprise a preservative, an antioxidant, a fragrance, or other appropriate additive.

The pharmaceutical composition in the form of a liniment may be prepared in accordance with a conventionally known preparation method, for example by dissolving, dispersing or emulsifying one or more active agents in a vehicle that may comprise water, an alcohol (such as ethanol, or polyethylene glycol), a higher fatty acid, glycerol, soap, an emulsifier, a dispersant, or a combination thereof. A liniment may additionally comprise a preservative, an antioxidant, a fragrance, or other appropriate additive.

The pharmaceutical composition in the form of a spray, or an inhalant may comprise active agent(s), and optionally a stabilizing agent such as sodium hydrogen sulfite, or a tonicity agent or buffer, such as sodium chloride, sodium citrate or citric acid, in a vehicle.

The pharmaceutical composition in a dosage form for injection may be in the form of a solution, a suspension, or an emulsion, which comprises one or more active agents dissolved, dispersed or emulsified in a liquid for injection, or may be provided as a solid formulation comprising active agent(s) to be dissolved or dispersed in a liquid for injection before use. A liquid for injection may comprise distilled water for injection, physiological saline, a vegetable oil, propylene glycol, polyethylene glycol, an alcohol such as ethanol, or a combination thereof. An injectable preparation may additionally comprise a stabilizer, a solubilizing aid (such as glutamic acid, aspartic acid, or polysorbate 80®), a dispersant, an emulsifier, an analgesic, a buffer, a preservative, or other appropriate additive. For providing an injectable preparation as a sterilized preparation, it may be subjected to sterilization in the final step of its production, or produced aseptically throughout its production. A formulation for injection may be provided as a sterilized solid formulation, for example a lyophilized formulation, which may be reconstituted in sterilized water for injection or other appropriate sterilized liquid before use.

The pharmaceutical composition in a dosage form for inhalation may be in the form of an aerosol, an inhalable powder, or an inhalable liquid, or may be provided as a liquid concentrate that is to be dissolved or dispersed in water or other appropriate vehicle to form an inhalable preparation before use. A preparation for inhalation may be prepared in accordance with a conventionally known preparation method. An inhalable liquid may optionally comprise a preservative (such as benzalkonium chloride, or paraben), a coloring agent, a buffer (such as sodium phosphate, or sodium acetate), a tonicity agent (such as sodium chloride, or concentrated glycerin), a thickening agent (such as a carboxyvinyl polymer), an absorption enhancer, or other appropriate additive.

An inhalable powder may optionally comprise a lubricant (such as stearic acid, or a salt thereof), a binder (such as starch, or dextrin), a filler (such as lactose, or cellulose), a coloring agent, a preservative (such as benzalkonium chloride, or paraben), an absorption enhancer, or other appropriate additive.

For administration of an inhalable liquid, a spray device (such as an atomizer, or a nebulizer) is usually used. An inhalable powder is usually dispensed from a powder inhalation device.

The pharmaceutical composition in the form of a spray may comprise active agent(s), and optionally a stabilizing agent (such as sodium hydrogen sulfite), or a tonicity agent or buffer (such as sodium chloride, sodium citrate, or citric acid) in a vehicle. A spray may be prepared in accordance with a preparation method as described, for example, in US 2,868,691, or US 3,095,355.

The pharmaceutical composition may be prepared in other parenteral dosage form. For example, one or more active agents may be formulated into a rectal suppository or a vaginal pessary by a conventionally known preparation method.

In one embodiment, the composition comprising the peptide or the compound of formula (1), or a pharmaceutically acceptable salt thereof, or a combination thereof as described herein comprises one or more pharmaceutically acceptable carriers selected from the group consisting of trehalose, mannitol, methionine, citric acid, lactic acid, tartaric acid, acetic acid, trifluoroacetic acid, and a pH adjusting agent.

The peptide or the compound of formula (1), or a pharmaceutically acceptable salt thereof, or a combination thereof as described herein can be administered to a subject by an appropriate method depending on a disease of the subject to treat, a condition of the subject, a target site of the administration, or other factor. For example, administration by intravenous, intramuscular, intradermal, intraperitoneal, subcutaneous or intraspinal injection or infusion, or other parenteral administration may be useful. The term "parenteral administration" as used herein refers to a usual mode of administration by injection or infusion other than enteral or topical administration, and includes, but is not limited to, intravenous, intramuscular, intraarterial, intrathecal, intracapsular, intraorbital, intracardiac, intradermal, intraperitoneal, intratracheal, subcutaneous, subepidermal, intraarticular, subcapsular, subarachnoidal, intraspinal, epidural or infrasternal injection or infusion. The peptide or compound as described herein may be administered in a lymphocyte therapy or a DC (dendritic cell) therapy. When an immunomodulator is coadministered, it may be administered transdermally, or transmucosally by intranasal, buccal, vaginal, rectal, or sublingual administration.

Frequency of dose, or dosing interval may be appropriately selected depending on a disease to treat by the drug, a condition of a recipient of the drug, a route of administration of the drug, or other factor. Administration is usually repeated, preferably every few days or few months.

The peptide or the compound of formula (1), or a pharmaceutically acceptable salt thereof, or a coadministration drug, if any, as described herein can be administered to a subject in an appropriate amount depending on a disease of the subject to treat, a condition of the subject, a particular route of the administration, or other factor. The peptide or the compound, or a pharmaceutically acceptable salt thereof may usually be administered in an amount of 0.0001 mg to 1000 mg, preferably 0.001 mg to 1000 mg, more preferably 0.1 mg to 10 mg, at one time. A coadministration drug may be administered in an amount appropriately selected on the basis of a known clinical dose of the drug. For example, an immunomodulator as a coadministration drug may usually be administered in an amount of 0.0001 mg to 1000 mg per kg body weight, preferably 0.001 mg to 1000 mg per kg body weight, more preferably 0.1 mg to 10 mg per kg body weight.

When more than one active agent is incorporated in a single composition, they may be incorporated at an amount ratio appropriately selected depending on a disease to treat, a condition of a recipient of the composition, a route of administration of the composition, or other factor. For example, for treating a human subject by administration of a composition comprising the peptide and/or the compound as described herein and an immunomodulator or other coadministration drug, the immunomodulator or coadministration drug may be used in an amount of 0.01 to 100 parts by weight relative to the peptide and/or the compound.

The term "subject" as used herein includes human and non-human mammals. Non-human mammals include, but are not limited to, non-human primate, ovine, canine, feline, equine, and bovine. A human subject, especially a human subject in need of potentiation of immune response is preferred.

The term "effective amount" as used herein means an amount of an active agent that completely or partially inhibits the progression of a cancer, or at least partially reduces one or more symptoms of a cancer. An effective amount may be a therapeutically or prophylactically effective amount. An effective amount an agent is determined depending on the age or sex of a recipient of the agent, the type or severity of a condition to treat with the agent, a desired outcome of the treatment with the agent, or other factor. A person skilled in the art can determine an effective amount for a particular patient.

The present invention is useful for treating or preventing (including prevention of recurrence) a "cancer accompanied by WT1 gene expression" or a "cancer accompanied by an elevated level of WT1 gene expression". Examples of such a cancer include a hematologic cancer such as leukemia, myelodysplastic syndrome, multiple myeloma, and malignant lymphoma, and a solid tumor such as gastric cancer, colorectal cancer, lung cancer, breast cancer, germ cell cancer, liver cancer, skin cancer, urinary bladder cancer, prostate cancer, uterine cancer, cervical cancer, ovarian cancer, glioblastoma multiforme, malignant melanoma, non-small cell lung cancer, renal cell carcinoma or brain tumor.

Further examples of cancers that can be treated or prevented by the present invention include bone cancer, pancreatic cancer, cancer of the head or neck, cutaneous or intraocular malignant melanoma, rectal cancer, cancer of the anal region, testicular cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia such as acute myeloid leukemia, chronic myeloid leukemia, acute lymphoblastic leukemia, or chronic lymphocytic leukemia, childhood solid tumor, lymphocytic lymphoma, cancer of the kidney or ureter, carcinoma of the

renal pelvis, central nervous system (CNS) tumor, primary CNS lymphoma, tumor angiogenesis, spinal tumor, brainstem glioma, pituitary adenoma, Kaposi's sarcoma, epidermoid cancer, squamous cell cancer, T-cell lymphoma, environmentally induced cancers including asbestos-induced cancer, and combinations of the cancers as described above.

An unwanted side effect, if any, of a peptide or a compound of formula (1), or a pharmaceutically acceptable salt thereof, or a combination thereof as described herein can be counteracted by coadministration of an agent appropriate for the purpose, such as an antiemetic agent, sleep-inducing agent, or anticonvulsant.

The peptide or the compound of formula (1), or a pharmaceutically acceptable salt thereof, or a combination thereof as described herein may be used in combination with other cancer antigen peptide. For example, a WT1 helper peptide can be administered before or after a WT1 killer peptide or the compound of formula (1) as described herein is administered. Such a combination is therapeutically beneficial, because the therapeutic effect of a WT1 killer peptide through the induction of CTL activity can be enhanced by a WT1 helper peptide that is able to induce WT1-specific helper T cells and activate B cells and other T cells.

Examples of such other cancer antigen peptide include peptides, or derivatives or conjugates thereof as described in the following publications: WO 2000/006602, WO 2002/079253, WO 2003/106682, WO 2004/026897, WO 2004/063903, WO 2007/063903, WO 2010/123065, WO 2014/157692, WO 2005/053618, WO 2007/047764, WO 2007/120673, WO 2005/045027, WO 2010037395, WO 2000/018795, WO 2002/028414, WO 2003/037060, and WO 2004/100870.

In another aspect, the present disclosure provides an antigen presenting cell (for example, a dendritic cell, B-lymphocyte, macrophage) that presents a WT1 peptide as described herein via an MHC class I molecule. WT1-specific cytotoxic T cells are induced efficiently by using the antigen-presenting cells of the present disclosure.

In another aspect, the present disclosure provides a method of inducing antigen presenting cells, wherein the method comprises culturing immature antigen presenting cells in the presence of the WT1 peptide or the compound of formula (1) as described herein, and inducing antigen presenting cells that present the peptide on the cells via an MHC class I molecule from the immature antigen presenting cells. As used herein, the term "immature antigen presenting cells" refers to cells that can mature into antigen presenting cells such as dendritic cells, B-lymphocytes or macrophages. The immature antigen presenting cells are found, for example, in a peripheral mononuclear cell population. Therefore, such a cell population may be cultured in the presence of a WT1 peptide or the compound of formula (1) in the method of inducing antigen presenting cells.

In another aspect, the present disclosure provides a method of preventing or treating a cancer in a subject, wherein the method comprises administering antigen presenting cells that present a WT1 peptide as described herein on the cells via an MHC class I molecule to a subject, wherein the subject has the same MHC class I molecule. The antigen presenting cells may be administered by any method appropriately selected depending on a disease to treat, a condition of the subject, or a target site of the administration, or other factor. The antigen presenting cells may be administered intravenously, intradermally, subcutaneously, intramuscularly, intranasally, or orally, or by other administration route.

In another aspect, the present disclosure relates to WT1-specific cytotoxic T cells induced by a WT1 peptide as described herein. The WT1-specific cytotoxic T cells can induce cytotoxicity in tumor cells expressing WT1.

In another aspect, the present disclosure relates to a method of inducing WT1-specific cytotoxic T cells, wherein the method comprises culturing peripheral blood mononuclear cells in the presence of a WT1 peptide or a compound of formula (1) of as described herein, so that WT1-specific cytotoxic T cells are induced from the peripheral blood mononuclear cells. In particular, when peripheral blood mononuclear cells are cultured in the presence of a WT1 peptide or a compound of formula (1), WT1-specific cytotoxic T cells are induced from precursor cells in the peripheral blood mononuclear cell population. The WT1-specific cytotoxic T cells obtained by the method can be administered to a subject to treat or prevent a cancer in the subject.

In another aspect, the present disclosure provides a method of preventing or treating a cancer, wherein the method comprises administering WT1-specific cytotoxic T cells to a subject. The WT1-specific cytotoxic T cells can be administered by a method appropriately selected depending on a disease to treat, a condition of the subject, or a target site of the administration, or other factor. The WT1-Specific cytotoxic T cells may be administered intravenously, intradermally, subcutaneously, intramuscularly, intranasally, or orally, or by other administration route.

In another aspect, the present disclosure provides a kit for inducing WT1-specific cytotoxic T cells, wherein the kit comprises a WT1 peptide or the compound of formula (1) as described herein as a component. In an embodiment, the kit is for use in the method for inducing WT1-specific cytotoxic T cells as described above. Besides a WT1 peptide or the compound of formula (1), the kit may comprise, for example, a means for harvesting peripheral blood mononuclear cells, an adjuvant, or a container for reaction, and usually instructions for use.

In another aspect, the present disclosure provides a kit for preventing or treating a cancer, wherein the kit comprises a peptide, the compound of formula (1), a polynucleotide or an expression vector as described herein as a component. In an embodiment, the kit is for use in inducing antigen presenting cells that present a WT1 peptide on the cells via an MHC class I molecule. Besides the essential component, the kit may comprise, for example, a means for

obtaining a sample, or a container for reaction, and usually instructions for use.

Examples

The present invention is described in further detail in the following Examples, which are not in any way intended to limit the scope of the invention.

Example 1

Synthesis of a peptide consisting of the amino acid sequence: HCYTWNQMNL (His-Cys-Tyr-Thr-Trp-Asn-Gln-Met-Asn-Leu) (SEQ ID NO: 34)

The peptide was synthesized by an Fmoc/tBu method for solid-phase peptide synthesis. Specifically, peptide chain elongation was performed by using as a starting material 1.00 g of Fmoc-Leu-Alko-PEG resin (wherein Fmoc represents 9-fluorenylmethyloxycarbonyl, Alko represents p-alkoxybenzyl alcohol, and PEG represents polyethylene glycol) (Watanabe Chemical Industries; 0.24 mmol/g, 0.24 mmol) in CS Bio CS336X peptide synthesizer. For removing the Fmoc protecting group, the resin was treated with a solution of 20% piperidine in N,N-dimethylformamide (DMF) for 5 minutes or 20 minutes. For coupling a protected amino acid to the resin, a solution of 1.05 mmol of a protected amino acid, 1 mmol of O-(benzotriazol-1-yl)-N,N,N',N'-tetramethyluronium hexafluorophosphate (HBTU), 2 mmol of N,N-diisopropylethylamine (DIPEA) in DMF was added to the resin and reacted for one hour. The resin from the reaction was washed with DMF and diethyl ether, and dried under vacuum. The resin to which a synthesized peptide chain was attached was incubated for two hours in 10 ml of a mixture of trifluoroacetic acid (TFA)/water/triisopropylsilane (TIS)/ethane dithiol (EDT) (volume ratio: 94/2.5/2.5/1) at room temperature. The resin was then filtered off. The filtrate was concentrated under vacuum, and then cooled on ice and diluted in 50 ml of diethyl ether to precipitate the peptide. The precipitated peptide was filtered, washed with ether, and dried under vacuum. A crude peptide product thus obtained was dissolved in a mixture of 20% acetic acid/acetonitrile (volume ratio 1/1) and subjected to purification under the conditions described below. The peptide consisting of the sequence: HCYTWNQMNL (His-Cys-Tyr-Thr-Trp-Asn-Gln-Met-Asn-Leu) (SEQ ID NO: 34) was obtained as TFA salt (0.14 g).

Purification conditions:

HPLC System: Gilson high throughput preparative HPLC system
Column: YMC ODS-A 3 cm ϕ x 25 cm, 10μm

Eluent 1: 0.1% TFA in water
Eluent 2: 0.035% TFA in acetonitrile

Flow rate: 20 ml/min

Gradient method:

| Time (min.) | Concentration of Eluent 2 (%) |
|---|---|
| 0 | 10 |
| 15 | 10 |
| 25 | 22 |
| 45 | 27 |

Identification of the product was conducted by analysis under the following conditions:

MS System: Shimazu LCMS-IT-TOF system

Column: Kinetex Minibore column, 2.1 mm ϕ x 50 mm, 1.7 μm

Eluent 1: 0.1% formic acid in water

Eluent 2: 0.1% formic acid in acetonitrile

Flow rate: 1.2 ml/min

Gradient method:

| Time (min.) | Concentration of Eluent 2 (%) |
|---|---|
| 0 | 10 |
| 1.4 | 95 |
| 1.6 | 95 |
| MS: m/z=655.8 $[M+2H]^{2+}$, retention time: 0.50 min. | |

Examples 2 to 20

In accordance with the procedure as described in Example 1, peptides listed in Table 1 below were synthesized from corresponding starting materials and obtained as TFA salts.

[Table 1]

| Example No. | SEQ ID NO: | Amino Acid Sequence | LC-TOFMS (m/z, retention time (min.)) |
|---|---|---|---|
| 2 | 55 | ACYTWNQMNL | 622.23$[M+2H]^{2+}$, 0.91 |
| 3 | 10 | CCYTWNQMNL | 638.70$[M+2H]^{2+}$, 1.03 |
| 4 | 56 | DCYTWNQMNL | 644.23$[M+2H]^{2+}$, 0.92 |
| 5 | 32 | ECYTWNQMNL | 651.$[M+2H]^{2+}$, 0.83 |
| 6 | 38 | FCYTWNQMNL | 660.25$[M+2H]^{2+}$, 0.95 |
| 7 | 57 | GCYTWNQMNL | 615.22$[M+2H]^{2+}$, 1.02 |
| 8 | 58 | ICYTWNQMNL | 643.26$[M+2H]^{2+}$, 0.93 |
| 9 | 36 | KCYTWNQMNL | 651.$[M+2H]^{2+}$, 0.84 |
| 10 | 59 | LCYTWNQMNL | 643.25$[M+2H]^{2+}$, 0.94 |
| 11 | 60 | MCYTWNQMNL | 652.23$[M+2H]^{2+}$, 0.97 |
| 12 | 61 | NCYTWNQMNL | 643.73$[M+2H]^{2+}$, 0.95 |
| 13 | 62 | PCYTWNQMNL | 635.24$[M+2H]^{2+}$, 0.93 |
| 14 | 30 | QCYTWNQMNL | 650.77 $[M+2H]^{2+}$, 0.85 |
| 15 | 28 | RCYTWNQMNL | 665.8 $[M+2H]^{2+}$, 0.92 |
| 16 | 63 | SCYTWNQMNL | 630.23$[M+2H]^{2+}$, 0.92 |
| 17 | 64 | TCYTWNQMNL | 637.23$[M+2H]^{2+}$, 0.96 |
| 18 | 65 | VCYTWNQMNL | 636.24$[M+2H]^{2+}$, 0.93 |
| 19 | 66 | WCYTWNQMNL | 679.71$[M+2H]^{2+}$, 0.96 |
| 20 | 40 | YCYTWNQMNL | 668.27$[M+2H]^{2+}$, 0.87 |

Reference Example 1

In accordance with the procedure as described in WO 2014157692, a compound listed in Table 2 below was synthesized from corresponding starting materials and obtained as a TFA salt (C-C shown in the following formula means that the C residues are linked together by a disulfide bond.).

[Table 2]

| Reference Example No. | Formula No. | Structure | LC-TOFMS (m/z, retention time (min.)) |
|---|---|---|---|
| 1 | (5) | CRMFPNAPYL<br>\|<br>CYTWNQMNL | 794.60$[M+3H]^{3+}$, 0.88 |

Reference Examples 2 to 5

In accordance with the procedure as described in Example 1, peptides listed in Table 3 below were synthesized from corresponding starting materials and obtained as TFA salts.

[Table 3]

| Reference Example No. | SEQ ID NO: | Amino Acid Sequence | LC-TOFMS (m/z, retention time (min.)) |
|---|---|---|---|
| 2 | 2 | RMFPNAPYL | 554.73$[M+2H]^{2+}$, 0.82 |
| 3 | 8 | CRMFPNAPYL | 606.22$[M+2H]^{2+}$, 0.81 |
| 4 | 67 | C(Npys)RMFPNAPYL | 683.21$[M+2H]^{2+}$, 0.95 |
| 5 | 4 | CYTWNQMNL | 586.69$[M+2H]^{2+}$, 0.87 |

Examples 21 to 26

In accordance with Reference Example 1, compounds listed in Table 4 below were synthesized from corresponding starting materials and obtained as TFA salts (C-C shown in the following formulae means that the C residues are linked together by a disulfide bond.).

[Table 4]

| Example No. | Formula No. | Structure | LC-TOFMS (m/z, retention time (min.)) |
|---|---|---|---|
| 21 | (15) | CRMFPNAPYL<br>\|<br>HCYTWNQMNL | 1260.03$[M+2H]^{2+}$, 0.94 |
| 22 | (10) | CRMFPNAPYL<br>\|<br>RCYTWNQMNL | 1269.55$[M+2H]^{2+}$, 1.02 |
| 23 | (11) | CRMFPNAPYL<br>\|<br>KCYTWNQMNL | 1255.55$[M+2H]^{2+}$, 1.03 |
| 24 | (13) | CRMFPNAPYL<br>\|<br>ECYTWNQMNL | 837.68$[M+3H]^{3+}$, 0.92 |
| 25 | (12) | CRMFPNAPYL<br>\|<br>YCYTWNQMNL | 849.03$[M+3H]^{3+}$, 0.93 |
| 26 | (14) | CRMFPNAPYL<br>\|<br>FCYTWNQMNL | 843.70$[M+3H]^{2+}$, 0.88 |

Experimental Example 1

*In vivo* CTL induction in HLA-A*24:02 transgenic mouse

The compound of formula (5) synthesized in Reference Example 1 and the compound of formula (15) synthesized in Example 21 were evaluated for ability to induce CTLs *in vivo* in an HLA-A*24:02 transgenic mouse.
The sequence RMFPNAPYL (SEQ ID NO: 2) included in the compound of formula (15):

CRMFPNAPYL
HCYTWNQMNL (15)

wherein C-C shown in the formula means that the C residues are linked together by a disulfide bond,
corresponds to an HLA-A*02:01-restricted WT1 peptide, and the sequence CYTWNQMNL (SEQ ID NO: 4) corresponds to an HLA-A*24:02-restricted WT1 peptide.

The HLA-A*24:02 transgenic mouse (C57BL/6CrHLA-A2402/$K^b$) is a mouse that expresses a chimeric HLA of a human MHC, HLA-A*24:02 with a mouse MHC, H-$2K^b$. The mouse is useful for screening peptides for potential ability to induce CTLs in HLA-A*24:02-positive human (Int J Cancer. 2002; 100:565-70).

Induction of CTLs specific to the peptide of SEQ ID NO: 4 by the compounds of formula (5) and formula (15) in the mouse was measured, as the level of IFNγ produced by splenocytes from the mouse to which the compounds of formula (5) and formula (15) had been administered upon stimulation of the cells with the peptide of SEQ ID NO: 4.

Specifically, the compound of formula (5) was dissolved in dimethyl sulfoxide (hereinafter described as DMSO) at a concentration of 133.3 mg/mL. The solution was diluted with water for injection to a concentration of 10 mg/mL, and then converted to an emulsion by addition of an equal volume of Montanide ISA 51 VG. The emulsion was injected to mice intradermally at two sites in the tail base area in an amount for administering 500 μg of the compound of formula (5) per site. On the other hand, a solution of the compound of formula (15) (146.7 mg/mL) in DMSO was dissolved, diluted with water for injection to a concentration of the compound of formula (15) of 11 mg/mL, and then converted into an emulsion by the addition of an equal volume of Montanide ISA 51 VG. The compound emulsion was injected to mice intradermally at two sites in the tail base area in an amount for administering 550 μg of the compound of formula (15) per site. The molar ratio of the compound of formula (5) and the compound of formula (15) administered per mouse was 1:1. The concentrations of DMSO contained in emulsions were also fixed. One week after the administration, the mice were sacrificed with $CO_2$ gas. Splenocytes were harvested from spleens removed from the mice. For detecting IFNy-producing splenocytes, an IFNγ ELISPOT assay kit was used. In particular, an ELISPOT plate was treated with an anti-mouse-IFNγ antibody on the day before preparation of the splenocyte samples. On the next day, the plate was blocked by treatment with an RPMI 1640 medium with 10% FBS. To the blocked ELISPOT plate, the splenocyte samples from the HLA-A*24:02 transgenic mice were added at 2.5 x $10^5$ cells/well. For *in vitro* stimulation of the cells, the peptide of SEQ ID NO: 4 was dissolved in DMSO at a concentration of 40 mg/ml, diluted with RPMI 1640 with 10% FBS to 40 μg/ml, and added to the splenocyte-containing wells at a final concentration of 10 μg/ml. The plate was incubated for 18-20 hours at 37°C under an atmosphere of 5% $CO_2$. Then, after removal of the culture medium from the wells, the ELISPOT plate was subjected to treatment for cell staining in accordance with the manufacturer's protocol. Stained spots were counted on ImmunoSpot S6 Analyzer (C.T.L.).

Figure 1 shows results from the IFNγ ELISPOT assay using the HLA-A*24:02 transgenic mouse. The scale on the vertical axis of the graph of Figure 1 indicates the number of cells (CTLs) that produced IFNγ in response to the stimulation with the peptide of SEQ ID NO: 4, which were comprised in the cells seeded on the plate. The compound administered to the mice are described under the bars. The black bar of Figure 1 shows the number of splenocytes of the HLA-A*24:02 transgenic mouse that produced IFNγ in response to the stimulation with the peptide of SEQ ID NO: 4. The white bar shows the number of splenocytes of the HLA-A*24:02 transgenic mouse that produced IFNγ in the absence of the peptide stimulation. Therefore, the difference in cell count between the black bar and the white bar shows the number of CLTs specific to a peptide. The value of the white bar is not found in Figure 1. This shows that HLA-A*24:02 transgenic splenocytes did not react in the absence of the target peptide. The results show that the compound of formula (5) or formula (15) induced CTLs responsive to the peptide of SEQ ID NO: 4 in the HLA-A*24:02 transgenic mouse, and that many CLTs responsive to the peptide of SEQ ID NO: 4 were found when the compound of formula (15) was administered as compared with the compound of formula (5).

The results demonstrate that the compound of formula (15) can induce CTLs specific to the peptide of SEQ ID NO: 4. The results also demonstrate that the compound of formula (15) induces more CTLs responsive to the peptide of SEQ ID NO: 4 as compared with the compound of formula (5).

Experimental Example 2

*In vivo* CTL induction in HLA-A*24:02 transgenic mouse

In accordance with the procedure as described in Experimental Example 1, the compound of formula (5) synthesized in Reference Example 1 and the compound of formula (10) synthesized in Example 22 were evaluated for ability to induce CTLs *in vivo* in an HLA-A*24:02 transgenic mouse.
The sequence RMFPNAPYL (SEQ ID NO: 2) included in the compound of formula (10):

```
 CRMFPNAPYL
 |              (10)
RCYTWNQMNL
```

wherein C-C shown in the formula means that the C residues are linked together by a disulfide bond, corresponds to an HLA-A*02:01-restricted WT1 peptide, and the sequence CYTWNQMNL (SEQ ID NO: 4) corresponds to an HLA-A*24:02-restricted WT1 peptide.

Specifically, in accordance with the procedure as described in Experimental Example 1, an emulsion of the formula (5) was administered. On the othre hand, the compound of formula (10) was dissolved in DMSO at a concentration of 142.7 mg/mL. The solution was diluted with water for injection to a concentration of 10.7 mg/mL, and then converted to an emulsion by addition of an equal volume of Montanide ISA 51 VG. The emulsion was injected to mice intradermally at two sites in the tail base area in an amount for administering 535 $\mu$g of the compound of formula (10) per site. The molar ratio of the compound of formula (5) and the compound of formula (10) administered per mouse was 1:1.

Figure 2 shows results from the IFN$\gamma$ ELISPOT assay using the HLA-A*24:02 transgenic mouse. The results show that the compound of formula (5) or formula (10) induced CTLs responsive to the peptide of SEQ ID NO: 4 in the HLA-A*24:02 transgenic mouse, and that many CTLs responsive to the peptide of SEQ ID NO: 4 were found when the compound of formula (10) was administered as compared with the compound of formula (5).

The results demonstrate that the compound of formula (10) can induce CTLs specific to the peptide of SEQ ID NO: 4. The results also demonstrate that the compound of formula (10) induces more CTLs responsive to the peptide of SEQ ID NO: 4 as compared with the compound of formula (5).

Experimental Example 3

*In vivo* CTL induction in HLA-A*24:02 transgenic mouse

In accordance with the procedure as described in Experimental Example 1, the compound of formula (5) synthesized in Reference Example 1 and the compound of formula (11) synthesized in Example 23 were evaluated for ability to induce CTLs *in vivo* in an HLA-A*24:02 transgenic mouse.
The sequence RMFPNAPYL (SEQ ID NO: 2) included in the compound of formula (11):

```
 CRMFPNAPYL
 |              (11)
KCYTWNQMNL
```

wherein C-C shown in the formula means that the C residues are linked together by a disulfide bond, corresponds to an HLA-A*02:01-restricted WT1 peptide, and the sequence CYTWNQMNL (SEQ ID NO: 4) corresponds to an HLA-A*24:02-restricted WT1 peptide.

Specifically, in accordance with the procedure as described in Experimental Example 1, an emulsion of the formula (5) was administered. On the other hand, the compound of formula (11) was dissolved in DMSO at a concentration of 140 mg/mL. The solution was diluted with water for injection to a concentration of 10.5 mg/mL, and then converted to an emulsion by addition of an equal volume of Montanide ISA 51 VG. The emulsion was injected to mice intradermally at two sites in the tail base area in an amount for administering 525 $\mu$g of the compound of formula (11) per site. The molar ratio of the compound of formula (5) and the compound of formula (11) administered per mouse was 1:1.

Figure 3 shows results from the IFN$\gamma$ ELISPOT assay using the HLA-A*24:02 transgenic mouse. The results show that the compound of formula (5) or formula (11) induced CTLs responsive to the peptide of SEQ ID NO: 4 in the HLA-A*24:02 transgenic mouse, and that many CTLs responsive to the peptide of SEQ ID NO: 4 were found when the compound of formula (11) was administered as compared with the compound of formula (5).

The results demonstrate that the compound of formula (11) can induce CTLs specific to the peptide of SEQ ID NO: 4. The results also demonstrate that the compound of formula (11) induced more CTLs responsive to the peptide

of SEQ ID NO: 4 as compared with the compound of formula (5).

Experimental Example 4

*In vivo* CTL induction in HLA-A*24:02 transgenic mouse

In accordance with the procedure as described in Experimental Example 1, the compound of formula (5) synthesized in Reference Example 1 and the compound of formula (13) synthesized in Example 24 were evaluated for ability to induce CTLs *in vivo* in an HLA-A*24:02 transgenic mouse.
The sequence RMFPNAPYL (SEQ ID NO: 2) included in the compound of formula (13):

CRMFPNAPYL
|
ECYTWNQMNL (13)

wherein C-C shown in the formula means that the C residues are linked together by a disulfide bond,
corresponds to an HLA-A*02:01-restricted WT1 peptide, and the sequence CYTWNQMNL (SEQ ID NO: 4) corresponds to an HLA-A*24:02-restricted WT1 peptide.
Specifically, in accordance with the procedure as described in Experimental Example 1, an emulsion of the formula (5) administered. On the other hand, the compound of formula (13) was dissolved in DMSO at a concentration of 140 mg/mL. The solution was diluted with water for injection to a concentration of 10.5 mg/mL, and then converted to an emulsion by addition of an equal volume of Montanide ISA 51 VG. The emulsion was injected to mice intradermally at two sites in the tail base area in an amount for administering 525 μg of the compound of formula (13) per site. The molar ratio of the compound of formula (5) and the compound of formula (13) administered per mouse was 1:1.
Figure 4 shows results from the IFNγ ELISPOT assay using the HLA-A*24:02 transgenic mouse. The results show that the compound of formula (5) or formula (13) induced CTLs responsive to the peptide of SEQ ID NO: 4 in the HLA-A*24:02 transgenic mouse, and that many CTLs responsive to the peptide of SEQ ID NO: 4 were found when the compound of formula (13) was administered as compared with the compound of formula (5).
The results demonstrate that the compound of formula (13) can induce CTLs specific to the peptide of SEQ ID NO: 4. The results also demonstrate that the compound of formula (13) induced more CTLs responsive to the peptide of SEQ ID NO: 4 as compared with the compound of formula (5).

Experimental Example 5

*In vivo* CTL induction in HLA-A*24:02 transgenic mouse

In accordance with the procedure as described in Experimental Example 1, the compound of formula (5) synthesized in Reference Example 1 and the compound of formula (12) synthesized in Example 25 were evaluated for ability to induce CTLs *in vivo* in an HLA-A*24:02 transgenic mouse.
The sequence RMFPNAPYL (SEQ ID NO: 2) included in the compound of formula (12):

CRMFPNAPYL
|
YCYTWNQMNL (12)

wherein C-C shown in the formula means that the C residues are linked together by a disulfide bond,
corresponds to an HLA-A*02: 01-restricted WT1 peptide, and the sequence CYTWNQMNL (SEQ ID NO: 4) corresponds to an HLA-A*24:02-restricted WT1 peptide.
Specifically, in accordance with the procedure as described in Experimental Example 1, an emulsion of the formula (5) was administered. On the other hand, the compound of formula (12) was dissolved in DMSO at a concentration of 142.7 mg/mL. The solution was diluted with water for injection to a concentration of 10.7 mg/mL, and then converted to an emulsion by addition of an equal volume of Montanide ISA 51 VG. The emulsion was injected to mice intradermally at two sites in the tail base area in an amount for administering 535 μg of the compound of formula (12) per site. The molar ratio of the compound of formula (5) and the compound of formula (12) administered per mouse was 1:1.
Figure 5 shows results from the IFNγ ELISPOT assay using the HLA-A*24:02 transgenic mouse. The results show that the compound of formula (5) or formula (12) induced CTLs responsive to the peptide of SEQ ID NO: 4 in the HLA-A*24:02 transgenic mouse, and that many CTLs responsive to the peptide of SEQ ID NO: 4 were found when the compound of formula (12) was administered as compared with the compound of formula (5).

The results demonstrate that the compound of formula (12) can induce CTLs specific to the peptide of SEQ ID NO: 4. The results also demonstrate that the compound of formula (12) induced more CTLs responsive to the peptide of SEQ ID NO: 4 as compared with the compound of formula (5).

Experimental Example 6

*In vivo* CTL induction in HLA-A*24:02 transgenic mouse

In accordance with the procedure as described in Experimental Example 1, the compound of formula (5) synthesized in Reference Example 1 and the compound of formula (14) synthesized in Example 26 were evaluated for ability to induce CTLs *in vivo* in an HLA-A*24:02 transgenic mouse.
The sequence RMFPNAPYL (SEQ ID NO: 2) included in the compound of formula (14):

```
 CRMFPNAPYL
 |               (14)
FCYTWNQMNL
```

wherein C-C shown in the formula means that the C residues are linked together by a disulfide bond,
corresponds to an HLA-A*02:01-restricted WT1 peptide, and the sequence CYTWNQMNL (SEQ ID NO: 4) corresponds to an HLA-A*24:02-restricted WT1 peptide.
Specifically, in accordance with the procedure as described in Experimental Example 1, an emulsion of the formula (5) was administered. On the other hand, the compound of formula (14) was dissolved in DMSO at a concentration of 141.3 mg/mL. The solution was diluted with water for injection to a concentration of 10.6 mg/mL, and then converted to an emulsion by addition of an equal volume of Montanide ISA 51 VG. The emulsion was injected to mice intradermally at two sites in the tail base area in an amount for administering 530 μg of the compound of formula (14) per site. The molar ratio of the compound of formula (5) and the compound of formula (14) administered per mouse was 1:1.
Figure 6 shows results from the IFNγ ELISPOT assay using the HLA-A*24:02 transgenic mouse. The results show that the compound of formula (5) or formula (14) induced CTLs responsive to the peptide of SEQ ID NO: 4 in the HLA-A*24:02 transgenic mouse, and that many CTLs responsive to the peptide of SEQ ID NO: 4 were found when the compound of formula (14) was administered as compared with the compound of formula (5).
The results demonstrate that the compound of formula (14) can induce CTLs specific to the peptide of SEQ ID NO: 4. The results also demonstrate that the compound of formula (14) induced more CTLs responsive to the peptide of SEQ ID NO: 4 as compared with the compound of formula (5).

Experimental Examples 7 to 24

Experimental Trimming of N-terminal amino acid by ERAP1

The peptides synthesized in Examples 1, 2, 3, 5, 6 and 8 to 20 were used as substrates, and the production rates of the peptide of Reference Example 5 (SEQ ID NO: 4) were evaluated. Specifically, a 50 μg/mL solution of ERAP1 (for example, it can be produced by the method in accordance with PLoS One November 2008, vol.3, Issue 11, e3658) in a pH8.0, 20 mM Tris·HCl-100 mM NaCl buffer (Tris·HCl buffer) was added to 210 μl of the same buffer. To the above-mentioned ERAP1 solution was added 30 μl of a 1mM peptide solution and well-mixed, and the mixture was then left to stand at room temperature. After 24 hours, 40 μL of the reaction liquid and 20 μL of an aqueous 5% DTT (dithiothreitol) solution were mixed, and 20 μL of an aqueous 1% TFA solution was further added. The solution was injected into an HPLC, and the production rate of the peptide of Reference Example 5 (SEQ ID NO: 4) was calculated from a substrate peptide based on the AUC (refer to Table 5). The production rate was calculated from the following expression.

Production rate =
AUC of SEQ ID NO: 4 ÷ (AUC of each substrate peptide + AUC of SEQ ID NO: 4) × 100

Conditions of HPLC are as shown below.
Analysis conditions 1
HPLC system: HPLC system manufactured by SHIMADZU CORPORATION
Column: Kinetex Minibore column, 4.6 mm φ × 150 mm, 5 μm

Eluent 1: 0.1% TFA in water
Eluent 2: 0.1% TFA in acetonitrile

Flow rate: 1.0 mL/min

Gradient method:

| Time (min.) | Concentration of Eluent 2 (%) |
|---|---|
| 0 | 25 |
| 15 | 40 |

Analysis conditions 2
HPLC system: HPLC system manufactured by SHIMADZU CORPORATION
Column: Kinetex Minibore column, 4.6 mm φ × 150 mm, 5 μm

Eluent 1: 0.1% TFA in water
Eluent 2: 0.1% TFA in acetonitrile

Flow rate: 1.0 mL/min

Gradient method:

| Time (min.) | Concentration of Eluent 2 (%) |
|---|---|
| 0 | 22 |
| 15 | 22 |

Analysis conditions 3
HPLC system: HPLC system manufactured by SHIMADZU CORPORATION
Column: Kinetex Minibore column, 4.6 mm φ × 150 mm, 5 μm

Eluent 1: 0.1% TFA in water
Eluent 2: 0.1% TFA in acetonitrile

Flow rate: 1.0 mL/min

Gradient method:

| Time (min.) | Concentration of Eluent 2 (%) |
|---|---|
| 0 | 25 |
| 40 | 40 |

Analysis conditions 4
HPLC system: HPLC system manufactured by SHIMADZU CORPORATION
Column: Kinetex Minibore column, 3 mm φ × 75 mm, 2.6 μm

Eluent 1: 0.1%TFA in water
Eluent 2: 0.1%TFA in acetonitrile

Flow rate: 0.2 mL/min

Gradient method:

| Time (min.) | Concentration of Eluent 2 (%) |
|---|---|
| 0 | 20 |
| 30 | 20 |

[Table 5]

| Experimental Example No. | Substrate Peptide Amino Acid Sequence | Substrate Peptide SEQ ID NO: | Production Rate of SEQ ID NO: 4 | Analysis Condition |
|---|---|---|---|---|
| 7 | ACYTWNQMNL | 55 | 10.0 | 2 |
| 8 | CCYTWNQMNL | 10 | 20.3 | 1 |
| 9 | ECYTWNQMNL | 32 | 32.5 | 4 |
| 10 | FCYTWNQMNL | 38 | 18.4 | 1 |
| 11 | HCYTWNQMNL | 34 | 24.4 | 1 |
| 12 | ICYTWNQMNL | 58 | 2.8 | 1 |
| 13 | KCYTWNQMNL | 36 | 45.0 | 1 |
| 14 | LCYTWNQMNL | 59 | 5.8 | 1 |
| 15 | MCYTWNQMNL | 60 | 32.1 | 1 |
| 16 | NCYTWNQMNL | 61 | 29.1 | 1 |
| 17 | PCYTWNQMNL | 62 | 11.1 | 1 |
| 18 | QCYTWNQMNL | 30 | 32.5 | 1 |
| 19 | RCYTWNQMNL | 28 | 49.2 | 1 |
| 20 | SCYTWNQMNL | 63 | 24.6 | 3 |
| 21 | TCYTWNQMNL | 64 | 15.6 | 4 |
| 22 | VCYTWNQMNL | 65 | 5.1 | 1 |
| 23 | WCYTWNQMNL | 66 | 5.2 | 1 |
| 24 | YCYTWNQMNL | 40 | 35.2 | 1 |

Experimental Example 25

*In vivo* CTL induction in HLA-A*24:02 transgenic mouse

In accordance with the procedure as described in Experimental Example 1, the peptide of SEQ ID NO: 34 synthesized in Example 1 was evaluated for ability to induce CTLs *in vivo* in an HLA-A*24:02 transgenic mouse.

Specifically, the peptide of SEQ ID NO: 34 was dissolved in DMSO at a concentration of 74.9 mg/mL. The solution was diluted with water for injection to a concentration of 5.6 mg/mL, and then converted to an emulsion by addition of an equal volume of Montanide ISA 51 VG. The emulsion was injected to mice intradermally at two sites in the tail base area in an amount for administering 280 μg of the peptide of SEQ ID NO: 34 per site.

Figure 7 shows results from the IFNγ ELISPOT assay using the HLA-A*24:02 transgenic mouse. The scale on the vertical axis of the graph of Figure 7 indicates the number of cells (CTLs) that produced IFNγ in response to the stimulation with the peptide of SEQ ID NO: 4, which were comprised in the cells seeded on the plate. The black bar of Figure 7 shows the number of splenocytes of the HLA-A*24:02 transgenic mouse that produced IFNγ in response to the stimulation with the peptide of SEQ ID NO: 4. The white bar shows the number of splenocytes of the HLA-A*24:02 transgenic mouse that produced IFNγ in the absence of the peptide stimulation. That is, the difference between the values of the black bar and the white bar shows the number of the peptide-specific CTLs. The results show that the peptide of SEQ ID NO: 34 induced CTLs responsive to the peptide of SEQ ID NO: 4 in the HLA-A*24:02 transgenic mouse.

The results demonstrate that the peptide of SEQ ID NO: 34 can induce CTLs specific to the peptide of SEQ ID NO: 4. Also, the results strongly suggest that the peptide of SEQ ID NO: 34 is suitably trimmed by ERAP-1 and then produce the peptide of SEQ ID NO: 4 in a living body of a mouse.

Experimental Examples 26 to 31

*In vivo* CTL induction in HLA-A*24:02 transgenic mouse

In accordance with the procedure as described in Experimental Example 1, the peptides of SEQ ID NOS: 32, 38, 36, 30, 28 and 40 synthesized in Examples 5, 6, 9, 14, 15 and 20 were evaluated for ability to induce CTLs *in vivo* in an HLA-A*24:02 transgenic mouse.

Specifically, each of the peptides of SEQ ID NOS: 32, 38, 36, 30, 28 and 40 was dissolved in DMSO at a concentration of 74.9 mg/mL. The solution was diluted with water for injection to a concentration of 5.6 mg/mL, and then converted to an emulsion by addition of an equal volume of Montanide ISA 51 VG. The emulsion was injected to mice intradermally at two sites in the tail base area in an amount for administering 280 μg of each of the peptides per site.

Figure 8 shows results from the IFNγ ELISPOT assay using the HLA-A*24:02 transgenic mouse. The scale on the vertical axis of the graph of Figure 8 indicates the number of cells (CTLs) that produced IFNγ in response to the stimulation with the peptide of SEQ ID NO: 4, which were comprised in the cells seeded on the plate. The peptides administered to the mice are described under the bars. The black bar of Figure 8 shows the number of splenocytes of the HLA-A*24:02 transgenic mouse that produced IFNγ in response to the stimulation with the peptide of SEQ ID NO: 4. The white bar shows the number of splenocytes of the HLA-A*24:02 transgenic mouse that produced IFNγ in the absence of the peptide stimulation. That is, the difference between the values of the black bar and the white bar shows the number of the peptide-specific CTLs. This shows that the peptides of SEQ ID NOS 32, 38, 36, 30, 28 and 40 induced CTLs responsive to the peptide of SEQ ID NO: 4 in the HLA-A*24:02 transgenic mouse.

The results demonstrate that the peptides of SEQ ID NOS: 32, 38, 36, 30, 28 and 40 can induce CTLs specific to the peptide of SEQ ID NO: 4. Also, the results strongly suggest that the peptide of SEQ ID NOS: 32, 38, 36, 30, 28 and 40 are suitably trimmed by ERAP-1 and then produce the peptide of SEQ ID NO: 4 in a living body of a mouse.

Reference Examples 6 to 7

The peptides shown in Table 6 were synthesized in accordance with the method as described in WO 2014/157692.

[Table 6]

| Reference Example No. | SEQ ID NO: | Amino Acid Sequence | LC-TOFMS (m/z, retention time (min.)) |
|---|---|---|---|
| 6 | 24 | WAPVLDFAPPGASAYGSL | 910.30[M+2H]$^{2+}$, 0.95 |
| 7 | 18 | PGCNKRYFKLSHLQMHSRKHTG | 1313.66[M+2H]$^{2+}$, 0.76 |

Experimental Example 32

*In vivo* CTL induction in HLA-A*24:02 transgenic mouse

In accordance with the procedure as described in Experimental Example 1, the compound of the formula (11) synthesized in Example 23 and a cocktail vaccine of the compound of the formula (11) and the peptide of SEQ ID NO:24 synthesized in Reference Example 6 were evaluated for ability to induce CTLs *in vivo* in an HLA-A*24:02 transgenic mouse.

The sequence RMFPNAPYL (SEQ ID NO: 2) included in the compound of formula (11):

CRMFPNAPYL
|
KCYTWNQMNL (11)

wherein C-C shown in the formula means that the C residues are linked together by a disulfide bond,
corresponds to an HLA-A*02:01-restricted WT1 peptide, and the sequence CYTWNQMNL (SEQ ID NO: 4) corresponds to an HLA-A*24:02-restricted WT1 peptide. The sequence WAPVLDFAPPGASAYGSL (SEQ ID NO: 24) corresponds to an MHC class II-restricted WT1 peptide (namely, a helper peptide).

Specifically, for the treatment with the compound of formula (11), the compound of formula (11') was dissolved in dimethyl sulfoxide (hereinafter described as DMSO) at a concentration of 66.67 mg/mL. The solution was diluted with water for injection to a concentration of 5.0 mg/mL, and then converted to an emulsion by addition of an equal volume of Montanide ISA 51 VG. The emulsion was injected to mice intradermally at two sites in the tail base area in an amount

for administering 250 μg of the compound of formula (11) per site. For the treatment with the cocktail vaccine, the compound of formula (11) and the peptide of SEQ ID NO: 24 were dissolved in DMSO at concentrations of 133.33 mg/mL and 96 mg/mL, respectively. The solutions were added to water for injection, mixed and diluted to concentrations of 5.0 mg/mL and 3.6 mg/mL, respectively. A mixture of the diluted aqueous solutions of the compound of formula (11) and the peptide of SEQ ID NO: 24 was converted to an emulsion by addition of an equal volume of Montanide ISA 51 VG. The emulsion was injected to mice intradermally at two sites in the tail base area in an amount for administering 250 μg of the compound of formula (11) per site and an amount for administering 180 μg of the peptide of SEQ ID NO: 24 per site, respectively. The amount of the compound of the formula (11) administered in the group treated with the compound of the formula (11) was the same as that in the group treated with the cocktail vaccine. In the cocktail vaccine treated group, the emulsion was prepared so that the molar amounts of the compound of formula (11) and the peptide of SEQ ID NO: 24 administered per mouse were substantially the same. Remaining procedures were performed as described in Experimental Example 1.

Figure 9 shows results from the IFNγ ELISPOT assay using the HLA-A*24:02 transgenic mouse. The results show that the compound of formula (11) induced CTLs responsive to the peptide of SEQ ID NO: 4 in the HLA-A*24:02 transgenic mouse, and that many CTLs responsive to the peptide of SEQ ID NO: 4 were found when the cocktail vaccine of the compound of formula (11) and the peptide of SEQ ID NO: 24 was administered.

The results demonstrate that the compound of formula (11) can induce CTLs specific to the peptide of SEQ ID NO: 4. The cocktail vaccine of the compound of formula (11) and the peptide of SEQ ID NO: 24 induced more IFNγ-producing cells specific to the peptide of SEQ ID NO: 4 as compared with the compound of formula (11). It would be because the induction of CTLs specific to the peptide of SEQ ID NO: 4 was enhanced by the induction of cells responsive to the helper peptide of SEQ ID NO: 24.

Experimental Example 33

*In vivo* CTL induction in HLA-A*24:02 transgenic mouse

In accordance with the procedure as described in Experimental Example 1, the compound of the formula (15) synthesized in Example 21 and a cocktail vaccine of the compound of the formula (15) and the peptide of SEQ ID NO:24 synthesized in Reference Example 6 were evaluated for ability to induce CTLs *in vivo* in an HLA-A*24:02 transgenic mouse.

The sequence RMFPNAPYL (SEQ ID NO: 2) included in the compound of formula (15):

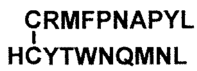 (15)

wherein C-C shown in the formula means that the C residues are linked together by a disulfide bond
corresponds to an HLA-A*02:01-restricted WT1 peptide, and the sequence CYTWNQMNL (SEQ ID NO: 4) corresponds to an HLA-A*24:02-restricted WT1 peptide. The sequence WAPVLDFAPPGASAYGSL (SEQ ID NO:24) corresponds to an MHC class II-restricted WT1 peptide (namely, a helper peptide).

Specifically, for the treatment with the compound of formula (15), the compound of formula (15) was dissolved in dimethyl sulfoxide (hereinafter described as DMSO) at a concentration of 66.67 mg/mL. The solution was diluted with water for injection to a concentration of 5.0 mg/mL, and then converted to an emulsion by addition of an equal volume of Montanide ISA 51 VG. The emulsion was injected to mice intradermally at two sites in the tail base area in an amount for administering 250 μg of the compound of formula (15) per site. For the treatment with the cocktail vaccine, the compound of formula (15) and the compound of SEQ ID NO: 24 were dissolved in DMSO at concentrations of 133.33 mg/mL and 96 mg/mL, respectively. The solutions were added to water for injection, mixed and diluted to concentrations of 5.0 mg/mL and 3.6 mg/mL, respectively. A mixture of the diluted aqueous solutions of the compound of formula (15) and the peptide of SEQ ID NO: 24 was converted to an emulsion by addition of an equal volume of Montanide ISA 51 VG. The emulsion was injected to mice intradermally at two sites in the tail base area in an amount for administering 250 μg of the compound of formula (15) per site and an amount for administering 180 μg of the compound of SEQ ID NO: 24 per site, respectively. The amount of the compound of the formula (15) administered in the group treated with the compound of the formula (15) was the same as that in the group treated with the cocktail vaccine. In the cocktail vaccine treated group, the emulsion was prepared so that the molar amounts of the compound of formula (15) and the peptide of SEQ ID NO: 24 administered per mouse were substantially the same. Remaining procedures were performed as described in Experimental Example 1.

Figure 10 shows results from the IFNγ ELISPOT assay using the HLA-A*24:02 transgenic mouse. The results show that the compound of formula (15) induced CTLs responsive to the peptide of SEQ ID NO: 4 in the HLA-A*24:02

transgenic mouse, and that many CTLs responsive to the peptide of SEQ ID NO: 4 were found when the cocktail vaccine of the compound of formula (15) and the peptide of SEQ ID NO: 24 was administered.

The results demonstrate that the compound of formula (15) can induce CTLs specific to the peptide of SEQ ID NO: 4. The cocktail vaccine of the compound of formula (15) and the peptide of SEQ ID NO: 24 induced more IFNy-producing cells specific to the peptide of SEQ ID NO: 4 as compared with the compound of formula (15). It would be because the induction of CTLs specific to the peptide of SEQ ID NO: 4 was enhanced by the induction of cells responsive to the helper peptide of SEQ ID NO: 24.

Experimental Example 34

*In vivo* CTL induction in HLA-A*24:02 transgenic mouse

In accordance with the procedure as described in Experimental Example 1, the compound of the formula (11) synthesized in Example 23 and a cocktail vaccine of the compound of the formula (11) and the peptide of SEQ ID NO: 18 synthesized in Reference Example 7 were evaluated for ability to induce CTLs *in vivo* in an HLA-A*24:02 transgenic mouse.
The sequence RMFPNAPYL (SEQ ID NO: 2) included in the compound of formula (11):

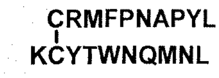

(11)

wherein C-C shown in the formula means that the C residues are linked together by a disulfide bond,
corresponds to an HLA-A*02:01-restricted WT1 peptide, and the sequence CYTWNQMNL (SEQ ID NO: 4) corresponds to an HLA-A*24:02-restricted WT1 peptide. The sequence PGCNKRYFKLSHLQMHSRKHTG (SEQ ID NO: 18) corresponds to an MHC class II-restricted WT1 peptide (namely, helper peptide).

Specifically, for the treatment with the compound of formula (11), the compound of formula (11) was dissolved in dimethyl sulfoxide (hereinafter described as DMSO) at a concentration of 66.67 mg/mL. The solution was diluted with water for injection to a concentration of 5.0 mg/mL, and then converted to an emulsion by addition of an equal volume of Montanide ISA 51 VG. The emulsion was injected to mice intradermally at two sites in the tail base area in an amount for administering 250 μg of the compound of formula (11) per site. For the treatment with the cocktail vaccine, the compound of formula (11) and the peptide of SEQ ID NO: 18 were dissolved in DMSO at concentrations of 133.33 mg/mL and 137.78 mg/mL, respectively. The solutions were added to water for injection, mixed and diluted to concentrations of 5.0 mg/mL and 5.2 mg/mL, respectively. A mixture of the diluted aqueous solutions of the compound of formula (11) and the peptide of SEQ ID NO: 18 was converted to an emulsion by addition of an equal volume of Montanide ISA 51 VG. The emulsion was injected to mice intradermally at two sites in the tail base area in an amount for administering 250 μg of the compound of formula (11) per site and an amount for administering 260 μg of the peptide of SEQ ID NO: 18 per site, respectively. The amount of the compound of the formula (11) administered in the group treated with the compound of the formula (11) was the same as that in the group treated with the cocktail vaccine. In the cocktail vaccine treated group, the emulsion was prepared so that the molar amounts of the compound of formula (11) and the peptide of SEQ ID NO: 18 administered per mouse were substantially the same. Remaining procedures were performed as described in Experimental Example 1.

Figure 11 shows results from the IFNγ ELISPOT assay using the HLA-A*24:02 transgenic mouse. The results show that the compound of formula (11) induced CTLs responsive to the peptide of SEQ ID NO: 4 in the HLA-A*24:02 transgenic mouse, and that many CTLs responsive to the peptide of SEQ ID NO: 4 were found when the cocktail vaccine of the compound of formula (11) and the peptide of SEQ ID NO: 18 was administered.

The results demonstrate that the compound of formula (11) can induce CTLs specific to the peptide of SEQ ID NO: 4. The cocktail vaccine of the compound of formula (11) and the peptide of SEQ ID NO: 18 induced more IFNγ-producing cells specific to the peptide of SEQ ID NO: 4 as compared with the compound of formula (11). It would be because the induction of CTLs specific to the peptide of SEQ ID NO: 4 was enhanced by the induction of cells responsive to the helper peptide of SEQ ID NO: 18.

Industrial Applicability

The present disclosure provides a cancer vaccine or a composition for cancer immunotherapy that induces CTLs efficiently. Therefore, the present disclosure can find applications in the medical field, for example in development or production of compositions for treatment or prevention of a cancer.

Sequence Listing Free Text

SEQ ID NO: 2 Peptide (RMFPNAPYL)
SEQ ID NO: 3 Peptide (CMTWNQMNL)
SEQ ID NO: 4 Peptide (CYTWNQMNL)
SEQ ID NO: 5 Peptide (ALLPAVPSL)
SEQ ID NO: 6 Peptide (SLGEQQYSV)
SEQ ID NO: 7 Peptide (RVPGVAPTL)
SEQ ID NO: 8 Peptide (CRMFPNAPYL)
SEQ ID NO: 9 Peptide (CCMTWNQMNL)
SEQ ID NO: 10 Peptide (CCYTWNQMNL)
SEQ ID NO: 11 Peptide (CALLPAVPSL)
SEQ ID NO: 12 Peptide (CSLGEQQYSV)
SEQ ID NO: 13 Peptide (CRVPGVAPTL)
SEQ ID NO: 14 Peptide (SGQARMFPNAPYLPSC)
SEQ ID NO: 15 Peptide (SGQARMFPNAPYLPSCLES)
SEQ ID NO: 16 Peptide (PGCNKRYFKLSHLQMHSRK)
SEQ ID NO: 17 Peptide (PGCNKRYFKLSHLQMHSRKH)
SEQ ID NO: 18 Peptide (PGCNKRYFKLSHLQMHSRKHTG)
SEQ ID NO: 19 Peptide (CNKRYFKLSHLQMHSRK)
SEQ ID NO: 20 Peptide (CNKRYFKLSHLQMHSRKH)
SEQ ID NO: 21 Peptide (CNKRYFKLSHLQMHSRKHTG)
SEQ ID NO: 22 Peptide (SGQAYMFPNAPYLPSC)
SEQ ID NO: 23 Peptide (SGQAYMFPNAPYLPSCLES)
SEQ ID NO: 24 Peptide (WAPVLDFAPPGASAYGSL)
SEQ ID NO: 25 Peptide (CWAPVLDFAPPGASAYGSL)
SEQ ID NO: 26 Peptide (WAPVLDFAPPGASAYGSLC)
SEQ ID NO: 27 Peptide (RCMTWNQMNL)
SEQ ID NO: 28 Peptide (RCYTWNQMNL)
SEQ ID NO: 29 Peptide (QCMTWNQMNL)
SEQ ID NO: 30 Peptide (QCYTWNQMNL)
SEQ ID NO: 31 Peptide (ECMTWNQMNL)
SEQ ID NO: 32 Peptide (ECYTWNQMNL)
SEQ ID NO: 33 Peptide (HCMTWNQMNL)
SEQ ID NO: 34 Peptide (HCYTWNQMNL)
SEQ ID NO: 35 Peptide (KCMTWNQMNL)
SEQ ID NO: 36 Peptide (KCYTWNQMNL)
SEQ ID NO: 37 Peptide (FCMTWNQMNL)
SEQ ID NO: 38 Peptide (FCYTWNQMNL)
SEQ ID NO: 39 Peptide (YCMTWNQMNL)
SEQ ID NO: 40 Peptide (YCYTWNQMNL)
SEQ ID NO: 41 Peptide (VLDFAPPGA)
SEQ ID NO: 42 Peptide (RYFPNAPYL)
SEQ ID NO: 43 Peptide (FMFPNAPYL)
SEQ ID NO: 44 Peptide (RLFPNAPYL)
SEQ ID NO: 45 Peptide (RMMPNAPYL)
SEQ ID NO: 46 Peptide (RMFPNAPYV)
SEQ ID NO: 47 Peptide (YMFPNAPYL)
SEQ ID NO: 48, Peptide (Xaa-Met-Thr-Trp-Asn-Gln-Met-Asn-Leu), wherein Xaa is Ser or Ala.
SEQ ID NO: 49, Peptide (Xaa-Tyr-Thr-Trp-Asn-Gln-Met-Asn-Leu), wherein Xaa is Ser, Ala, Abu, Arg, Lys, Orn, Cit, Leu, Phe or Asn.
SEQ ID NO: 50 Peptide (AYLPAVPSL)
SEQ ID NO: 51 Peptide (FLGEQQYSV)
SEQ ID NO: 52 Peptide (SMGEQQYSV)
SEQ ID NO: 53 Peptide (SLMEQQYSV)
SEQ ID NO: 54 Peptide (RYPGVAPTL)

SEQ ID NO: 55 Peptide (ACYTWNQMNL)
SEQ ID NO: 56 Peptide (DCYTWNQMNL)
SEQ ID NO: 57 Peptide (GCYTWNQMNL)
SEQ ID NO: 58 Peptide (ICYTWNQMNL)
SEQ ID NO: 59 Peptide (LCYTWNQMNL)
SEQ ID NO: 60 Peptide (MCYTWNQMNL)
SEQ ID NO: 61 Peptide (NCYTWNQMNL)
SEQ ID NO: 62 Peptide (PCYTWNQMNL)
SEQ ID NO: 63 Peptide (SCYTWNQMNL)
SEQ ID NO: 64 Peptide (TCYTWNQMNL)
SEQ ID NO: 65 Peptide (VCYTWNQMNL)
SEQ ID NO: 66 Peptide (WCYTWNQMNL)
SEQ ID NO: 67 Peptide (C(Npys)RMFPNAPYL)
SEQ ID NO: 68 Peptide (KRYFKLSHLQMHSRKH)

## Claims

1. A compound consisting of 10 to 12 amino acids or a pharmaceutically acceptable salt thereof, wherein the compound comprises the amino acid sequence:

   CMTWNQMNL (SEQ ID NO: 3), or
   CYTWNQMNL (SEQ ID NO: 4)

   and 1 to 3 amino acid residues attached to the amino group of the N-terminal cysteine residue of the amino acid sequence.

2. The compound or a pharmaceutically acceptable salt thereof according to claim 1, wherein the compound consists of 10 to 12 amino acids and comprises the amino acid sequence of SEQ ID NO: 3.

3. The compound or a pharmaceutically acceptable salt thereof according to claim 1, wherein the compound consists of 10 to 12 amino acids and comprises the amino acid sequence of SEQ ID NO: 4.

4. The compound or a pharmaceutically acceptable salt thereof according to any one of claims 1-3, wherein the compound consists of 10 amino acids.

5. The compound or a pharmaceutically acceptable salt thereof according to any one of claims 1-4, wherein the 1 to 3 amino acid residues attached to the amino group of the N-terminal cysteine residue are independently selected from an arginine residue, a glutamine residue, a glutamic acid residue, a histidine residue, a lysine residue, a phenylalanine residue and a tyrosine residue.

6. The compound or a pharmaceutically acceptable salt thereof according to any one of claims 1-5, wherein the compound is a peptide consisting of an amino acid sequence selected from the amino acid sequences:

   RCMTWNQMNL (SEQ ID NO: 27),
   QCMTWNQMNL (SEQ ID NO: 29),
   ECMTWNQMNL (SEQ ID NO: 31),
   HCMTWNQMNL (SEQ ID NO: 33),
   KCMTWNQMNL (SEQ ID NO: 35),
   FCMTWNQMNL (SEQ ID NO: 37), and
   YCMTWNQMNL (SEQ ID NO: 39).

7. The compound or a pharmaceutically acceptable salt thereof according to any one of claims 1-5, wherein the compound is a peptide consisting of an amino acid sequence selected from the amino acid sequences:

   RCYTWNQMNL (SEQ ID NO: 28),
   QCYTWNQMNL (SEQ ID NO: 30),
   ECYTWNQMNL (SEQ ID NO: 32),
   HCYTWNQMNL (SEQ ID NO: 34),
   KCYTWNQMNL (SEQ ID NO: 36),
   FCYTWNQMNL (SEQ ID NO: 38), and
   YCYTWNQMNL (SEQ ID NO: 40).

8. A compound of formula (1):

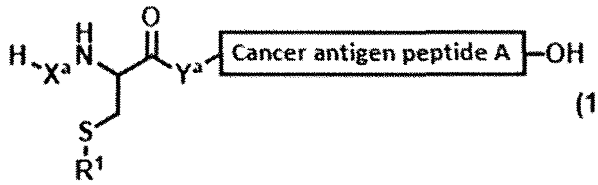

(1)

wherein $X^a$ and $Y^a$ independently represent a single bond or a divalent peptide group consisting of 1 to 4 amino acid residues, provided that the sum of the number of amino acid residues in $X^a$ and $Y^a$ is an integer of 0 to 4; cancer antigen peptide A is a peptide comprising an amino acid sequence selected from the amino acid sequences:

RMFPNAPYL (SEQ ID NO: 2),
ALLPAVPSL (SEQ ID NO: 5),
SLGEQQYSV (SEQ ID NO: 6), and
RVPGVAPTL (SEQ ID NO: 7)

or a peptide comprising an amino acid sequence that differs from the amino acid sequence selected from SEQ ID NOS: 2, 5, 6 and 7 by alteration of one to three amino acid residues and having an ability to induce CTLs, wherein an amino group of an N-terminal amino acid of the cancer antigen peptide A binds to $Y^a$ in the formula (1), and a carbonyl group of a C-terminal amino acid of the cancer antigen peptide A binds to the hydroxyl group in the formula (1); and
$R^1$ is cancer antigen peptide C,
wherein the cancer antigen peptide C represents the compound according to any one of claims 1-7 and the cysteine residue in the compound binds to the formula (1) via a disulfide bond;
or a pharmaceutically acceptable salt thereof.

**9.** The compound or a pharmaceutically acceptable salt thereof according to claim 8, wherein the cancer antigen peptide A is a peptide consisting of an amino acid sequence selected from the amino acid sequences:

RMFPNAPYL (SEQ ID NO: 2),
ALLPAVPSL (SEQ ID NO: 5),
SLGEQQYSV (SEQ ID NO: 6), and
RVPGVAPTL (SEQ ID NO: 7).

**10.** The compound or a pharmaceutically acceptable salt thereof according to claim 8 or 9, wherein the compound of formula (1) is a compound of formula (10):

CRMFPNAPYL
RCYTWNQMNL (10)

wherein C-C shown in the formula means that the C residues are linked together by a disulfide bond.

**11.** The compound or a pharmaceutically acceptable salt thereof according to claim 8 or 9, wherein the compound of formula (1) is a compound of formula (11):

CRMFPNAPYL
KCYTWNQMNL (11)

wherein C-C shown in the formula means that the C residues are linked together by a disulfide bond.

**12.** The compound or a pharmaceutically acceptable salt thereof according to claim 8 or 9, wherein the compound of formula (1) is a compound of formula (12):

CRMFPNAPYL
YCYTWNQMNL (12)

wherein C-C shown in the formula means that the C residues are linked together by a disulfide bond.

**13.** The compound or a pharmaceutically acceptable salt thereof according to claim 8 or 9, wherein the compound of formula (1) is a compound of formula (13):

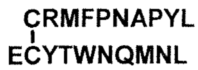 (13)

wherein C-C shown in the formula means that the C residues are linked together by a disulfide bond.

**14.** The compound or a pharmaceutically acceptable salt thereof according to claim 8 or 9, wherein the compound of formula (1) is a compound of formula (14):

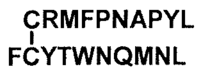 (14)

wherein C-C shown in the formula means that the C residues are linked together by a disulfide bond.

**15.** The compound or a pharmaceutically acceptable salt thereof according to claim 8 or 9, wherein the compound of formula (1) is a compound of formula (15):

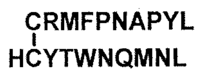 (15)

wherein C-C shown in the formula means that the C residues are linked together by a disulfide bond.

**16.** A composition comprising the compound or a pharmaceutically acceptable salt thereof according to any one of claims 1-15, wherein the compound or a pharmaceutically acceptable salt thereof is used in combination with cancer antigen peptide D or a pharmaceutically acceptable salt thereof, and the cancer antigen peptide D is a peptide consisting of an amino acid sequence selected from the group consisting of the amino acid sequences:

SGQARMFPNAPYLPSC (SEQ ID NO: 14),
SGQARMFPNAPYLPSCLES (SEQ ID NO: 15),
PGCNKRYFKLSHLQMHSRK (SEQ ID NO: 16),
PGCNKRYFKLSHLQMHSRKH (SEQ ID NO: 17),
PGCNKRYFKLSHLQMHSRKHTG (SEQ ID NO: 18),
CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21),
SGQAYMFPNAPYLPSC (SEQ ID NO: 22),
SGQAYMFPNAPYLPSCLES (SEQ ID NO: 23)
WAPVLDFAPPGASAYGSL (SEQ ID NO: 24),
CWAPVLDFAPPGASAYGSL (SEQ ID NO: 25),
WAPVLDFAPPGASAYGSLC (SEQ ID NO: 26), and
KRYFKLSHLQMHSRKH (SEQ ID NO: 68).

**17.** The composition according to claim 16, wherein the cancer antigen peptide D is a peptide consisting of an amino acid sequence selected from the group consisting of the amino acid sequences:

CNKRYFKLSHLQMHSRK (SEQ ID NO: 19),
CNKRYFKLSHLQMHSRKH (SEQ ID NO: 20),
CNKRYFKLSHLQMHSRKHTG (SEQ ID NO: 21),
WAPVLDFAPPGASAYGSL (SEQ ID NO: 24),
CWAPVLDFAPPGASAYGSL (SEQ ID NO: 25), and
WAPVLDFAPPGASAYGSLC (SEQ ID NO: 26).

**18.** The composition according to claim 16, wherein the cancer antigen peptide D is a peptide consisting of an amino acid sequence selected from the group consisting of

WAPVLDFAPPGASAYGSL (SEQ ID NO: 24),
CWAPVLDFAPPGASAYGSL (SEQ ID NO: 25), and
WAPVLDFAPPGASAYGSLC (SEQ ID NO: 26).

**19.** The composition according to claim 16, wherein the cancer antigen peptide D is a peptide consisting of an amino acid sequence selected from the group consisting of

PGCNKRYFKLSHLQMHSRK (SEQ ID NO: 16),
PGCNKRYFKLSHLQMHSRKH (SEQ ID NO: 17), and
PGCNKRYFKLSHLQMHSRKHTG (SEQ ID NO: 18).

**20.** A pharmaceutical composition comprising the compound or a pharmaceutically acceptable salt thereof according to any one of claims 1-15, or the composition according to any one of claims 16-19, and a pharmaceutically acceptable carrier.

**21.** The pharmaceutical composition according to claim 20, wherein the pharmaceutical composition is for use as a composition for treating a cancer associated with WT1 gene expression or an elevated level of WT1 gene expression.

**22.** The pharmaceutical composition according to claim 20, wherein the pharmaceutical composition is for use as a composition for inducing CTLs in cellular immunotherapy for a cancer.

**23.** The pharmaceutical composition according to claim 20, wherein the pharmaceutical composition is for use as a cancer vaccine.

**24.** The compound or a pharmaceutically acceptable salt thereof according to any one of claims 1-15, or the composition according to any one of claims 16-19, wherein the compound or a pharmaceutically acceptable salt thereof or the composition is for use in treatment or prevention of a cancer.

**25.** A kit for preventing or treating a cancer, wherein the kit comprises the compound or a pharmaceutically acceptable salt thereof according to any one of claims 1-15, or the composition according to any one of claims 16-19, and a pharmaceutically acceptable carrier.

**26.** Use of the compound or a pharmaceutically acceptable salt thereof according to any one of claims 1-15, or the composition according to any one of claims 16-19, for the manufacture of a cancer vaccine.

**27.** A method of treating or preventing a cancer, comprising administering to a patient in need thereof a therapeutically or prophylactically effective amount of the compound or a pharmaceutically acceptable salt thereof according to any one of claims 1-15, or the composition according to any one of claims 16-19.

**28.** The method according to claim 27, wherein the patient is WT1-positive.

**29.** The compound or a pharmaceutically acceptable salt thereof, the composition, the pharmaceutical composition, the kit, the use or the method according to any one of claims 21-28, wherein the cancer is selected from the group consisting of leukemia, myelodysplastic syndrome, multiple myeloma, malignant lymphoma, gastric cancer, colorectal cancer, lung cancer, breast cancer, germ cell cancer, liver cancer, skin cancer, urinary bladder cancer, prostate cancer, uterine cancer, cervical cancer, ovarian cancer, brain tumor, bone cancer, pancreatic cancer, cancer of the head or neck, cutaneous or intraocular malignant melanoma, rectal cancer, cancer of the anal region, testicular cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia such as acute myeloid leukemia, chronic myeloid leukemia, acute lymphoblastic leukemia, or chronic lymphocytic leukemia, childhood solid tumor, lymphocytic lymphoma, cancer of the kidney or ureter, carcinoma of the renal pelvis, central nervous system (CNS) tumor, primary CNS lymphoma, tumor angiogenesis, spinal tumor, brainstem glioma, pituitary adenoma, Kaposi's sarcoma, epidermoid cancer, squamous cell cancer, T-cell lymphoma,

glioblastoma multiforme, malignant melanoma, non-small cell lung cancer, renal cell carcinoma, and asbestos-induced cancer.

Fig. 1

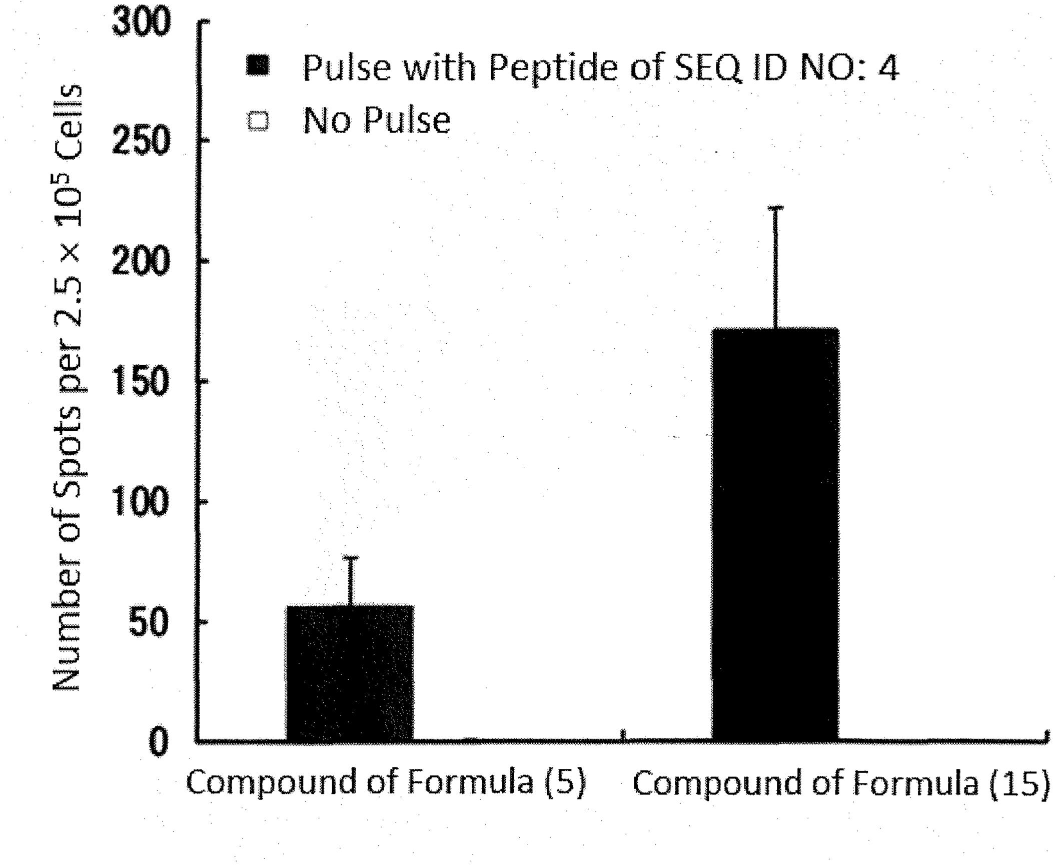
300
250
200
150
100
50
0
Number of Spots per 2.5 × 10⁵ Cells
Pulse with Peptide of SEQ ID NO: 4
No Pulse
Compound of Formula (5)
Compound of Formula (15)

Fig. 2

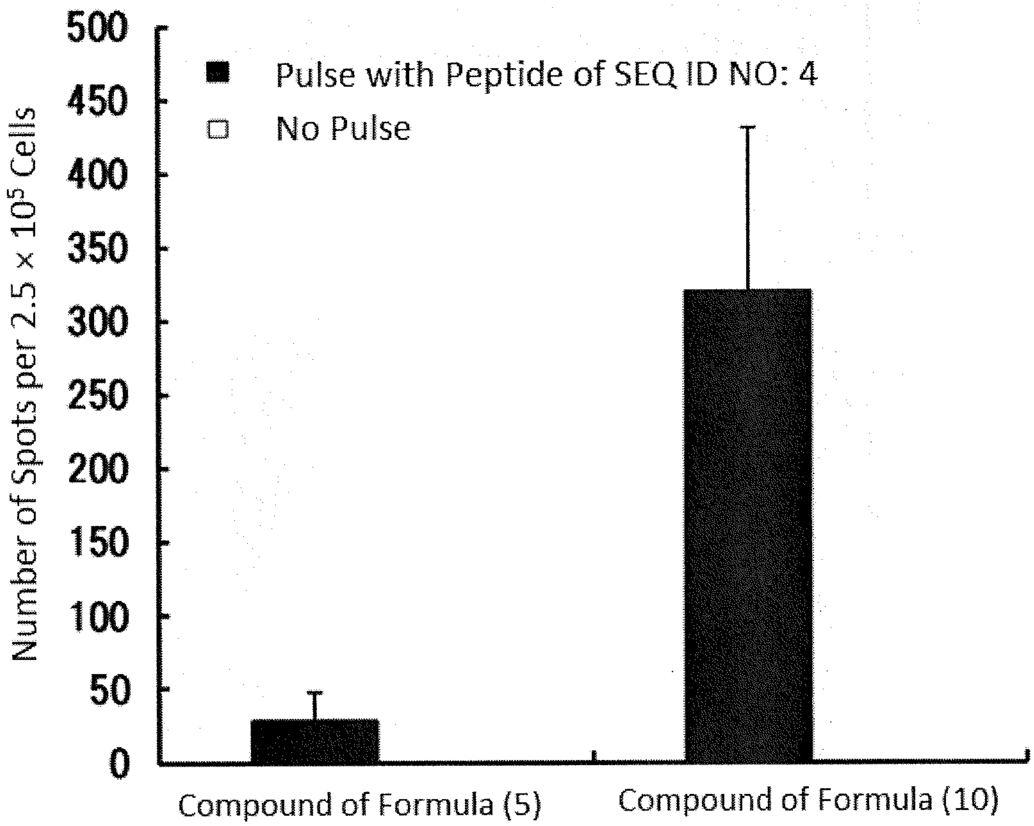
500
450
400
350
300
250
200
150
100
50
0
Number of Spots per 2.5 × 10^5 Cells
Pulse with Peptide of SEQ ID NO: 4
No Pulse
Compound of Formula (5)
Compound of Formula (10)

Fig. 3

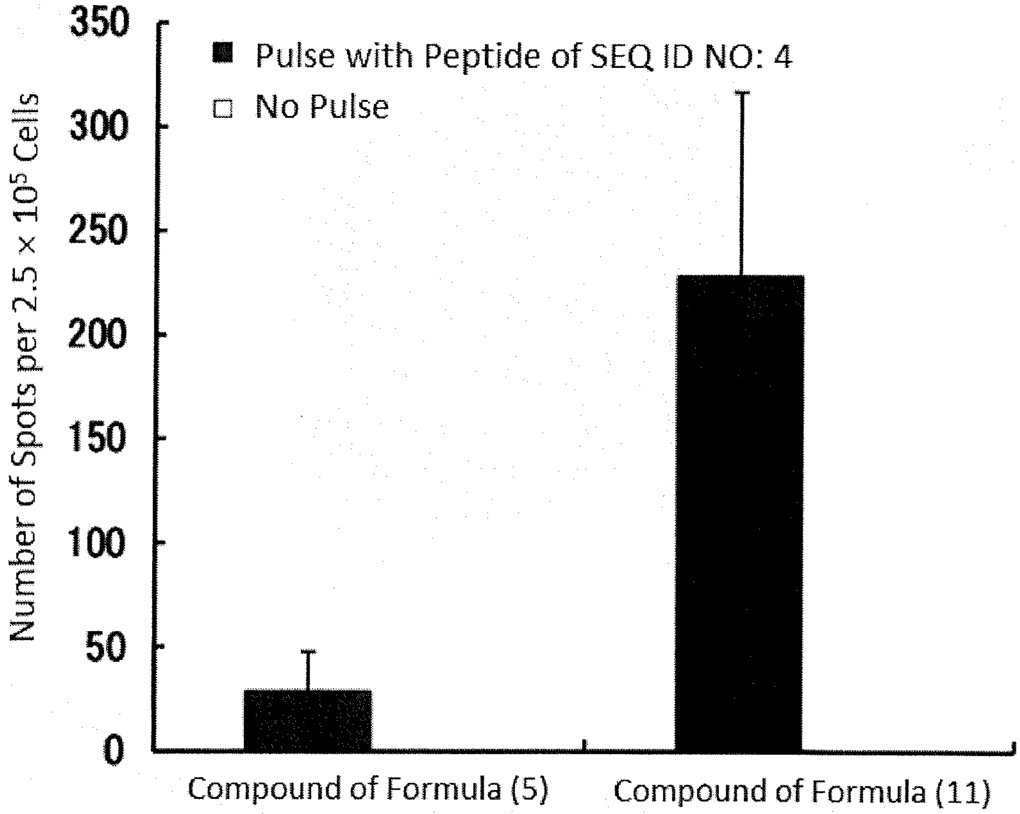
350
300
250
200
150
100
50
0
Number of Spots per 2.5 × 10[5] Cells
Pulse with Peptide of SEQ ID NO: 4
No Pulse
Compound of Formula (5)
Compound of Formula (11)

Fig. 4

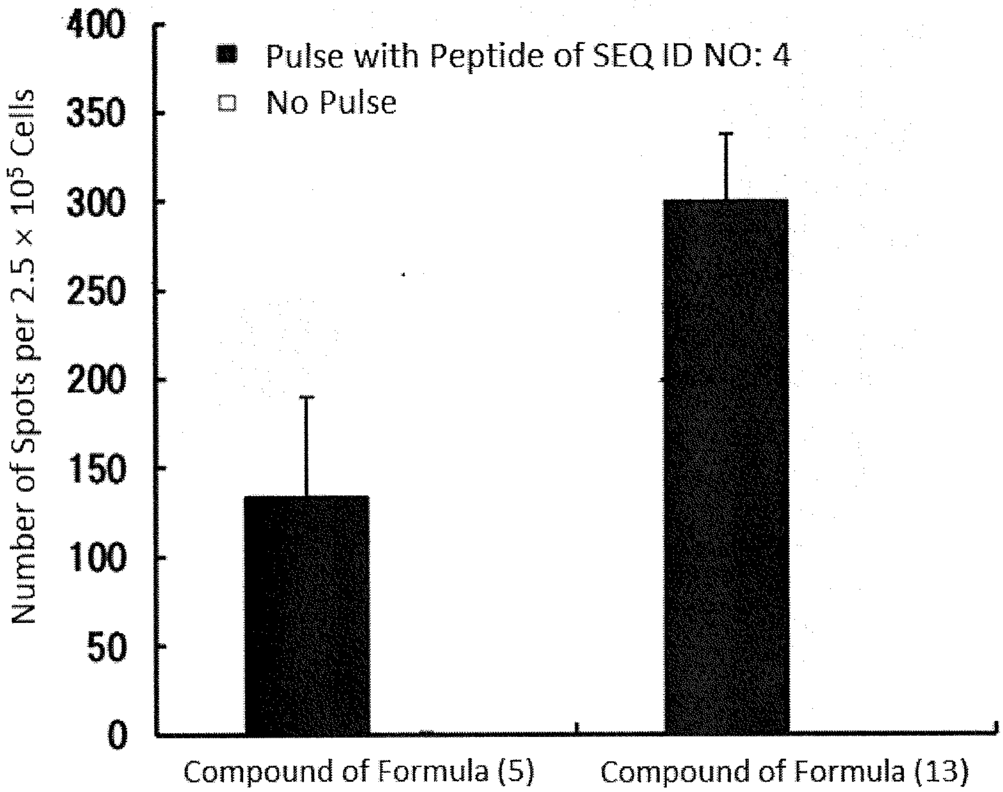
400
350
300
250
200
150
100
50
0
Number of Spots per 2.5 × 10^5 Cells
Pulse with Peptide of SEQ ID NO: 4
No Pulse
Compound of Formula (5)
Compound of Formula (13)

Fig. 5

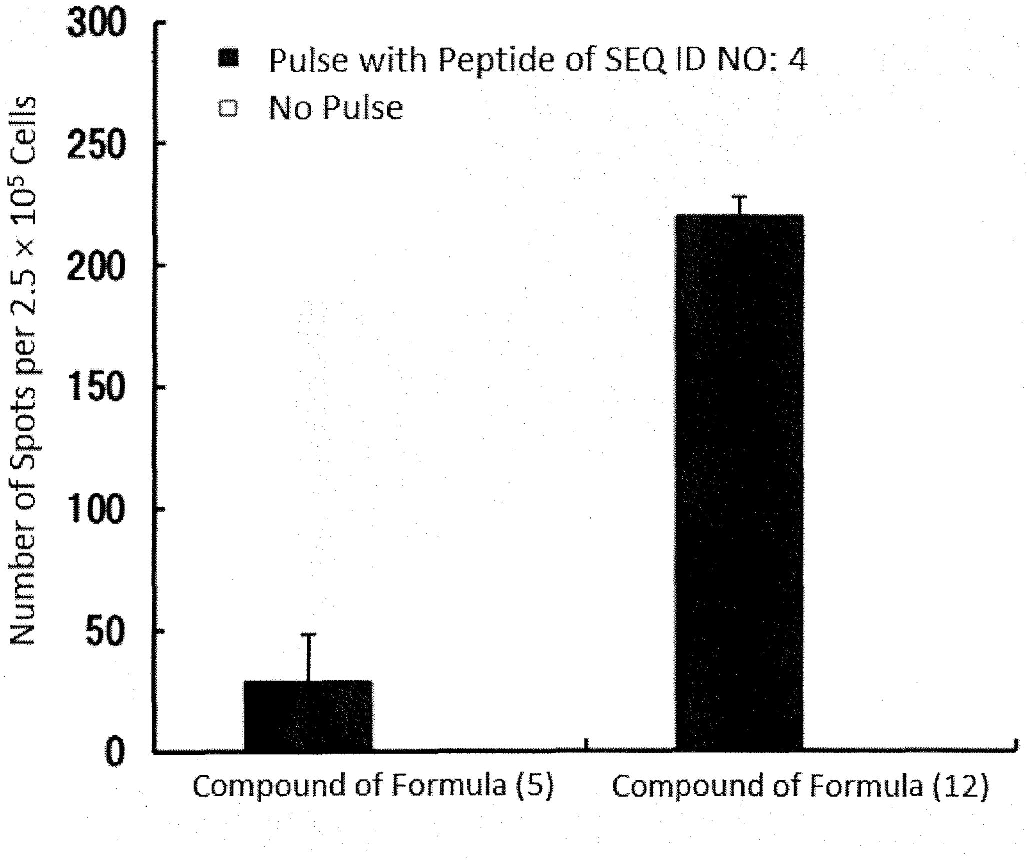
300
250
200
150
100
50
0
Number of Spots per 2.5 × 10^5 Cells
Pulse with Peptide of SEQ ID NO: 4
No Pulse
Compound of Formula (5)
Compound of Formula (12)

Fig. 6

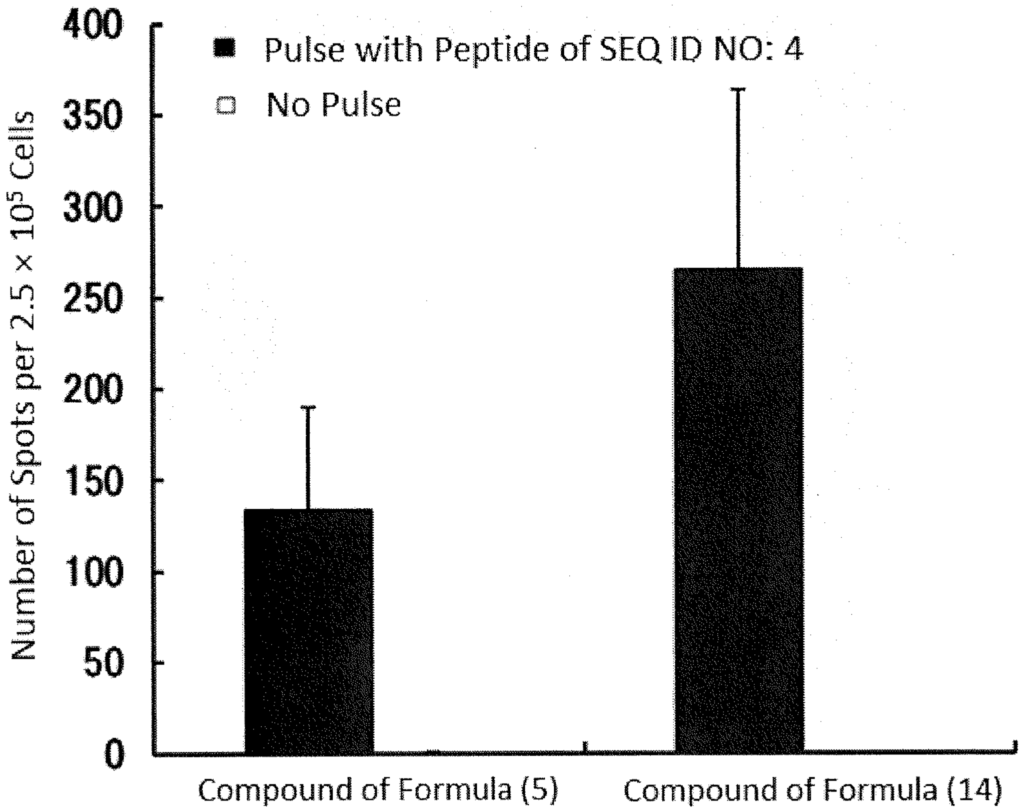
Pulse with Peptide of SEQ ID NO: 4
No Pulse
Number of Spots per 2.5 × 10^5 Cells
400
350
300
250
200
150
100
50
0
Compound of Formula (5)
Compound of Formula (14)

Fig. 7

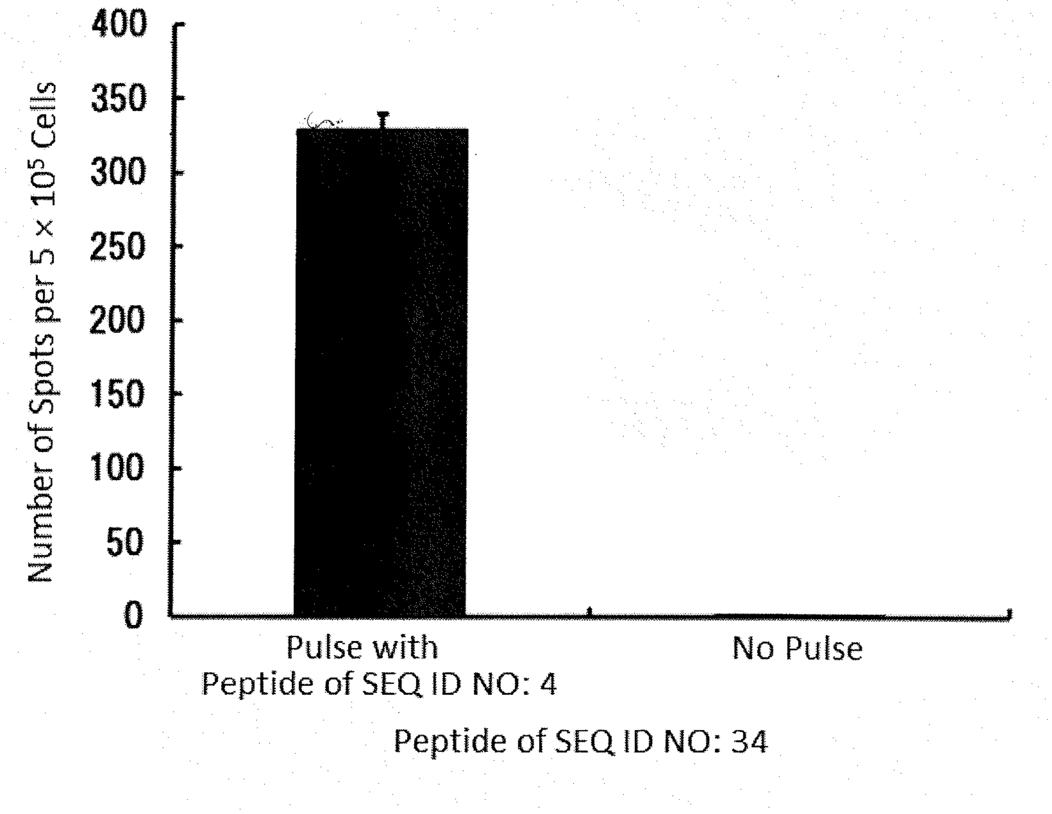
400
350
300
250
200
150
100
50
0
Number of Spots per 5 × 10⁵ Cells
Pulse with
Peptide of SEQ ID NO: 4
No Pulse
Peptide of SEQ ID NO: 34

Fig. 8

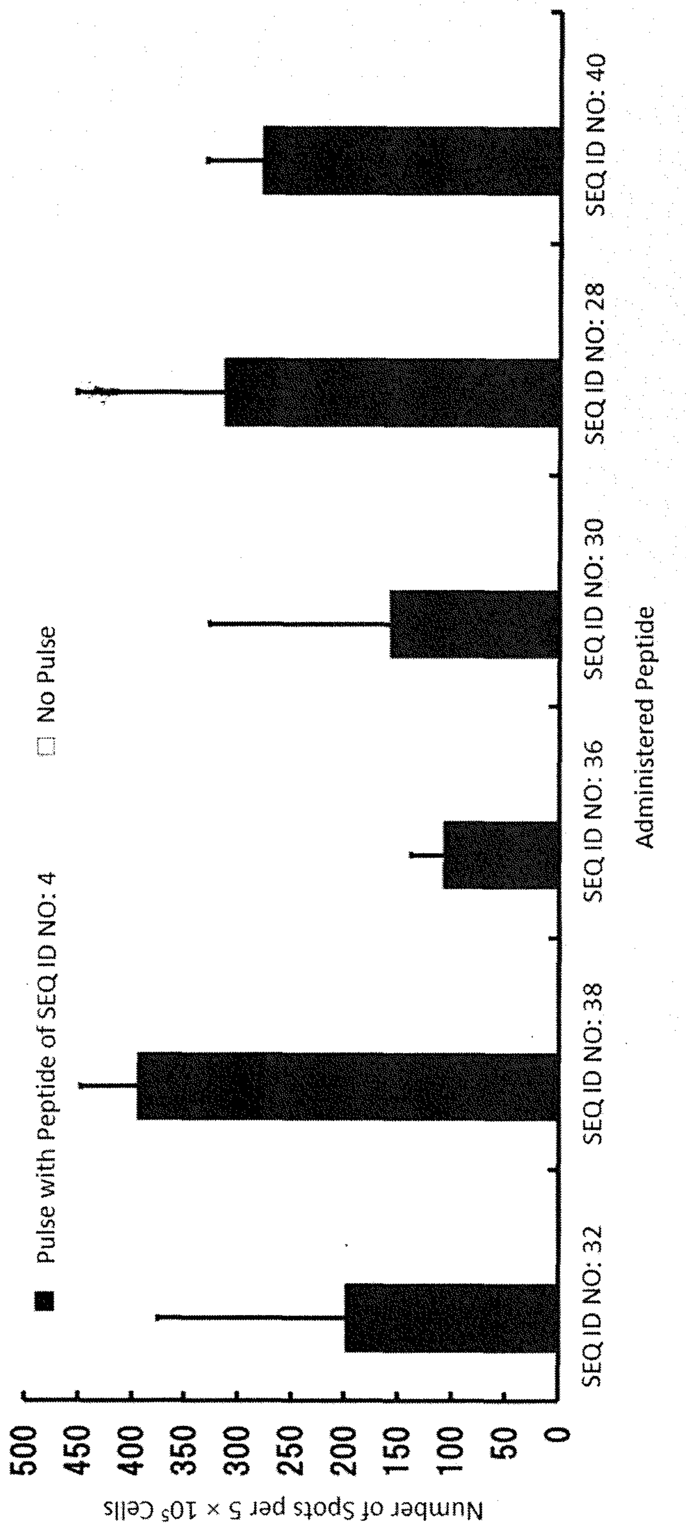
Pulse with Peptide of SEQ ID NO: 4
No Pulse
Number of Spots per 5 × 10^5 Cells
500
450
400
350
300
250
200
150
100
50
0
SEQ ID NO: 32
SEQ ID NO: 38
SEQ ID NO: 36
SEQ ID NO: 30
SEQ ID NO: 28
SEQ ID NO: 40
Administered Peptide

Fig. 9

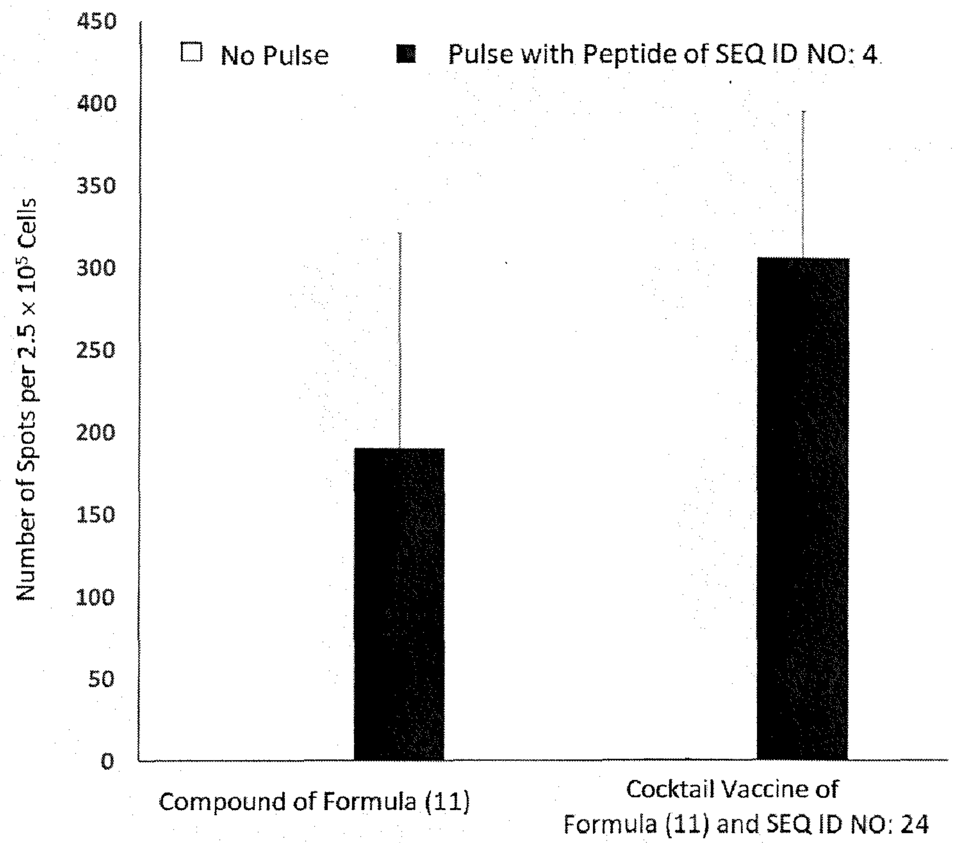

450
400
350
300
250
200
150
100
50
0
Number of Spots per 2.5 × 10^5 Cells
No Pulse
Pulse with Peptide of SEQ ID NO: 4
Compound of Formula (11)
Cocktail Vaccine of Formula (11) and SEQ ID NO: 24

Fig. 10

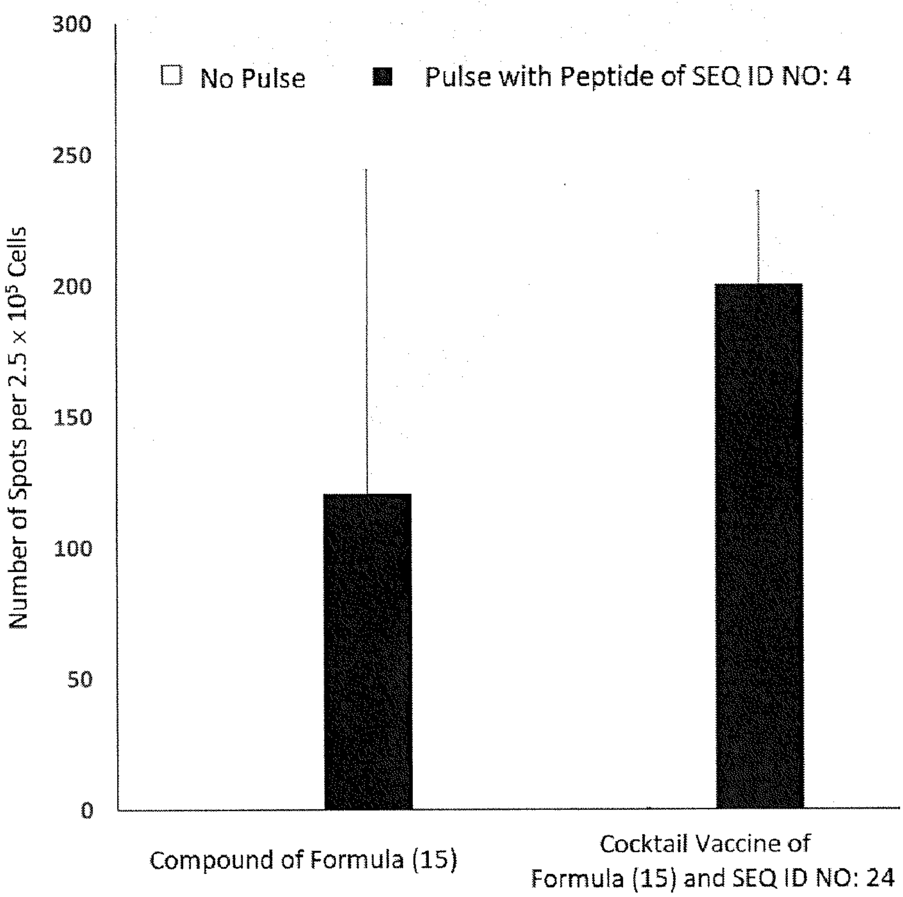
300
250
200
150
100
50
0
No Pulse
Pulse with Peptide of SEQ ID NO: 4
Number of Spots per 2.5 × 10^5 Cells
Compound of Formula (15)
Cocktail Vaccine of Formula (15) and SEQ ID NO: 24

Fig. 11

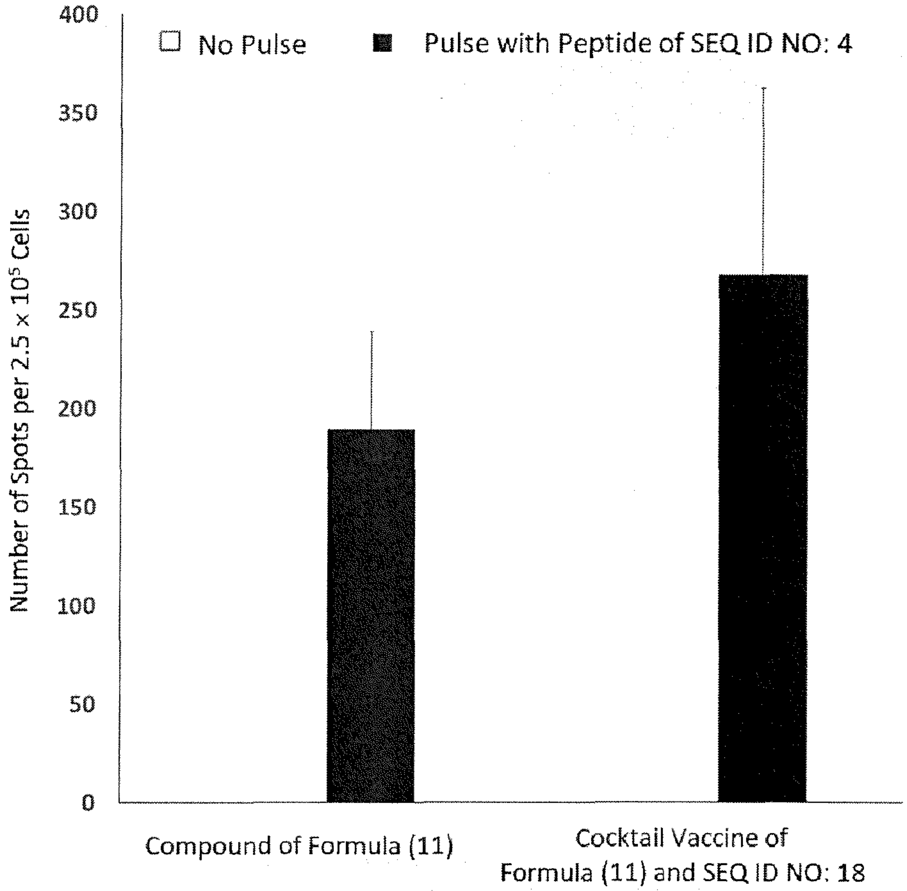
No Pulse
Pulse with Peptide of SEQ ID NO: 4
Number of Spots per 2.5 x 10⁵ Cells
400
350
300
250
200
150
100
50
0
Compound of Formula (11)
Cocktail Vaccine of
Formula (11) and SEQ ID NO: 18

## INTERNATIONAL SEARCH REPORT

International application No. PCT/JP2018/013092

A. CLASSIFICATION OF SUBJECT MATTER
Int.Cl. C07K7/06(2006.01)i, A61K39/00(2006.01)i, A61P35/00(2006.01)i, A61P35/02(2006.01)i, A61P37/04(2006.01)i, C07K7/08(2006.01)i, C07K19/00(2006.01)i
According to International Patent Classification (IPC) or to both national classification and IPC

B. FIELDS SEARCHED
Minimum documentation searched (classification system followed by classification symbols)
Int.Cl. C07K7/06, A61K39/00, A61P35/00, A61P35/02, A61P37/04, C07K7/08, C07K19/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS (STN), WPIDS/WPIX (STN)

C. DOCUMENTS CONSIDERED TO BE RELEVANT

| Category* | Citation of document, with indication, where appropriate, of the relevant passages | Relevant to claim No. |
|---|---|---|
| X | WO 2010/037395 A2 (DAKO DENMARK A/S) 08 April 2010, table 10, claims & US 2011/0318380 A1 & EP 2337795 A2 | 1, 2, 4-6, 20-29 |
| Y/A | WO 2014/157692 A1 (SUMITOMO DAINIPPON PHARMA CO., LTD.) 02 October 2014, claims, paragraph [0291], examples, test example 1 & US 2015/0080321 A1, claims, examples, paragraphs [0122]-[0124], experimental example 1 & EP 2982681 A1 | 1-6, 8-29/7 |
| Y/A | WO 2014/157704 A1 (SUMITOMO DAINIPPON PHARMA CO., LTD.) 02 October 2014, test examples 1, 2 & US 2016/0045582 A1, experimental examples 1, 2 & EP 2980097 A1 | 1-6, 8-29/7 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>08 June 2018 (08.06.2018) | Date of mailing of the international search report<br>19 June 2018 (19.06.2018) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017068541 A
- WO 0006602 A
- WO 02079253 A
- WO 2014157692 A
- WO 03106682 A
- WO 2009072610 A
- WO 0279253 A
- WO 2004026897 A
- WO 2003106682 A
- WO 0247474 A
- WO 2006078059 A
- US 2868691 A
- US 3095355 A
- WO 2000006602 A
- WO 2002079253 A
- WO 2004063903 A
- WO 2007063903 A
- WO 2010123065 A
- WO 2005053618 A
- WO 2007047764 A
- WO 2007120673 A
- WO 2005045027 A
- WO 2010037395 A
- WO 2000018795 A
- WO 2002028414 A
- WO 2003037060 A
- WO 2004100870 A


### Non-patent literature cited in the description


- *Clinical Cancer Research,* 2009, vol. 15 (17), 5323-5337
- *Proceedings of the national academy of sciences of United States of America,* 2005, vol. 102 (47), 17107-17112
- *The Journal of Immunology,* 2009, vol. 183, 5526-5536
- *The Journal of Immunology,* 2010, vol. 184, 4725-4732
- *J. Immunol.,* 1994, vol. 152, 3913
- *J. Immunol.,* 1994, vol. 155, 4307
- *Immunogenetics,* 1995, vol. 41, 178
- *Int. J. Cancer,* 2002, vol. 100, 565-570
- *Nat. Med.,* 1998, vol. 4, 321-327
- *Cancer Immunol. Immunother.,* 2002, vol. 51, 271
- **SAMBROOK J. ; FRISCH E. F. ; MANIATIS T.** Molecular Cloning. Cold Spring Harbor Laboratory press
- Molecular Biology. John Wiley & Sons, 1989, 6.3.1-6.3.6
- Current protocols in Molecular Biology. John Wiley and Sons, 1987
- Antibodies; A Laboratory Manual. Cold Spring Harber Laboratory Press, 1989
- Peptide Synthesis. Interscience, 1966
- The Proteins. Academic Press Inc, 1976, vol. 2
- peptide synthesis. Maruzen Co., LTD, 1975
- Basics and Experiment of Peptide Synthesis. Maruzen Co., LTD, 1985
- Development of Pharmaceutical Product subsequent. Peptide Synthesis. Hirokawa Shoten, 1991, vol. 14
- **T. MANIATIS et al.** Molecular Cloning. CSH Laboratory, 1983
- **DM. GLOVER.** DNA Cloning. IRL PRESS, 1985
- Protective Groups in Organic Synthesis. John Wiley & Sons, Inc, 1990
- Basics and Experiment of peptide synthesis. Maruzen Co., LTD, 1985
- Development of Pharmaceutical Product sequential. Peptide Synthesis. Hirokawa Shoten, 1991, vol. 14
- *Tetrahedron Letters,* vol. 37 (9), 1347-1350
- Jikken Kagaku Kouza. Maruzen, vol. 1
- *Nat Rev Drug Discov.,* 2013, vol. 12, 130-146
- *Nikkei Medical Cancer Review,* 2014, 9
- *Nat Rev Immunol.,* 2014, vol. 14, 559-69
- *Clin. Microbiol. Rev.,* 1994, vol. 7, 277-289
- *Int J Cancer.,* 2002, vol. 100, 565-70
- *PLoS One,* November 2008, vol. 3 (11), e3658

SEQUENCE LISTING

<110> Sumitomo Dainippon Pharma Co., Ltd.
International Institute of Cancer Immunology, Inc.

<120> WT1 Cancer Antigen Peptides and Peptide Conjugates Comprising the Peptides

<130> 674039

<150> JP 2017-068541
<151> 2017-03-30

<160> 68

<170> PatentIn version 3.5

<210> 1
<211> 449
<212> PRT
<213> Homo sapiens

<400> 1

```
Met Gly Ser Asp Val Arg Asp Leu Asn Ala Leu Leu Pro Ala Val Pro
1               5                   10                  15

Ser Leu Gly Gly Gly Gly Gly Cys Ala Leu Pro Val Ser Gly Ala Ala
            20                  25                  30

Gln Trp Ala Pro Val Leu Asp Phe Ala Pro Pro Gly Ala Ser Ala Tyr
        35                  40                  45

Gly Ser Leu Gly Gly Pro Ala Pro Pro Pro Ala Pro Pro Pro Pro Pro
    50                  55                  60

Pro Pro Pro Pro His Ser Phe Ile Lys Gln Glu Pro Ser Trp Gly Gly
65                  70                  75                  80

Ala Glu Pro His Glu Glu Gln Cys Leu Ser Ala Phe Thr Val His Phe
                85                  90                  95

Ser Gly Gln Phe Thr Gly Thr Ala Gly Ala Cys Arg Tyr Gly Pro Phe
            100                 105                 110

Gly Pro Pro Pro Pro Ser Gln Ala Ser Ser Gly Gln Ala Arg Met Phe
        115                 120                 125

Pro Asn Ala Pro Tyr Leu Pro Ser Cys Leu Glu Ser Gln Pro Ala Ile
    130                 135                 140

Arg Asn Gln Gly Tyr Ser Thr Val Thr Phe Asp Gly Thr Pro Ser Tyr
145                 150                 155                 160
```

```
Gly His Thr Pro Ser His His Ala Ala Gln Phe Pro Asn His Ser Phe
                165                 170                 175

Lys His Glu Asp Pro Met Gly Gln Gln Gly Ser Leu Gly Glu Gln Gln
            180                 185                 190

Tyr Ser Val Pro Pro Pro Val Tyr Gly Cys His Thr Pro Thr Asp Ser
        195                 200                 205

Cys Thr Gly Ser Gln Ala Leu Leu Leu Arg Thr Pro Tyr Ser Ser Asp
    210                 215                 220

Asn Leu Tyr Gln Met Thr Ser Gln Leu Glu Cys Met Thr Trp Asn Gln
225                 230                 235                 240

Met Asn Leu Gly Ala Thr Leu Lys Gly Val Ala Ala Gly Ser Ser Ser
                245                 250                 255

Ser Val Lys Trp Thr Glu Gly Gln Ser Asn His Ser Thr Gly Tyr Glu
            260                 265                 270

Ser Asp Asn His Thr Thr Pro Ile Leu Cys Gly Ala Gln Tyr Arg Ile
        275                 280                 285

His Thr His Gly Val Phe Arg Gly Ile Gln Asp Val Arg Arg Val Pro
    290                 295                 300

Gly Val Ala Pro Thr Leu Val Arg Ser Ala Ser Glu Thr Ser Glu Lys
305                 310                 315                 320

Arg Pro Phe Met Cys Ala Tyr Pro Gly Cys Asn Lys Arg Tyr Phe Lys
                325                 330                 335

Leu Ser His Leu Gln Met His Ser Arg Lys His Thr Gly Glu Lys Pro
            340                 345                 350

Tyr Gln Cys Asp Phe Lys Asp Cys Glu Arg Arg Phe Ser Arg Ser Asp
        355                 360                 365

Gln Leu Lys Arg His Gln Arg Arg His Thr Gly Val Lys Pro Phe Gln
    370                 375                 380

Cys Lys Thr Cys Gln Arg Lys Phe Ser Arg Ser Asp His Leu Lys Thr
385                 390                 395                 400

His Thr Arg Thr His Thr Gly Lys Thr Ser Glu Lys Pro Phe Ser Cys
                405                 410                 415
```

Arg Trp Pro Ser Cys Gln Lys Lys Phe Ala Arg Ser Asp Glu Leu Val
420 425 430

Arg His His Asn Met His Gln Arg Asn Met Thr Lys Leu Gln Leu Ala
435 440 445

Leu

<210> 2
<211> 9
<212> PRT
<213> Artificial Sequence

<220>
<223> Peptide

<400> 2

Arg Met Phe Pro Asn Ala Pro Tyr Leu
1 5

<210> 3
<211> 9
<212> PRT
<213> Artificial Sequence

<220>
<223> Peptide

<400> 3

Cys Met Thr Trp Asn Gln Met Asn Leu
1 5

<210> 4
<211> 9
<212> PRT
<213> Artificial Sequence

<220>
<223> Peptide

<400> 4

Cys Tyr Thr Trp Asn Gln Met Asn Leu
1 5

<210> 5
<211> 9
<212> PRT
<213> Artificial Sequence

<220>
<223> Peptide

<400> 5

Ala Leu Leu Pro Ala Val Pro Ser Leu
1 5

<210> 6
<211> 9
<212> PRT
<213> Artificial Sequence

<220>
<223> Peptide

<400> 6

Ser Leu Gly Glu Gln Gln Tyr Ser Val
1 5

<210> 7
<211> 9
<212> PRT
<213> Artificial Sequence

<220>
<223> Peptide

<400> 7

Arg Val Pro Gly Val Ala Pro Thr Leu
1 5

<210> 8
<211> 10
<212> PRT
<213> Artificial Sequence

<220>
<223> Peptide

<400> 8

Cys Arg Met Phe Pro Asn Ala Pro Tyr Leu
1 5 10

<210> 9
<211> 10
<212> PRT
<213> Artificial Sequence

<220>
<223> Peptide

<400> 9

Cys Cys Met Thr Trp Asn Gln Met Asn Leu
1 5 10

<210> 10
<211> 10
<212> PRT
<213> Artificial Sequence

<220>
<223> Peptide

<400> 10

Cys Cys Tyr Thr Trp Asn Gln Met Asn Leu
1 5 10

<210> 11
<211> 10
<212> PRT
<213> Artificial Sequence

<220>
<223> Peptide

<400> 11

Cys Ala Leu Leu Pro Ala Val Pro Ser Leu
1 5 10

<210> 12
<211> 10
<212> PRT
<213> Artificial Sequence

<220>
<223> Peptide

<400> 12

Cys Ser Leu Gly Glu Gln Gln Tyr Ser Val
1 5 10

<210> 13
<211> 10
<212> PRT
<213> Artificial Sequence

<220>
<223> Peptide

<400> 13

Cys Arg Val Pro Gly Val Ala Pro Thr Leu
1 5 10

<210> 14
<211> 16
<212> PRT
<213> Artificial Sequence

<220>
<223> Peptide

<400> 14

Ser Gly Gln Ala Arg Met Phe Pro Asn Ala Pro Tyr Leu Pro Ser Cys
1               5                   10                  15

<210> 15
<211> 19
<212> PRT
<213> Artificial Sequence

<220>
<223> Peptide

<400> 15

Ser Gly Gln Ala Arg Met Phe Pro Asn Ala Pro Tyr Leu Pro Ser Cys
1               5                   10                  15

Leu Glu Ser

<210> 16
<211> 19
<212> PRT
<213> Artificial Sequence

<220>
<223> Peptide

<400> 16

Pro Gly Cys Asn Lys Arg Tyr Phe Lys Leu Ser His Leu Gln Met His
1               5                   10                  15

Ser Arg Lys

<210> 17
<211> 20
<212> PRT
<213> Artificial Sequence

<220>
<223> Peptide

<400> 17

Pro Gly Cys Asn Lys Arg Tyr Phe Lys Leu Ser His Leu Gln Met His
1               5                   10                  15

Ser Arg Lys His
            20

<210> 18
<211> 22

<212> PRT
<213> Artificial Sequence

<220>
<223> Peptide

<400> 18

Pro Gly Cys Asn Lys Arg Tyr Phe Lys Leu Ser His Leu Gln Met His
1 5 10 15

Ser Arg Lys His Thr Gly
20

<210> 19
<211> 17
<212> PRT
<213> Artificial Sequence

<220>
<223> Peptide

<400> 19

Cys Asn Lys Arg Tyr Phe Lys Leu Ser His Leu Gln Met His Ser Arg
1 5 10 15

Lys

<210> 20
<211> 18
<212> PRT
<213> Artificial Sequence

<220>
<223> Peptide

<400> 20

Cys Asn Lys Arg Tyr Phe Lys Leu Ser His Leu Gln Met His Ser Arg
1 5 10 15

Lys His

<210> 21
<211> 20
<212> PRT
<213> Artificial Sequence

<220>
<223> Peptide

<400> 21

Cys Asn Lys Arg Tyr Phe Lys Leu Ser His Leu Gln Met His Ser Arg

1 5 10 15

Lys His Thr Gly
20

<210> 22
<211> 16
<212> PRT
<213> Artificial Sequence

<220>
<223> Peptide

<400> 22

Ser Gly Gln Ala Tyr Met Phe Pro Asn Ala Pro Tyr Leu Pro Ser Cys
1 5 10 15

<210> 23
<211> 19
<212> PRT
<213> Artificial Sequence

<220>
<223> Peptide

<400> 23

Ser Gly Gln Ala Tyr Met Phe Pro Asn Ala Pro Tyr Leu Pro Ser Cys
1 5 10 15

Leu Glu Ser

<210> 24
<211> 18
<212> PRT
<213> Artificial Sequence

<220>
<223> Peptide

<400> 24

Trp Ala Pro Val Leu Asp Phe Ala Pro Pro Gly Ala Ser Ala Tyr Gly
1 5 10 15

Ser Leu

<210> 25
<211> 19
<212> PRT
<213> Artificial Sequence

<220>

<223> Peptide

<400> 25

Cys Trp Ala Pro Val Leu Asp Phe Ala Pro Pro Gly Ala Ser Ala Tyr
1               5                   10                  15

Gly Ser Leu

<210> 26
<211> 19
<212> PRT
<213> Artificial Sequence

<220>
<223> Peptide

<400> 26

Trp Ala Pro Val Leu Asp Phe Ala Pro Pro Gly Ala Ser Ala Tyr Gly
1               5                   10                  15

Ser Leu Cys

<210> 27
<211> 10
<212> PRT
<213> Artificial Sequence

<220>
<223> Peptide

<400> 27

Arg Cys Met Thr Trp Asn Gln Met Asn Leu
1               5                   10

<210> 28
<211> 10
<212> PRT
<213> Artificial Sequence

<220>
<223> Peptide

<400> 28

Arg Cys Tyr Thr Trp Asn Gln Met Asn Leu
1               5                   10

<210> 29
<211> 10
<212> PRT
<213> Artificial Sequence

<220>
<223> Peptide

<400> 29

Gln Cys Met Thr Trp Asn Gln Met Asn Leu
1 5 10

<210> 30
<211> 10
<212> PRT
<213> Artificial Sequence

<220>
<223> Peptide

<400> 30

Gln Cys Tyr Thr Trp Asn Gln Met Asn Leu
1 5 10

<210> 31
<211> 10
<212> PRT
<213> Artificial Sequence

<220>
<223> Peptide

<400> 31

Glu Cys Met Thr Trp Asn Gln Met Asn Leu
1 5 10

<210> 32
<211> 10
<212> PRT
<213> Artificial Sequence

<220>
<223> Peptide

<400> 32

Glu Cys Tyr Thr Trp Asn Gln Met Asn Leu
1 5 10

<210> 33
<211> 10
<212> PRT
<213> Artificial Sequence

<220>
<223> Peptide

<400> 33

His Cys Met Thr Trp Asn Gln Met Asn Leu
1 5 10

<210> 34
<211> 10
<212> PRT
<213> Artificial Sequence

<220>
<223> Peptide

<400> 34

His Cys Tyr Thr Trp Asn Gln Met Asn Leu
1 5 10

<210> 35
<211> 10
<212> PRT
<213> Artificial Sequence

<220>
<223> Peptide

<400> 35

Lys Cys Met Thr Trp Asn Gln Met Asn Leu
1 5 10

<210> 36
<211> 10
<212> PRT
<213> Artificial Sequence

<220>
<223> Peptide

<400> 36

Lys Cys Tyr Thr Trp Asn Gln Met Asn Leu
1 5 10

<210> 37
<211> 10
<212> PRT
<213> Artificial Sequence

<220>
<223> Peptide

<400> 37

Phe Cys Met Thr Trp Asn Gln Met Asn Leu
1 5 10

<210> 38
<211> 10
<212> PRT
<213> Artificial Sequence

<220>
<223> Peptide

<400> 38

Phe Cys Tyr Thr Trp Asn Gln Met Asn Leu
1 5 10

<210> 39
<211> 10
<212> PRT
<213> Artificial Sequence

<220>
<223> Peptide

<400> 39

Tyr Cys Met Thr Trp Asn Gln Met Asn Leu
1 5 10

<210> 40
<211> 10
<212> PRT
<213> Artificial Sequence

<220>
<223> Peptide

<400> 40

Tyr Cys Tyr Thr Trp Asn Gln Met Asn Leu
1 5 10

<210> 41
<211> 9
<212> PRT
<213> Artificial Sequence

<220>
<223> Peptide

<400> 41

Val Leu Asp Phe Ala Pro Pro Gly Ala
1 5

<210> 42
<211> 9
<212> PRT
<213> Artificial Sequence

<220>
<223> Peptide

<400> 42

Arg Tyr Phe Pro Asn Ala Pro Tyr Leu
1 5

<210> 43
<211> 9
<212> PRT
<213> Artificial Sequence

<220>
<223> Peptide

<400> 43

Phe Met Phe Pro Asn Ala Pro Tyr Leu
1 5

<210> 44
<211> 9
<212> PRT
<213> Artificial Sequence

<220>
<223> Peptide

<400> 44

Arg Leu Phe Pro Asn Ala Pro Tyr Leu
1 5

<210> 45
<211> 9
<212> PRT
<213> Artificial Sequence

<220>
<223> Peptide

<400> 45

Arg Met Met Pro Asn Ala Pro Tyr Leu
1 5

<210> 46
<211> 9
<212> PRT
<213> Artificial Sequence

<220>
<223> Peptide

<400> 46

Arg Met Phe Pro Asn Ala Pro Tyr Val
1 5

<210> 47
<211> 9
<212> PRT
<213> Artificial Sequence

<220>
<223> Peptide

<400> 47

Tyr Met Phe Pro Asn Ala Pro Tyr Leu
1 5

<210> 48
<211> 9
<212> PRT
<213> Artificial Sequence

<220>
<223> Peptide

<220>
<221> MISC_FEATURE
<222> (1)..(1)
<223> Xaa is Ser or Ala

<400> 48

Xaa Met Thr Trp Asn Gln Met Asn Leu
1 5

<210> 49
<211> 9
<212> PRT
<213> Artificial Sequence

<220>
<223> Peptide

<220>
<221> MISC_FEATURE
<222> (1)..(1)
<223> Xaa is Ser, Ala, Abu, Arg, Lys, Orn, Cit, Leu, Phe, or Asn

<400> 49

Xaa Tyr Thr Trp Asn Gln Met Asn Leu
1 5

<210> 50
<211> 9
<212> PRT
<213> Artificial Sequence

<220>
<223> Peptide

<400> 50

Ala Tyr Leu Pro Ala Val Pro Ser Leu
1 5

<210> 51
<211> 9
<212> PRT
<213> Artificial Sequence

<220>
<223> Peptide

<400> 51

Phe Leu Gly Glu Gln Gln Tyr Ser Val
1 5

<210> 52
<211> 9
<212> PRT
<213> Artificial Sequence

<220>
<223> Peptide

<400> 52

Ser Met Gly Glu Gln Gln Tyr Ser Val
1 5

<210> 53
<211> 9
<212> PRT
<213> Artificial Sequence

<220>
<223> Peptide

<400> 53

Ser Leu Met Glu Gln Gln Tyr Ser Val
1 5

<210> 54
<211> 9
<212> PRT
<213> Artificial Sequence

<220>
<223> Peptide

<400> 54

Arg Tyr Pro Gly Val Ala Pro Thr Leu
1 5

<210> 55
<211> 10
<212> PRT
<213> Artificial Sequence

<220>
<223> Peptide

<400>  55

Ala Cys Tyr Thr Trp Asn Gln Met Asn Leu
1               5                   10

<210>  56
<211>  10
<212>  PRT
<213>  Artificial Sequence

<220>
<223>  Peptide

<400>  56

Asp Cys Tyr Thr Trp Asn Gln Met Asn Leu
1               5                   10

<210>  57
<211>  10
<212>  PRT
<213>  Artificial Sequence

<220>
<223>  Peptide

<400>  57

Gly Cys Tyr Thr Trp Asn Gln Met Asn Leu
1               5                   10

<210>  58
<211>  10
<212>  PRT
<213>  Artificial Sequence

<220>
<223>  Peptide

<400>  58

Ile Cys Tyr Thr Trp Asn Gln Met Asn Leu
1               5                   10

<210>  59
<211>  10
<212>  PRT
<213>  Artificial Sequence

<220>
<223>  Peptide

<400>  59

Leu Cys Tyr Thr Trp Asn Gln Met Asn Leu
1               5                   10

<210> 60
<211> 10
<212> PRT
<213> Artificial Sequence

<220>
<223> Peptide

<400> 60

Met Cys Tyr Thr Trp Asn Gln Met Asn Leu
1 5 10

<210> 61
<211> 10
<212> PRT
<213> Artificial Sequence

<220>
<223> Peptide

<400> 61

Asn Cys Tyr Thr Trp Asn Gln Met Asn Leu
1 5 10

<210> 62
<211> 10
<212> PRT
<213> Artificial Sequence

<220>
<223> Peptide

<400> 62

Pro Cys Tyr Thr Trp Asn Gln Met Asn Leu
1 5 10

<210> 63
<211> 10
<212> PRT
<213> Artificial Sequence

<220>
<223> Peptide

<400> 63

Ser Cys Tyr Thr Trp Asn Gln Met Asn Leu
1 5 10

<210> 64
<211> 10
<212> PRT
<213> Artificial Sequence

<220>
<223> Peptide

<400> 64

Thr Cys Tyr Thr Trp Asn Gln Met Asn Leu
1 5 10

<210> 65
<211> 10
<212> PRT
<213> Artificial Sequence

<220>
<223> Peptide

<400> 65

Val Cys Tyr Thr Trp Asn Gln Met Asn Leu
1 5 10

<210> 66
<211> 10
<212> PRT
<213> Artificial Sequence

<220>
<223> Peptide

<400> 66

Trp Cys Tyr Thr Trp Asn Gln Met Asn Leu
1 5 10

<210> 67
<211> 10
<212> PRT
<213> Artificial Sequence

<220>
<223> Peptide

<220>
<221> MOD_RES
<222> (1)..(1)
<223> Protected with Npys group

<400> 67

Cys Arg Met Phe Pro Asn Ala Pro Tyr Leu
1 5 10

<210> 68
<211> 16
<212> PRT
<213> Artificial Sequence

<220>
<223> Peptide

<400> 68

```
Lys Arg Tyr Phe Lys Leu Ser His Leu Gln Met His Ser Arg Lys His
1               5                   10                  15
```